# EUROPEAN PATENT APPLICATION

(11) **EP 2 039 681 A1**
(43) Date of publication of application: **25.03.2009**
(21) Application number: 07790466.2
(22) Date of filing: 06.07.2007
(51) Int. Cl.: C07C 311/14, A61K 31/215, A61K 31/223, A61K 31/225, A61K 31/265, A61K 31/27, A61K 31/401, A61K 31/445, A61P 1/00, A61P 9/00, A61P 19/00, A61P 25/00, A61P 29/00, A61P 31/00, A61P 37/02, A61P 43/00, C07C 303/36, C07D 207/16, C07D 211/34

(54) **CYCLOALKENE DERIVATIVES, PROCESS FOR PRODUCTION OF THE DERIVATIVES, AND USE OF THE SAME**

(30) Priority: 07.07.2006 JP 2006188238
(71) Applicant: Takeda Pharmaceutical Company Limited, Osaka-shi, Osaka 541-0045 (JP)
(72) Inventor: ICHIKAWA, Takashi, Osaka-shi Osaka 532-8686 (JP); KITAZAKI, Tomoyuki, Tsukuba-shi Ibaraki 300-4293 (JP); TAMURA, Norikazu, Osaka-shi Osaka 532-8686 (JP)
(74) Representative: Best, Michael
(86) International application number: PCT/JP2007/063595
(87) International publication number: WO 2008/004673

(57) **Abstract**

The present invention relates to a cycloalkene derivative represented by the formula (I): 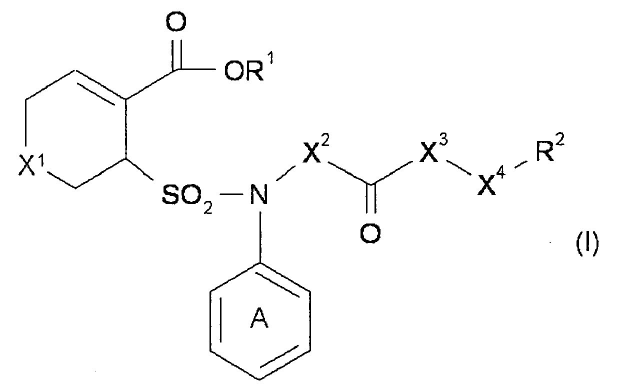 wherein each symbol is as defined in the specification, a pharmaceutical agent containing the derivative, and a production method thereof. The cycloalkene derivative of the present invention has high solubility in water and is suitable for use as an injection.

## Description

### Technical Field

The present invention relates to a novel cycloalkene derivative useful as a prophylactic or therapeutic agent for diseases such as cardiac disease, autoimmune disease, inflammatory disease, central nervous system disease, infectious disease, sepsis, severe sepsis, septic shock and the like, a production method thereof and use thereof.

### Background of the Invention

Nitric oxide (NO) has been reported to have various in vivo physiological activities in mammals. NO is produced principally from L-arginine by NO synthetase (NOS). NOS is currently proven to exist as three genetic isoforms, namely, neuronal NOS, endothelial NOS and inducible NOS (iNOS) (Cell, Vol. 70, p. 705-707, 1992).
Of these, iNOS is induced in macrophages, neutrophile and the like by various cytokines, bacterial lipopolysaccharides (LPS) and the like to continuously produce a large amount of NO. Therefore, iNOS is considered to have not only the pharmacological effects described above but also cell- and tissue-damaging effects at the site of the production (Immunol. Today, Vol. 13, p. 157-160, 1992). NO produced in cells and tissues expressing iNOS has been reported to be involved in various diseases and pathologies. Accordingly, a substance that inhibits the NO production by iNOS inducible cells is expected to be effective as an agent for the prophylaxis or treatment of such various diseases.
On the other hand, cytokines such as TNF-α, IL-1, IL-6 and the like are secreted from various cells such as monocyte, macrophage, lymphocyte, neutrophile, fibroblast, vascular endothelial cells and the like, and involved widely in inflammation-related biological defense and immune mechanisms (The Cytokine Handbook, 2nd ed., Academic Press Limited, 1994; Advances Immunol., Vol. 62, p. 257-304, 1996), and thus are referred to as inflammatory cytokines. However, these cytokines, once produced excessively or produced in a wrong site or at a wrong time, exhibit undesirable biological effects, and are proven to be involved in various diseases such as cachexia due to protozoa, bacteria, fungi, viruses, cancers and the like, allergic diseases, chronic rheumatoid arthritis, abscess, graft rejection, anemia, arteriosclerosis, autoimmune disease, diabetes, central nervous system diseases, inflammatory bowel diseases, cardiac failure, hepatitis, hepatocirrhosis, nephritis, osteoporosis, psoriasis, septic shock and the like. In addition, substances which have inhibitory effects on the production of these cytokines or antagonistic effects on these cytokines were reported to be expected to serve as the therapeutic agents for the diseases listed above (Eur. J. Immunol., Vol. 18, p. 951-956, 1991; Immunol., Vol. 83, p. 262-267, 1994; Proc. Natl. Acad. Sci., Vol. 93, p. 3967-3971, 1997; J. Immunol., Vol. 147, p. 1530-1536, 1991; Immunol. Today, Vol. 12, p. 404-410, 1991).
Patent reference 1 describes that (i) a compound represented by the formula:

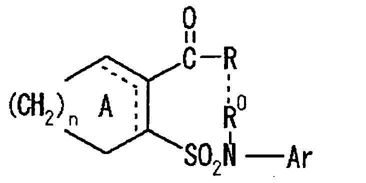

wherein
R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula:

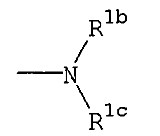

wherein R^{1b} is a hydrogen atom or an aliphatic hydrocarbon group optionally having substituent(s), and R^{1c} is the same as or different from R^{1b}, a hydrogen atom or an aliphatic hydrocarbon group optionally having substituent(s),
R⁰ is a hydrogen atom or an aliphatic hydrocarbon group, or R¹ and R⁰ in combination form a bond,
ring A is a cycloalkene substituted by 1 to 4 substituents selected from (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom,
Ar is an aromatic hydrocarbon group optionally having substituent(s),
a group represented by the formula:

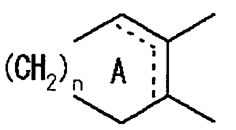

is a group represented by the formula:

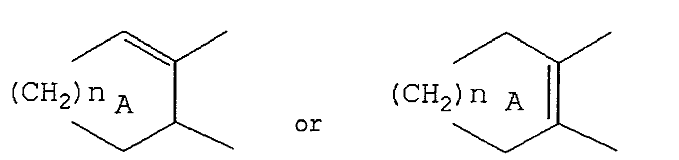

and n is an integer of 1 to 4, and
(ii) a compound represented by the formula:

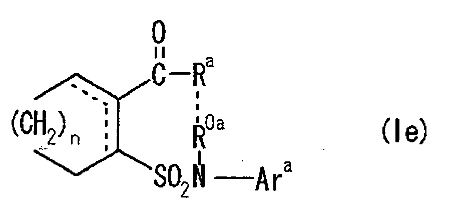

wherein
R^{a} is an aliphatic hydrocarbon group optionally having substituent(s), an aromatic hydrocarbon group optionally having substituent(s), a heterocyclic group optionally having substituent(s), a group represented by the formula: -OR^{1a} wherein R^{1a} is a hydrogen atom or an aliphatic hydrocarbon group optionally having substituent(s), or a group represented by the formula:

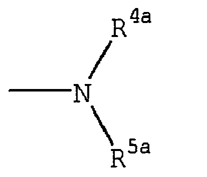

wherein R^{4a} and R^{5a} are the same or different and each is a hydrogen atom or an aliphatic hydrocarbon group optionally having substituent(s),
R^{0a} is a hydrogen atom or an aliphatic hydrocarbon group, or R^{a} and R^{0a} in combination form a bond,
Ar^{a} is an aromatic hydrocarbon group optionally having substituent(s),
a group represented by the formula:

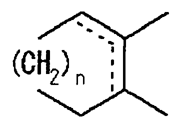

is a group represented by the formula:

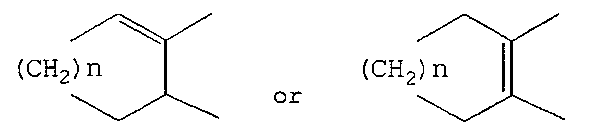

and n is an integer of 1 to 4,
a salt thereof and a prodrug thereof have a nitric oxide (NO) production inhibitory effect and an inflammatory cytokine production inhibitory effects, such as TNF-α, IL-1, IL-6 and the like, and are useful as an agent for the prophylaxis or treatment of diseases including cardiac diseases, autoimmune diseases, inflammatory diseases, central nervous system diseases, infectious diseases, sepsis, septic shock and the like; and
patent reference 2 describes that a compound represented by the formula:

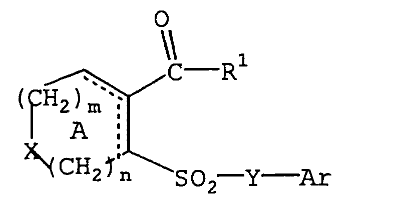

wherein
R¹ is an aliphatic hydrocarbon group optionally having substituent(s), an aromatic hydrocarbon group optionally having substituent(s), a heterocyclic group optionally having substituent(s), a group represented by the formula: -OR^{1a} wherein R^{1a} is a hydrogen atom or an aliphatic hydrocarbon group optionally having substituent(s), or a group represented by the formula:

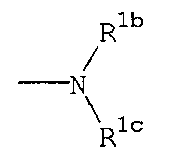

wherein R^{1b} and R^{1c} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s),
ring A is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR² wherein R² is a hydrogen atom or an aliphatic hydrocarbon group optionally having substituent(s), and (4) a halogen atom, Ar is an aromatic hydrocarbon group optionally having substituent(s),
a group represented by the formula:

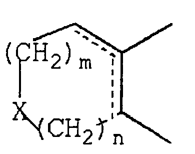

is a group represented by the formula:

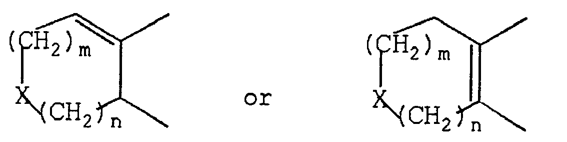

m is an integer of 0 to 2,
n is an integer of 1 to 3, and
the total of m and n is 4 or less;
provided that when X is a methylene group, then Y should be a methylene group optionally having substituent(s), a salt thereof and a prodrug thereof
have a nitric oxide (NO) production inhibitory effect and an inflammatory cytokine production inhibitory effects, such as TNF-α, IL-1, IL-6 and the like, and are useful as an agent for the prophylaxis or treatment of diseases including cardiac diseases, autoimmune diseases, inflammatory diseases, central nervous system diseases, infectious diseases, sepsis, septic shock and the like.
Patent reference 3 describes that the above-mentioned compound is useful as a TLR signaling inhibitor or an agent for the prophylaxis or treatment of severe sepsis.
patent reference 1: WO99/46242
patent reference 2: WO01/10826
patent reference 3: WO03/84527

### Disclosure of the Invention

### Problems to be Solved by the Invention

When the above-mentioned compound having nitric oxide (NO) production inhibitory effect and inflammatory cytokine production inhibitory effect is used as, for example, a liquid preparation (e.g., injection), it does not necessarily show sufficient solubility in water. Thus, the improvement of water solubility is desired.

### Means of Solving the Problems

The present inventors have conducted intensive studies in an attempt to solve the aforementioned problems and found that a compound represented by the following formula (I) and a salt thereof show superior water solubility, and further studied to complete the present invention.

Accordingly, the present invention relates to
[1] a compound represented by the formula (I):

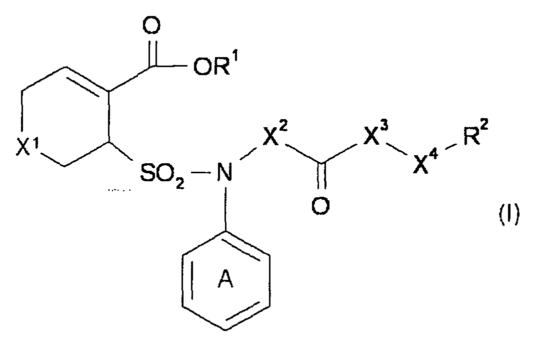

wherein
X¹ is a methylene group or an oxygen atom;
R¹ is a C₁₋₆ alkyl group;
ring A is a phenyl group optionally having one or two the same or different halogen atoms;
X² is a bond, -CHR^{x}-O- wherein R^{x} is a hydrogen atom or a C₁₋₆ alkyl group, or a group represented by the formula:

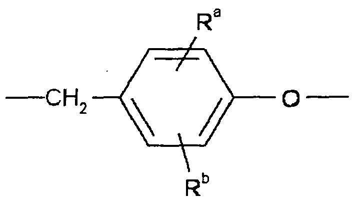

wherein R^{a} and R^{b} are the same or different and each is a hydrogen atom, a halogen atom, a C₁₋₆ alkyl group, a C₁₋₆ alkoxy group, a carboxyl group or a C₁₋₆ alkoxy-carbonyl group;
X³ is a bond, -O- or -NR³- wherein R³ is a hydrogen atom or an aliphatic hydrocarbon group optionally having substituent(s);
X⁴ is a divalent aliphatic hydrocarbon group optionally having substituent(s); and
R² is -COOH, -OPO(OH)₂ or a -NHR⁴ group wherein R⁴ is an aliphatic hydrocarbon group optionally having substituent(s), or R³ or R⁴ is optionally bonded to X⁴ to form a 5- to 8-membered ring optionally having substituent(s), or a salt thereof (hereinafter sometimes to be abbreviated as compound (I));
[2] the compound of the above-mentioned [1], wherein R¹ is an ethyl group, or a salt thereof;
[3] the compound of the above-mentioned [1], wherein ring A is a group represented by the formula:

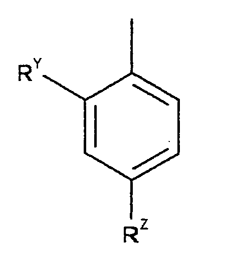

wherein R^{Y} and R^{Z} are the same or different and each is a halogen atom, or a salt thereof;
[4] the compound of the above-mentioned [1], wherein X² is a - CH₂-O- group; and
X³ is -O- or -NR^{3a}- wherein R^{3a} is a hydrogen atom or a C₁₋₆ alkyl group optionally having substituent(s), or R^{3a} is optionally bonded to X⁴ to form a 5- to 8-membered ring optionally having substituent(s), or a salt thereof;
[5] the compound of the above-mentioned [1], wherein X² is a group represented by the formula:

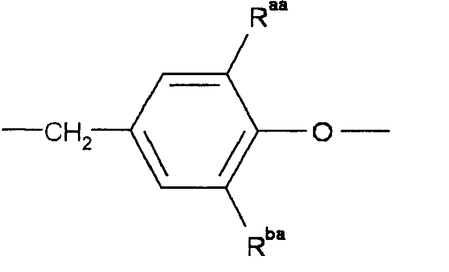

wherein R^{aa} and R^{ba} are the same or different and each is a C₁₋₆ alkyl group; and
X³ is a bond, or a salt thereof;
   [6] the compound of the above-mentioned [1], wherein X² and X³ are each a bond, or a salt thereof;
   [7] the compound of the above-mentioned [1], wherein R² is - COOH or a -NHR^{4a} group wherein R^{4a} is a C₁₋₆ alkyl group optionally having substituent(s), or R^{4a} is optionally bonded to X⁴ to form a 5- to 8-membered ring optionally having substituent(s), or a salt thereof;
   [8] the compound of the above-mentioned [1], wherein X¹ is a methylene group, or a salt thereof;
   [9] the compound of the above-mentioned [1], wherein X¹ is a methylene group;
R¹ is an ethyl group;
ring A is a group represented by the formula:

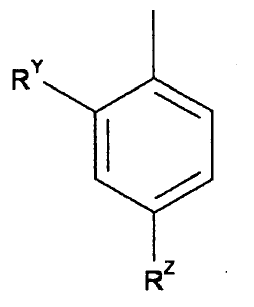

wherein R^{Y} and R^{Z} are the same or different and each is a halogen atom;
X² is a -CH₂-O- group;
X³ is -O- or -NR^{3a}- wherein R^{3a} is a hydrogen atom or an optionally substituted C₁₋₆ alkyl group, or R^{3a} is optionally bonded to X⁴ to form a 5- to 8-membered ring optionally having substituent(s);
X⁴ is an optionally substituted C₁₋₆ alkylene group; and
R² is -COOH, or a salt thereof;
   [10] the compound of the above-mentioned [1], wherein X¹ is a methylene group;
R¹ is an ethyl group;
ring A is a group represented by the formula:

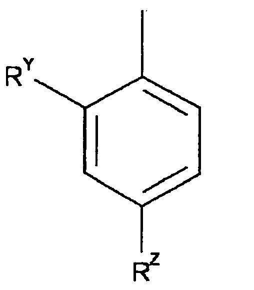

wherein R^{Y} and R^{Z} are the same or different and each is a halogen atom;
X² is a group represented by the formula:

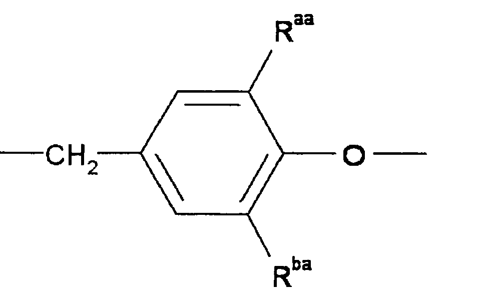

wherein R^{aa} and R^{ba} are the same or different and each is a C₁₋₆ alkyl group;
X³ is a bond;
X⁴ is an optionally substituted C₁₋₆ alkylene group; and
R² is a -NHR^{4a} group wherein R^{4a} is a C₁₋₆ alkyl group optionally having substituent(s), or R^{4a} is optionally bonded to X⁴ to form a 5- to 8-membered ring optionally having substituent(s), or a salt thereof;
   [11] the compound of the above-mentioned [1], wherein X¹ is a methylene group;
R¹ is an ethyl group;
ring A is a group represented by the formula:

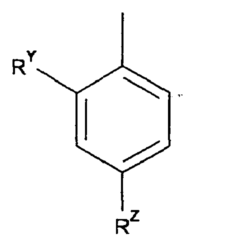

wherein R^{Y} and R^{Z} are the same or different and each is a halogen atom;
X² and X³ are each a bond;
X⁴ is an optionally substituted C₁₋₆ alkylene group; and
R² is -COOH, or a salt thereof;
   [12] the compound of the above-mentioned [1], wherein X¹ is -O-;
R¹ is an ethyl group;
ring A is a group represented by the formula:

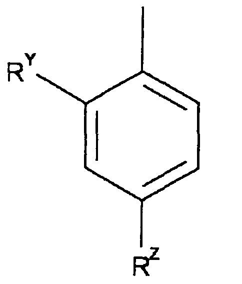

wherein R^{Y} and R^{Z} are the same or different and each is a halogen atom;
X² is a -CH₂-O- group;
X³ is -O- or -NR^{3a}- wherein R^{3a} is a hydrogen atom or an optionally substituted C₁₋₆ alkyl group, or R^{3a} optionally bonded to X⁴ to form a 5- to 8-membered ring optionally having substituent(s);
X⁴ is an optionally substituted C₁₋₆ alkylene group; and
R² is -COOH, or a salt thereof;
   [13] the compound of the above-mentioned [1], wherein X¹ is -O-;
R¹ is an ethyl group;
ring A is a group represented by the formula:

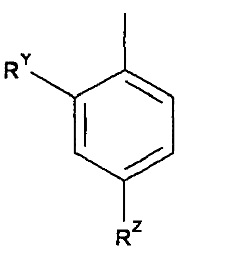

wherein R^{Y} and R^{Z} are the same or different and each is a halogen atom;
X² is a group represented by the formula:

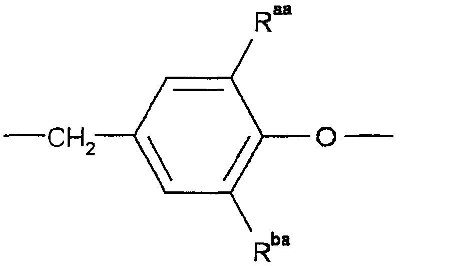

wherein R^{aa} and R^{ba} are the same or different and each is a C₁₋₆ alkyl group;
X³ is a bond;
X⁴ is an optionally substituted C₁₋₆ alkylene group; and
R² is a -NHR^{4a} group wherein R^{4a} is a C₁₋₆ alkyl group optionally having substituent(s), or R^{4a} optionally bonded to X⁴ to form a 5- to 8-membered ring optionally having substituent(s), or a salt thereof;
   [14] the compound of the above-mentioned [1] wherein X¹ is -O-;
R¹ is an ethyl group;
ring A is a group represented by the formula:

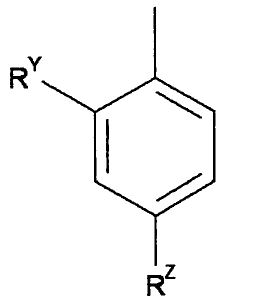

wherein R^{Y} and R^{Z} are the same or different and each is a halogen atom;
X² and X³ are each a bond;
X⁴ is an optionally substituted C₁₋₆ alkylene group; and
R² is -COOH, or a salt thereof;
   [15] the compound of the above-mentioned [7], wherein the salt is selected from the group consisting of a sodium salt, a potassium salt and a hydrochloride;
   [16] a method of producing the compound of the above-mentioned [1] or a salt thereof, which comprises condensing a compound represented by the formula (II):

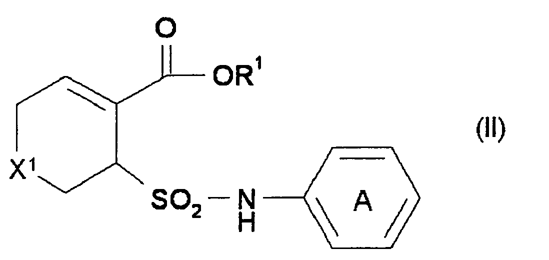

wherein each symbol is as defined in the above-mentioned [1], or a salt thereof, with a compound represented by the formula (III) :

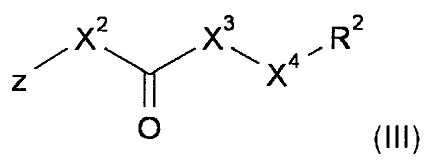

wherein Z is a halogen atom, and other symbols are as defined in the above-mentioned [1], or a salt thereof;
[17] a pharmaceutical composition comprising the compound of the above-mentioned [1] or a salt thereof;
[18] the pharmaceutical composition of the above-mentioned [17], which is an agent for the prophylaxis or treatment of cardiac disease, autoimmune disease or septic shock;
[19] the pharmaceutical composition of the above-mentioned [17], which is an agent for the prophylaxis or treatment of an organ disorder or severe sepsis;
[20] the pharmaceutical composition of the above-mentioned [19], wherein the organ is an organ of the central nerve system, the circulatory system, the bone and joint system or the digestive system;
[21] a method for the prophylaxis or treatment of a cardiac disease, an autoimmune disease or septic shock, which comprises administering an effective amount of the compound of the above-mentioned [1] or a salt thereof to a mammal;
[22] a method for the prophylaxis or treatment of an organ disorder or severe sepsis to a mammal, which comprises administering an effective amount of the compound of the above-mentioned [1] or a salt thereof to a mammal;
[23] use of the compound of the above-mentioned [1] or a salt thereof for the production of an agent for the prophylaxis or treatment of a cardiac disease, an autoimmune disease or septic shock;
[24] use of the compound of the above-mentioned [1] or a salt thereof for the production of an agent for the prophylaxis or treatment of an organ disorder or severe sepsis; and the like.

Compound (I) of the present invention has high solubility in water and can be easily formulated into a liquid preparation (e.g., injection) as compared to conventional compounds having a nitric oxide (NO) production inhibitory effect and an inflammatory cytokine production inhibitory effect.

Each term used in the present specification is explained in the following.
In the present specification, unless otherwise specified, the "halogen atom" is a fluorine atom, a chlorine atom, a bromine atom or an iodine atom.
In the present specification, unless otherwise specified, the "C₁₋₆ alkyl group" is an alkyl group having a carbon number of 1 to 6 (e.g., methyl group, ethyl group, n-propyl group, isopropyl group, n-butyl group, isobutyl group, sec-butyl group, tert-butyl group, pentyl group, hexyl group and the like).
In the present specification, unless otherwise specified, the "C₁₋₆ alkoxy group" is an alkoxy group having a carbon number of 1 to 6 (e.g., methoxy group, ethoxy group, n-propoxy group, isopropoxy group, n-butoxy group, tert-butoxy group, n-pentyloxy group, n-hexyloxy group and the like).
In the present specification, unless otherwise specified, "C₁₋₆ alkoxy-carbonyl group" is an alkoxy-carbonyl group having a carbon number of 1 to 6 (e.g., methoxycarbonyl group, ethoxycarbonyl group, n-propoxycarbonyl group, isopropoxycarbonyl group, n-butoxycarbonyl group, isobutoxycarbonyl group, tert-butoxycarbonyl group, n-pentyloxycarbonyl group, n-hexyloxycarbonyl group and the like).

Each symbol in the formula (I) is explained in the following.
X¹ is a methylene group or an oxygen atom.
R¹ is a C₁₋₆ alkyl group.
ring A is a phenyl group optionally having one or the same or different two halogen atoms.

X² is a bond, -CHR^{x}-O- wherein R^{x} is a hydrogen atom or a C₁₋₆ alkyl group, or a group represented by the formula:

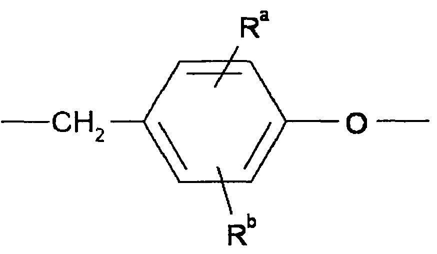

wherein R^{a} and R^{b} are the same or different and each is a hydrogen atom, a halogen atom, a C₁₋₆ alkyl group, a C₁₋₆ alkoxy group, a carboxyl group or a C₁₋₆ alkoxy-carbonyl group.

X³ is a bond, -O- or -NR³- wherein R³ is a hydrogen atom or an aliphatic hydrocarbon group optionally having substituent(s).
R² is -COOH, -OPO(OH)₂ or a -NHR⁴ group wherein R⁴ is an aliphatic hydrocarbon group optionally having substituent(s).

As the "aliphatic hydrocarbon group" of the "aliphatic hydrocarbon group optionally having substituent(s)" for R³ or R⁴, for example, an alkyl group, a cycloalkyl group, a cycloalkyl alkyl group, an alkenyl group, an alkynyl group, etc. can be used.
As the alkyl group, for example, a linear or branched alkyl group having 1 to 20 carbon atoms (e.g., a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, a dodecyl group, etc.) and the like are preferable, and particularly, an alkyl group having 1 to 6 carbon atoms (e.g., a methyl group, an ethyl group, an n-propyl group, an isopropyl group, an n-butyl group, an isobutyl group, a sec-butyl group, a tert-butyl group, a pentyl group, a hexyl group, etc.) and the like are preferable.
As the cycloalkyl group, for example, a cycloalkyl group having 3 to 10 carbon atoms (e.g., a cyclopropyl group, a cyclobutyl group, a cyclopentyl group, a cyclohexyl group, a cycloheptyl group, a cyclooctyl group, etc.) is preferable, and particularly, a cycloalkyl group having 3 to 6 carbon atoms (e.g., a cyclopropyl group, a cyclobutyl group, a cyclopentyl group, a cyclohexyl group, etc.) is preferable.
As the cycloalkyl alkyl group, for example, a cycloalkyl alkyl group having 4 to 12 carbon atoms (e.g., a cyclopropylmethyl group, a cyclopentylmethyl group, a cyclohexylmethyl group, a cycloheptylmethyl group, etc.) is preferable, and particularly, a cycloalkyl alkyl group having 4 to 8 (particularly 4 to 7) carbon atoms (e.g., a cyclopropylmethyl group, a cyclopentylmethyl group, a cyclohexylmethyl group, etc.) is preferable.
As the alkenyl group, for example, a lower alkenyl group having 3 to 6 carbon atoms (e.g., a propenyl group, a butenyl group, a pentenyl group, etc.) is preferable, and particularly, a lower alkenyl group having 3 or 4 carbon atoms (e.g., a propenyl group, a butenyl group, etc.) is preferable.
As the alkynyl group, for example, a lower alkynyl group having 3 to 6 carbon atoms (e.g., a propynyl group, a butynyl group, a pentynyl group, etc.) is preferable, and particularly, a lower alkynyl group having 3 or 4 carbon atoms (e.g., a propynyl group, a butynyl group, etc.) is preferable.

As the "substituent(s)" of the "aliphatic hydrocarbon group" of the above-mentioned "aliphatic hydrocarbon group optionally having substituent(s)", for example, the following can be used:
(1) a heterocyclic group;
(2) an oxo group;
(3) a hydroxy group;
(4) a C₁₋₆ alkoxy group;
(5) a C₃₋₁₀ (particularly C₃₋₆) cycloalkyloxy group;
(6) a C₆₋₁₀ aryloxy group;
(7) a C₇₋₁₉ (particularly C₇₋₁₂) aralkyloxy group;
(8) a heterocyclyloxy group;
(9) a C₁₋₆ alkylthio group (the sulfur atom may be oxidized);
(10) a C₃₋₁₀ (particularly C₃₋₆) cycloalkylthio group (the sulfur atom may be oxidized);
(11) a C₆₋₁₀ arylthio group (the sulfur atom may be oxidized);
(12) a C₇₋₁₉ (particularly C₇₋₁₂) aralkylthio group (the sulfur atom may be oxidized);
(13) a heterocyclylthio group;
(14) a heterocyclylsulfinyl group;
(15) a heterocyclylsulfonyl group;
(16) a nitro group;
(17) a halogen atom;
(18) a cyano group;
(19) a carboxyl group;
(20) a C₁₋₁₀ (particularly C₁₋₆) alkoxy-carbonyl group;
(21) a C₃₋₆ cycloalkyloxy-carbonyl group;
(22) a C₆₋₁₀ aryloxy-carbonyl group;
(23) a C₇₋₁₉ (particularly C₇₋₁₂) aralkyloxy-carbonyl group;
(24) a heterocyclyloxy-carbonyl group;
(25) a C₆₋₁₀ aryl-carbonyl group;
(26) a C₁₋₆ alkanoyl group;
(27) a C₃₋₅ alkenoyl group;
(28) a C₆₋₁₀ aryl-carbonyloxy group;
(29) a C₂₋₆ alkanoyloxy group;
(30) a C₃₋₅ alkenoyloxy group;
(31) a carbamoyl group optionally having substituent(s);
(32) a thiocarbamoyl group optionally having substituent(s);
(33) a carbamoyloxy group optionally having substituent(s);
(34) a C₁₋₆ alkanoylamino group;
(35) a C₆₋₁₀ aryl-carbonylamino group;
(36) a C₁₋₁₀ (particularly C₁₋₆) alkoxy-carboxamido group;
(37) a C₆₋₁₀ aryloxy-carboxamido group;
(38) a C₇₋₁₉ (particularly C₇₋₁₂) aralkyloxy-carboxamido group;
(39) a C₁₋₁₀ (particularly C₁₋₆) alkoxy-carbonyloxy group;
(40) a C₆₋₁₀ aryloxy-carbonyloxy group;
(41) a C₇₋₁₉ (particularly C₇₋₁₂) aralkyloxy-carbonyloxy group;
(42) a C₃₋₁₀ (particularly C₃₋₆) cycloalkyloxy-carbonyloxy group;
(43) a ureido group optionally having substituent(s); and
(44) a C₆₋₁₀ aryl group optionally having substituent(s).
These substituents substitute at substitutable positions in the above-mentioned "aliphatic hydrocarbon group", wherein the substituents are not limited to a single substituent but may be the same or different plural (preferably 2 to 4) substituents.

As the "C₁₋₆ alkoxy group", for example, a methoxy group, an ethoxy group, an n-propoxy group, an isopropoxy group, an n-butoxy group, a tert-butoxy group, an n-pentyloxy group, an n-hexyloxy group, etc. can be used.
As the "C₃₋₁₀ cycloalkyloxy group", for example, a cyclopropyloxy group, a cyclohexyloxy group, etc. can be used.
As the "C₆-₁₀ aryloxy group", for example, a phenoxy group, a naphthyloxy group, etc. can be used.
As the "C₇₋₁₉ aralkyloxy group", for example, a benzyloxy group, a 1-phenylethyloxy group, a 2-phenylethyloxy group, a benzhydryloxy group, a 1-naphthylmethyloxy group, etc. can be used.
As the "C₁₋₆ alkylthio group (the sulfur atom may be oxidized)", for example, a methylthio group, an ethylthio group, an n-propylthio group, an n-butylthio group, a methylsulfinyl group, a methylsulfonyl group, etc. can be used.
As the "C₃₋₁₀ cycloalkylthio group (the sulfur atom may be oxidized)", for example, a cyclopropylthio group, a cyclohexylthio group, a cyclopentylsulfinyl group, a cyclohexylsulfonyl group, etc. can be used.
As the "C₆₋₁₀ arylthio group (the sulfur atom may be oxidized)", for example, a phenylthio group, a naphthylthio group, a phenylsulfinyl group, a phenylsulfonyl group, etc. can be used.
As the "C₇₋₁₉ aralkylthio group (the sulfur atom may be oxidized)", for example, a benzylthio group, a phenylethylthio group, a benzhydrylthio group, a benzylsulfinyl group, a benzylsulfonyl group, etc. can be used.
As the "halogen atom", a fluorine atom, a chlorine atom, a bromine atom and an iodine atom can be used.
As the "C₁₋₁₀ alkoxy-carbonyl group", for example, a methoxycarbonyl group, an ethoxycarbonyl group, an n-propoxycarbonyl group, an isopropoxycarbonyl group, an n-butoxycarbonyl group, an isobutoxycarbonyl group, a tert-butoxycarbonyl group, an n-pentyloxycarbonyl group, an n-hexyloxycarbonyl group, etc. can be used.
As the "C₃₋₆ cycloalkyloxy-carbonyl group", for example, a cyclopropyloxycarbonyl group, a cyclopentyloxycarbonyl group, a cyclohexyloxycarbonyl group, etc. can be used.
As the "C₆₋₁₀ aryloxy-carbonyl group", for example, a phenoxycarbonyl group, a naphthyloxycarbonyl group, etc. can be used.
As the "C₇₋₁₉ aralkyloxy-carbonyl group", for example, a benzyloxycarbonyl group, a benzhydryloxycarbonyl group, a 2-phenethyloxycarbonyl group, etc. can be used.
As the "C₆₋₁₀ aryl-carbonyl group", for example, a benzoyl group, a naphthoyl group, etc. can be used.
As the "C₁₋₆ alkanoyl group", for example, a formyl group, an acetyl group, a propionyl group, a butyryl group, a valeryl group, a pivaloyl group, etc. can be used.
As the "C₃₋₅ alkenoyl group", for example, an acryloyl group, a crotonoyl group, etc. can be used.
As the "C₆₋₁₀ aryl-carbonyloxy group", for example, a benzoyloxy group, a naphthoyloxy group, etc. can be used.
As the "C₂₋₆ alkanoyloxy group", for example, an acetoxy group, a propionyloxy group, a butyryloxy group, a valeryloxy group, a pivaloyloxy group, etc. can be used.
As the "C₃₋₅ alkenoyloxy group", for example, an acryloyloxy group, a crotonoyloxy group, etc. can be used.
As the "carbamoyl group optionally having substituent(s)", for example, a carbamoyl group or a cyclic amino (e.g., pyrrolidinyl, piperidinyl, piperazinyl, morpholinyl, etc.)-carbonyl group, which may be substituted by 1 or 2 substituents selected from C₁₋₄ alkyl (e.g., methyl, ethyl, etc.), phenyl, C₁₋₇ acyl (e.g., acetyl, propionyl, benzoyl, etc.), C₁₋₄ alkoxy-phenyl (e.g., methoxyphenyl, etc.), etc., and the like can be used, and specifically, for example, a carbamoyl group, an N-methylcarbamoyl group, an N-ethylcarbamoyl group, an N,N-dimethylcarbamoyl group, an N,N-diethylcarbamoyl group, an N-phenylcarbamoyl group, an N-acetylcarbamoyl group, an N-benzoylcarbamoyl group, an N-(p-methoxyphenyl)carbamoyl group, a 1-pyrrolidinylcarbonyl group, a piperidinocarbonyl group, a 1-piperazinylcarbonyl group, a morpholinocarbonyl group, etc. can be used.
As the "thiocarbamoyl group optionally having substituent(s)", for example, a thiocarbamoyl group which may be substituted by 1 or 2 substituents selected from C₁₋₄ alkyl (e.g., methyl, ethyl, etc.), phenyl, etc. can be used, and specifically, for example, a thiocarbamoyl group, an N-methylthiocarbamoyl group, an N-phenylthiocarbamoyl group, etc. can be used.
As the "carbamoyloxy group optionally having substituent(s)", for example, a carbamoyloxy group which may be substituted by 1 or 2 substituents selected from C₁₋₄ alkyl (e.g., methyl, ethyl, etc.), phenyl, etc. can be used, and specifically, for example, a carbamoyloxy group, an N-methylcarbamoyloxy group, an N,N-dimethylcarbamoyloxy group, an N-ethylcarbamoyloxy group, an N-phenylcarbamoyloxy group, etc. can be used.
As the "C₁₋₆ alkanoylamino group", for example, an acetamido group, a propionamido group, a butyramido group, a valeramido group, a pivalamido group, etc. can be used.
As the "C₆₋₁₀ aryl-carbonylamino group", for example, a benzamido group, a naphthamido group, a phthalimido group, etc. can be used.
As the "C₁₋₁₀ alkoxy-carboxamido group", for example, a methoxycarboxamido (CH₃OCONH-) group, an ethoxycarboxamido group, a tert-butoxycarboxamido group, etc. can be used.
As the "C₆₋₁₀ aryloxy-carboxamido group", for example, a phenoxycarboxamido (C₆H₅OCONH-) group, etc. can be used.
As the "C₇₋₁₉ aralkyloxy-carboxamido group", for example, a benzyloxycarboxamido (C₆H₅CH₂OCONH-) group, a benzhydryloxycarboxamido group, etc. can be used.
As the "C₁₋₁₀ alkoxy-carbonyloxy group", for example, a methoxycarbonyloxy group, an ethoxycarbonyloxy group, an n-propoxycarbonyloxy group, an isopropoxycarbonyloxy group, an n-butoxycarbonyloxy group, a tert-butoxycarbonyloxy group, an n-pentyloxycarbonyloxy group, an n-hexyloxycarbonyloxy group, etc. can be used.
As the "C₆₋₁₀-aryloxy-carbonyloxy group", for example, a phenoxycarbonyloxy group, a naphthyloxycarbonyloxy group, etc. can be used.
As the "C₇₋₁₉ aralkyloxy-carbonyloxy group", for example, a benzyloxycarbonyloxy group, a 1-phenylethyloxycarbonyloxy group, a 2-phenylethyloxycarbonyloxy group, a benzhydryloxycarbonyloxy group, etc. can be used.
As the "C₃₋₁₀ cycloalkyloxy-carbonyloxy group", for example, a cyclopropyloxycarbonyloxy group, a cyclohexyloxycarbonyloxy group, etc. can be used.
As the "ureido group optionally having substituent(s)", for example, a ureido group optionally substituted by 1 to 3 (preferably 1 or 2) substituents selected from a C₁₋₄ alkyl group (e.g., a methyl group, an ethyl group, etc.), a phenyl group, etc. can be used, and, for example, a ureido group, a 1-methylureido group, a 3-methylureido group, a 3,3-dimethylureido group, a 1,3-dimethylureido group, a 3-phenylureido group, etc. can be used.

When a heterocyclic group, a heterocyclyloxy group, a heterocyclylthio group, a heterocyclylsulfinyl group, a heterocyclylsulfonyl group or a heterocyclyloxy-carbonyl group is used as the "substituents" of the "aliphatic hydrocarbon group" of the "aliphatic hydrocarbon group optionally having substituent(s)", the heterocyclic group is a group formed by excluding one hydrogen atom that binds to the heterocycle. It is, for example, a 5- to 8-membered ring (preferably a 5- or 6-membered ring) group containing 1 to several, preferably 1 to 4 hetero atoms such as a nitrogen atom (optionally oxidized), an oxygen atom, a sulfur atom, etc., or its condensed cyclic group. As these heterocyclic groups, for example, a pyrrolyl group, a pyrazolyl group, an imidazolyl group, a 1,2,3-triazolyl group, a 1,2,4-triazolyl group, a tetrazolyl group, a furyl group, a thienyl group, an oxazolyl group, an isoxazolyl group, a 1,2,3-oxadiazolyl group, a 1,2,4-oxadiazolyl group, a 1,2,5-oxadiazolyl group, a 1,3,4-oxadiazolyl group, a thiazolyl group, an isothiazolyl group, a 1,2,3-thiadiazolyl group, a 1,2,4-thiadiazolyl group, a 1,2,5-thiadiazolyl group, a 1,3,4-thiadiazolyl group, a pyridyl group, a pyridazinyl group, a pyrimidinyl group, a pyrazinyl group, an indolyl group, a pyranyl group, a thiopyranyl group, a dioxinyl group, a dioxolyl group, a quinolyl group, a pyrido[2,3-d]pyrimidyl group, a 1,5-, 1,6-, 1,7-, 1,8-, 2,6-or 2,7-naphthyridyl group, a thieno[2,3-d]pyridyl group, a benzopyranyl group, a tetrahydrofuryl group, a tetrahydropyranyl group, a dioxolanyl group, a dioxanyl group, etc. can be used.
These heterocyclic groups may be substituted at substitutable positions by 1 to 3 substituents selected from a C₁₋₄ alkyl (e.g., methyl, ethyl, etc.), a hydroxy, an oxo, a C₁₋₄ alkoxy (e.g., methoxy, ethoxy, etc.), and the like.
As the "C₆₋₁₀ aryl group" of the "C₆₋₁₀ aryl group optionally having substituent(s)", for example, a phenyl group, a naphthyl group, etc. can be used. The C₆₋₁₀ aryl group may be substituted at a substitutable position by a substituent selected from those exemplified as the "substituent" (except for a C₆₋₁₀ aryl group optionally having substituent(s)) of the "aliphatic hydrocarbon group optionally having substituent(s)" described above. Such substituent is not limited to a single substituent, but the same or different, more than one (preferably 2 to 4) substituents may be used.
In the "aliphatic hydrocarbon group optionally having substituent(s)", the substituent may form, together with the aliphatic hydrocarbon group, an optionally substituted fused ring group, and as such fused ring group, an indanyl group, a 1,2,3,4-tetrahydronaphthyl group, etc. can be used. This fused ring group may be substituted at a substitutable position by a substituent selected from those exemplified as the "substituent" of the "aliphatic hydrocarbon group optionally having substituent(s)" described above. Such substituent substitutes at a substitutable position of the fused ring group, wherein the substituent is not limited to a single substituent, but the same or different, more than one (preferably 2 to 4) substituents may be used.

X⁴ is a divalent aliphatic hydrocarbon group optionally having substituent(s).
As the "divalent aliphatic hydrocarbon group" of the "divalent aliphatic hydrocarbon group optionally having substituent(s)" for X⁴, for example, alkylene, alkenylene, alkynylene and the like are used. Preferred is a divalent aliphatic hydrocarbon group having a carbon number of 1 to 20, more preferably 1 to 6, more preferably:
(1) C₁₋₂₀ alkylene (preferably C₁₋₆ alkylene, for example, -CH₂-, -(CH₂)₂-, -(CH₂)₃-, -(CH₂)₄-, -(CH₂)₅-, -(CH₂)₆-, -CH(CH₃) -, - C(CH₃)₂-, -(CH(CH₃))₂-, -(CH₂)₂C(CH₃)₂-, -(CH₂)₃C(CH₃)₂-, - CH(CH₂CH₂CH₃)- and the like);
(2) C₂₋₂₀ alkenylene (preferably C₂₋₆ alkenylene, for example, - CH=CH-, -CH₂-CH=CH-, -C(CH₃)₂-CH=CH-, -CH₂-CH=CH-CH₂-, -CH₂-CH₂-CH=CH-, -CH=CH-CH=CH-, -CH=CH-CH₂-CH₂-CH₂- and the like); and (3) C₂₋₂₀ alkynylene (preferably C₂₋₆ alkynylene, for example, -C ≡C-, -CH₂-C≡C-, -CH₂-C≡C-CH₂-CH₂- and the like); particularly preferred are C₁₋₆ alkylene and C₂₋₆ alkenylene.
The "divalent aliphatic hydrocarbon group" may have a substituent, and as the substituent, for example, those exemplified as the substituent optionally possessed by the "aliphatic hydrocarbon group" of the aforementioned "aliphatic hydrocarbon group optionally having substituent(s)" can be mentioned.
These substituents are present at substitutable positions of the "divalent aliphatic hydrocarbon group". The number of the substituents is not limited to one, and may be plural (preferably 2 to 4), where the substituents may be the same or different.

R³ in "-NR³-" for X³ is optionally bonded to X⁴ to form a 5- to 8-membered ring optionally having substituent(s). Here, R³ is bonded to any bondable position in the "divalent aliphatic hydrocarbon group" of the "divalent aliphatic hydrocarbon group optionally having substituent(s)" for X⁴, and forms the ring structure together with the nitrogen atom adjacent to R³.
The "5- to 8-membered ring" of the "5- to 8-membered ring optionally having substituent(s)" formed by R³ bonded to X⁴ may be a saturated or unsaturated (preferably saturated) 5- to 8-membered (preferably 5- or 6-membered, more preferably 5-membered) nitrogen-containing heterocycle. Specifically,

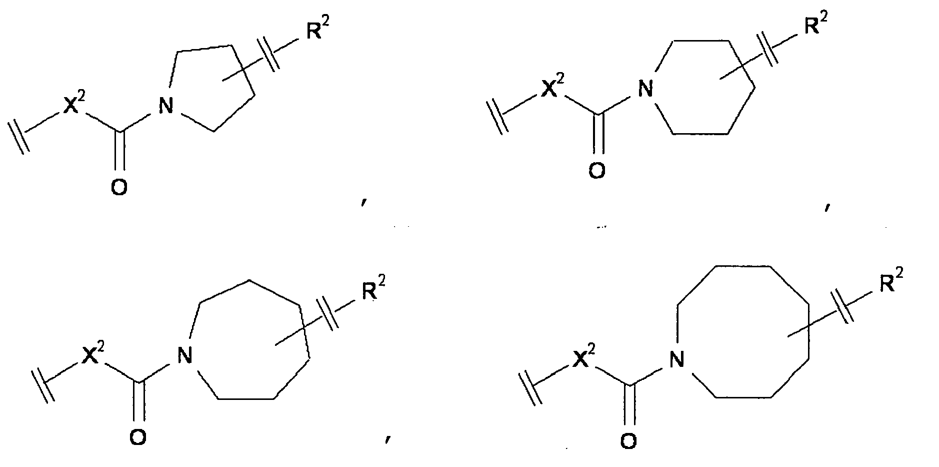

can be mentioned.
As the substituent that the "5- to 8-membered ring" may have, those exemplified as the substituents that the "aliphatic hydrocarbon group" of the aforementioned "aliphatic hydrocarbon group optionally having substituent(s)" may have can be mentioned. These substituents are present at substitutable positions of the "5- to 8-membered ring". The number of the substituents is not limited to one, and may be plural (preferably 2 to 4), where the substituents may be the same or different.

R⁴ in "-NHR⁴ group" for R² is optionally bonded to X⁴ to form a 5- to 8-membered ring optionally having substituent(s). Here, R⁴ is bonded to any bondable position in the "divalent aliphatic hydrocarbon group" of the "divalent aliphatic hydrocarbon group optionally having substituent(s)" for X⁴, and forms the ring structure together with the nitrogen atom adjacent to R⁴.
The "5- to 8-membered ring" of the "5- to 8-membered ring optionally having substituent(s)" formed by R⁴ bonded to X⁴ may be a saturated or unsaturated (preferably saturated) 5- to 8-membered (preferably 5- or 6-membered) nitrogen-containing heterocycle. Specifically,

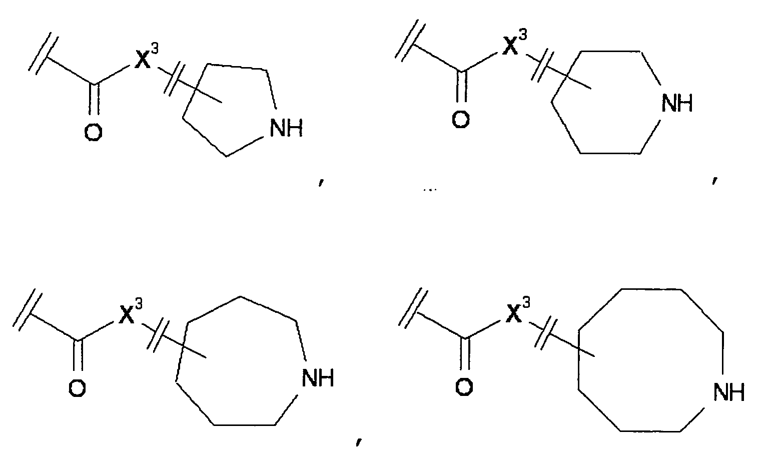

can be mentioned.
As the substituent that the "5- to 8-membered ring" may have, those exemplified as the substituents that the "aliphatic hydrocarbon group" of the aforementioned "aliphatic hydrocarbon group optionally having substituent(s)" may have can be mentioned. These substituents are present at substitutable positions (except nitrogen atom) of the "5- to 8-membered ring". The number of the substituents is not limited to one, and may be plural (preferably 2 to 4), where the substituents may be the same or different.

X¹ is preferably a methylene group.
The "C₁₋₆ alkyl group" for R¹ is preferably an ethyl group.
The "phenyl group optionally having one or the same or different two halogen atoms" for ring A is preferably a group represented by the formula:

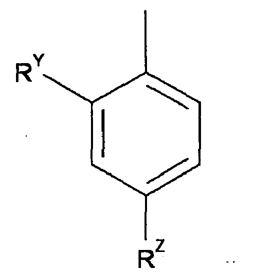

wherein R^{Y} and R^{Z} are the same or different and each is a halogen atom. More preferably, R^{Y} is a chlorine atom and R^{Z} is a fluorine atom in the above-mentioned formula.

When X² is "-CHR^{x}-O-", R^{x} is preferably a hydrogen atom (i.e., X² is preferably "-CH₂-O-").
When X² is a group represented by the formula:

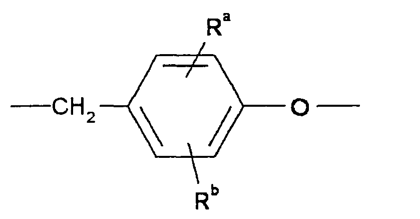

wherein each symbol is as defined above, the group represented by the above-mentioned formula is preferably a group represented by the following formula:

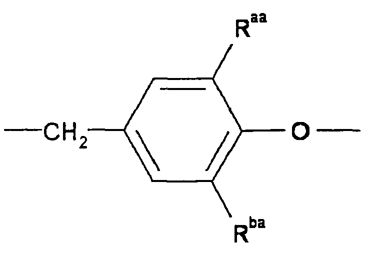

wherein each symbol is as defined above. More preferably, R^{aa} and R^{ba} are each a methyl group in the above-mentioned formula.

When X³ is "-NR³-", the "aliphatic hydrocarbon group optionally having substituent(s)" for R³ is preferably a "C₁₋₆ alkyl group optionally having substituent(s)". As the substituents that the "C₁₋₆ alkyl group" optionally has, those exemplified as the substituents that the "aliphatic hydrocarbon group" of the aforementioned "aliphatic hydrocarbon group optionally having substituent(s)" may have can be mentioned. These substituents are present at substitutable positions of the "C₁₋₆ alkyl group". The number of the substituents is not limited to one, and may be plural (preferably 2 to 4), where the substituents may be the same or different.

The "divalent aliphatic hydrocarbon group optionally having substituent(s)" for X⁴ is preferably C₁₋₂₀ alkylene, more preferably C₁₋₆ alkylene [for example, -CH₂-, - (CH₂)₂-, - (CH₂)₃-, -(CH₂)₄-, -(CH₂)₅-, -(CH₂)₆-, -CH(CH₃)-, -C(CH₃)₂-, -(CH(CH₃))₂-, -(CH₂)₂C(CH₃)₂-, -(CH₂)₃C(CH₃)₂-, -CH(CH₂CH₂CH₃) - and the like].

When R² is "-NHR⁴", the "aliphatic hydrocarbon group optionally having substituent(s)" for R⁴ is preferably a "C₁₋₆ alkyl group optionally having substituent(s)". As the substituent that the "C₁₋₆ alkyl group" optionally has, those exemplified as the substituents that the "aliphatic hydrocarbon group" of the aforementioned "aliphatic hydrocarbon group optionally having substituent(s)" may have can be used.

X² and X³ are preferably a combination of any of the following (a) to (c).
(a) X² is a -CH₂-O- group and X³ is -O- or -NR^{3a}-wherein R^{3a} is a hydrogen atom or a C₁₋₆ alkyl group optionally having substituent(s), or R^{3a} is optionally bonded to X⁴ to form a 5- to 8-membered ring optionally having substituent(s). As the "5- to 8-membered ring optionally having substituent(s)" formed by R^{3a} bonded to X⁴, those similar to the aforementioned "5- to 8-membered ring optionally having substituent(s)" formed by R³ bonded to X⁴ can be mentioned. More preferably, when X³ is -O-, X⁴ is -CH₂-, -(CH₂)₂-, -(CH₂)₃-, -(CH₂)₄-, - (CH₂)₅- or -(CH₂)₆-;
   when X³ is -NR^{3a}-, R^{3a} is a hydrogen atom or an optionally substituted C₁₋₆ alkyl group, or R^{3a} is bonded to X⁴ to form a 5-membered ring optionally having substituent(s).
(b) X² is a group represented by the formula:

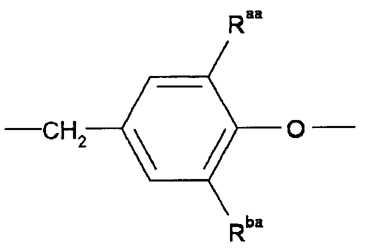

and X³ is a bond, wherein R^{aa} and R^{ba} are the same or different and each is a C₁₋₆ alkyl group.
(c) X² and X³ are each a bond.

R² is preferably -COOH or a -NHR^{4a} group, wherein R^{4a} is a C₁₋₆ alkyl group optionally having substituent(s), or R^{4a} is optionally bonded to X⁴ to form a 5- to 8-membered ring optionally having substituent(s). As the "5- to 8-membered ring optionally having substituent(s)" formed by R^{4a} bonded to X⁴, those similar to the aforementioned "5- to 8-membered ring optionally having substituent(s)" formed by R⁴ bonded to X⁴ can be mentioned.

Preferable examples of the compound represented by the formula (I) include the following compounds (A) to (C), and compounds (A) and (C) are preferable:

### (Compound A)

A compound of the formula (I), wherein
X¹ is a methylene group;
R¹ is an ethyl group;
ring A is a group represented by the formula:

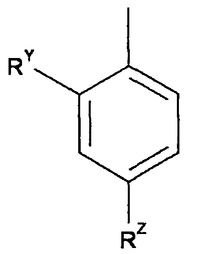

wherein R^{Y} and R^{Z} are the same or different and each is a halogen atom;
X² is a -CH₂-O- group;
X³ is -O- or -NR^{3a}- wherein R^{3a} is a hydrogen atom or an optionally substituted C₁₋₆ alkyl group, or R^{3a} is optionally bonded to X⁴ to form a 5- to 8-membered ring optionally having substituent(s); and
X⁴ is an optionally substituted C₁₋₆ alkylene group [more preferably, when X³ is -O-, X⁴ is -CH₂-, -(CH₂)₂-, -(CH₂)₃-, -(CH₂)₄-, -(CH₂)₅- or -(CH₂)₆-;
   when X³ is -NR^{3a}-, R^{3a} is a hydrogen atom or an optionally substituted C₁₋₆ alkyl group, or R^{3a} is bonded to X⁴ to form a 5-membered ring optionally having substituent(s); and
R² is -COOH.

### (Compound B)

A compound of the formula (I), wherein
X¹ is a methylene group;
R¹ is an ethyl group;
ring A is a group represented by the formula:

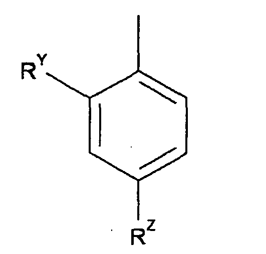

wherein R^{Y} and R^{Z} are the same or different and each is a halogen atom;
X² is a group represented by the formula:

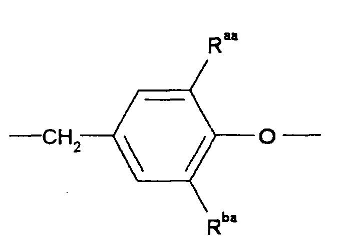

wherein R^{aa} and R^{ba} are the same or different and each is a C₁₋₆ alkyl group;
X³ is a bond;
X⁴ is an optionally substituted C₁₋₆ alkylene group;
R² is a -NHR^{4a} group wherein R^{4a} is a C₁₋₆ alkyl group optionally having substituent(s), or R^{4a} is optionally bonded to X⁴ to form a 5- to 8-membered ring optionally having substituent(s).

### (Compound C)

A compound of the formula (I), wherein
X¹ is a methylene group;
R¹ is an ethyl group;
ring A is a group represented by the formula:

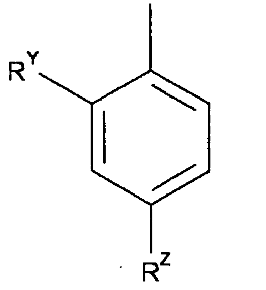

wherein R^{Y} and R^{Z} are the same or different and each is a halogen atom;
X² and X³ are each a bond;
X⁴ is an optionally substituted C₁₋₆ alkylene group; and
R² is -COOH.
In the above-mentioned compounds (A) and (C), a compound wherein R^{Y}=Cl and R^{Z}=F is more preferable, and a compound wherein R^{Y}=Cl, R^{Z}=F, and the steric configuration at the substitutable position (the 6-position of cyclohexene ring) of a sulfonamide group on the cyclohexene ring including X¹ being R configuration is still more preferable.

In addition, as a compound represented by the formula (I), the following compounds (D) to (F) can also be used:

### (Compound D)

A compound of the formula (I), wherein
X¹ is -O-;
R¹ is an ethyl group;
ring A is a group represented by the formula:

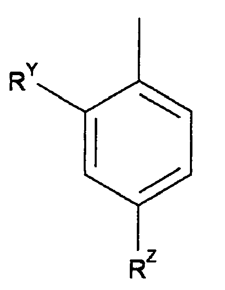

wherein R^{Y} and R^{Z} are the same or different and each is a halogen atom;
X² is a -CH₂-O- group;
X³ is -O- or -NR^{3a}- wherein R^{3a} is a hydrogen atom or an optionally substituted C₁₋₆ alkyl group, or R^{3a} is optionally bonded to X⁴ to form a 5- to 8-membered ring optionally having substituent(s);
X⁴ is an optionally substituted C₁₋₆ alkylene group [more preferably, when X³ is -O-, X⁴ is -CH₂-, -(CH₂)₂-, -(CH₂)₂-, -(CH₂)₄-, -(CH₂)₅- or -(CH₂)₆-;
   when X³ is -NR^{3a}-, R^{3a} is a hydrogen atom or an optionally substituted C₁₋₆ alkyl group, or R^{3a} is bonded to X⁴ to form a 5-membered ring optionally having substituent(s); and
R² is -COOH.

### (Compound E)

A compound of the formula (I), wherein
X¹ is -O-;
R¹ is an ethyl group;
ring A is a group represented by the formula:

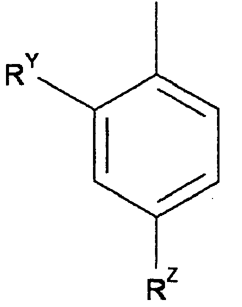

wherein R^{Y} and R^{Z} are the same or different and each is a halogen atom;
X² is a group represented by the formula:

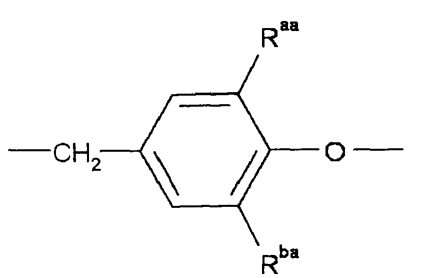

wherein R^{aa} and R^{ba} are the same or different and each is a C₁₋₆ alkyl group;
X³ is a bond;
X⁴ is an optionally substituted C₁₋₆ alkylene group; and
R² is a -NHR^{4a} group wherein R^{4a} is a C₁₋₆ alkyl group optionally having substituent(s), or R^{4a} is optionally bonded to X⁴ to form a 5- to 8-membered ring optionally having substituent(s).

### (Compound F)

A compound of the formula (I), wherein
X¹ is -O-;
R¹ is an ethyl group;
ring A is a group represented by the formula:

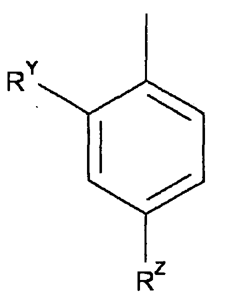

wherein R^{Y} and R^{Z} are the same or different and each is a halogen atom;
X² and X³ are each a bond;
X⁴ is an optionally substituted C₁₋₆ alkylene group; and
R² is -COOH.

In the above-mentioned compounds (D) to (F), a compound wherein R^{Y}=Cl and R^{Z}=F, or a compound wherein R^{Y}=F and R^{Z}=F is preferable. In addition thereto, an optically active form wherein the steric configuration at the substitutable position (the 6-position of cyclohexene ring) of a sulfonamide group on the cyclohexene ring including X¹ is R configuration or S configuration is still more preferable

As the salt of a compound represented by the formula (I), a salt with an inorganic base, organic base, inorganic acid, organic acid, basic amino acid, acidic amino acid, and the like can be used.
As the salt with an inorganic base, for example, an alkaline metal salt such as sodium and potassium salts, etc.; an alkaline earth metal salt such as calcium and magnesium salts, etc.; aluminum salt; ammonium salt; and the like are used.
As the salt with an organic base, for example, a salt with trimethylamine, triethylamine, pyridine, picoline, ethanolamine, diethanolamine, triethanolamine, dicyclohexylamine, N,N'-dibenzylethylenediamine, etc can be used.
As the salt with an inorganic acid, for example, a salt with hydrochloric acid, hydrobromic acid, nitric acid, sulfuric acid, phosphoric acid, etc can be used.
As the salt with an organic acid, for example, a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like can be used.
As the salt with a basic amino acid, for example, a salt with arginine, lysine, ornithine, etc can be used.
As the salt with acidic amino acid, for example, a salt with aspartic acid, glutamic acid, and the like can be used.

When the compound represented by the formula (I) or a salt thereof includes stereoisomers, both the isomers alone and mixtures of such isomers are encompassed in the scope of the present invention. Particularly, the compound represented by the formula (1) includes optical isomers based on the asymmetric carbon in the cyclohexene ring containing X¹. Such optical isomers and mixtures of such optical isomers are all encompassed in the scope of the present invention.
Moreover, when the compound represented by the formula (I) or a salt thereof includes optical isomers, both the optical isomers and mixtures of the isomers are encompassed in the scope of the present invention.

A compound represented by the formula (I) or a salt thereof can be produced, for example, by the production methods detailed in the following, or a method analogous thereto.
The compound to be used as a starting material compound in the following production methods may be in the form of a salt. Examples of such salt include those exemplified as the salts of the aforementioned compound represented by the formula (I).
In each of the following production methods, when alkylation reaction, hydrolysis, amination reaction, esterification reaction, amidation reaction, etherification reaction, oxidation reaction, reduction reaction and the like are to be performed, these reactions are performed according to a method known per se. Examples of such method include the methods described in ORGANIC FUNCTIONAL GROUP PREPARATIONS, 2nd edition, ACADEMIC PRESS, INC., 1989; Comprehensive Organic Transformations, VCH Publishers Inc., 1989, and the like, and the like.

### [Production methods]

A compound represented by the formula (I):

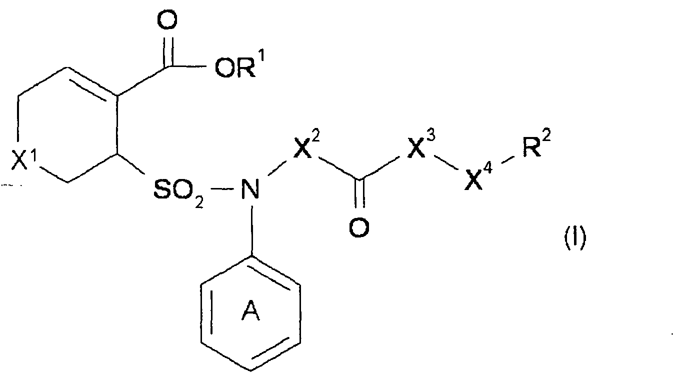

wherein each symbol is as defined above, or a salt thereof [compound (I)] can be produced, for example, by condensing a compound represented by the formula (II):

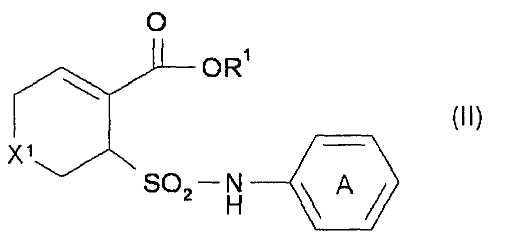

wherein each symbol is as defined above or a salt thereof [hereinafter to be abbreviated as compound (II)] with a compound represented by the formula (III):

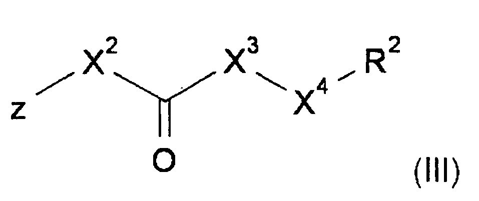

wherein Z is a halogen atom, and other symbols are as defined above, or a salt thereof [hereinafter to be abbreviated as compound (III)].
The above-mentioned production method is performed, for example, reacting compound (II) with compound (III) in the presence of a base. Specifically, for example, a base is added to a mixed solution of compound (II) and compound (III) and the mixture is stirred.
The reaction of the above-mentioned production method is generally performed in a solvent, where a solvent that does not inhibit the above-mentioned reaction is appropriately selected. Examples of such solvent include alcohols (e.g., methanol, ethanol, propanol, isopropanol, butanol, tert-butanol etc.), ethers (e.g., dioxane, tetrahydrofuran, diethyl ether, tert-butyl methyl ether, diisopropyl ether, ethylene glycol, dimethyl ether etc.), esters (e.g., ethyl formate, ethyl acetate, n-butyl acetate etc.), halogenated hydrocarbons (e.g., dichloromethane, chloroform, carbon tetrachloride, trichloroethylene, 1,2-dichloroethane etc.), hydrocarbons (e.g., n-hexane, benzene, toluene etc.), amides (e.g., formamide, N,N-dimethylformamide, N,N-dimethylacetamide etc.), ketones (e.g., acetone, methyl ethyl ketone, methyl isobutyl ketone etc.), nitriles (e.g., acetonitrile, propionitrile etc.) and the like, dimethyl sulfoxide, sulfolane, hexamethyl phosphoramide, water and the like. These are used alone or in the form of a mixed solvent. The amount of the solvent to be used is not particularly limited as long as a reaction mixture can be stirred therein. The solvent is used in an amount of generally 2- to 100-fold weight relative to compound (II) or a salt thereof.
Examples of the base to be used in the above-mentioned production method include inorganic bases such as C₁₋₆ alkyllithium and C₆₋₁₀ aryllithium (e.g., methyllithium, ethyllithium, n-butyllithium, sec-butyllithium, tert-butyllithium, phenyllithium etc.), C₂₋₆ lithium alkylamide (e.g., lithium dimethylamide, lithium diethylamide, lithium diisopropylamide etc.), metal hydride (e.g., lithium hydride, sodium hydride etc.), alkali metal C₁₋₆ alkoxide (e.g., lithium ethoxide, lithium-tert-butoxide, sodium methoxide, sodium ethoxide, potassium-tert-butoxide etc.), alkali metal amide (e.g., lithium amide, potassium amide, sodium amide etc.), alkali metal hydroxide (e.g., lithium hydroxide, potassium hydroxide, sodium hydroxide etc.), carbonate and bicarbonate of alkali metal (e.g., sodium carbonate, potassium carbonate, cesium carbonate, sodium hydrogen carbonate etc.) and the like; organic bases such as tertiary amines such as triethylamine, tri(n-propyl)amine, tri(n-butyl)amine, diisopropylethylamine, cyclohexyldimethylamine, pyridine, 4-(dimethylamino)pyridine, lutidine, γ-collidine, N,N-dimethylaniline, N-methylpiperidine, N-methylpyrrolidine, N-methylmorpholine and the like; and the like. The lower limit of the amount of the base to be used is generally not less than 0.1 mol, preferably not less than 0.5 mol, more preferably not less than 1 mol, and the upper limit thereof is generally not more than 10 mol, preferably not more than 5 mol, both per 1 mol of compound (II) or a salt thereof.
In the above-mentioned production method, compound (III) can be used in an amount of generally 1 mol to 5 mol, preferably 1 mol to 3 mol, per 1 mol of compound (II) or a salt thereof.
The reaction in the above-mentioned production method is generally performed at about -80°C to 100°C, preferably 0°C to 60°C, and completes generally in 1 min to 72 hr, preferably 15 min to 24 hr, though subject to variation depending on the kind of compounds (II) and (III) and the solvent, reaction temperature and the like.
Of the compounds represented by the formula (II), which are the starting compounds for the above-mentioned production methods, a compound wherein X¹ is a methylene group can be produced according to a method known *per* se, for example, a production method described in WO99/46242 or a method analogous thereto. In addition, a compound represented by the formula (II) wherein X¹ is an oxygen atom can be produced according to a production method described in WO01/10826 or a method analogous thereto.
Here, as compound (II), ethyl 6-[N-(2-chloro-4-fluorophenyl)sulfamoyl]cyclohex-1-ene-1-carboxylate is preferable, and ethyl (6R)-6-[N-(2-chloro-4-fluorophenyl)sulfamoyl]cyclohex-1-ene-1-carboxylate is more preferable.
Besides these, as compound (II), ethyl 3-[(2-chloro-4-fluorophenyl)sulfamoyl]-3,6-dihydro-2H-pyran-4-carboxylate, ethyl 3-[(2,4-difluorophenyl)sulfamoyl]-3,6-dihydro-2H-pyran-4-carboxylate, as well as an R form or an S form at the 6-position of the 3,6-dihydro-2H-pyran ring therein can also be used.
A compound of the formula (III), which is a starting material compound of the above-mentioned production method, can be produced by a method known per se (e.g., the production method described in J. Med. Chem., 37, 4423-4429, 1994; Cancer Research, 37, 619-624, 1977) or a method analogous thereto.

In the thus-obtained compound, a functional group in a molecule can also be converted to a desired functional group by a combination of chemical reactions known *per* se. Examples of the chemical reaction include oxidation reaction, reduction reaction, alkylation reaction, hydrolysis, amination reaction, esterification reaction, aryl coupling reaction, deprotection and the like.
In the aforementioned production methods, when the starting compound has an amino group, a carboxyl group, a hydroxy group or a carbonyl group as a substituent, a protecting group generally used in peptide chemistry and the like may be introduced into these groups. By removing the protecting group as necessary after the reaction, the objective compound can be obtained.
Examples of the amino-protecting group include formyl group, C₁₋₆ alkyl-carbonyl group, C₁₋₆ alkoxy-carbonyl group, benzoyl group, C₇₋₁₀ aralkyl-carbonyl group (e.g., benzylcarbonyl), C₇₋₁₄ aralkyloxy-carbonyl group (e.g., benzyloxycarbonyl, 9-fluorenylmethoxycarbonyl), trityl group, phthaloyl group, N,N-dimethylaminomethylene group, substituted silyl group (e.g., trimethylsilyl, triethylsilyl, dimethylphenylsilyl, tert-butyldimethylsilyl, tert-butyldiethylsilyl), C₂₋₆ alkenyl group (e.g., 1-allyl) and the like. These groups are optionally substituted by 1 to 3 substituents selected from a halogen atom, a C₁₋₆ alkoxy group and a nitro group.
Examples of the carboxy-protecting group include C₁₋₆ alkyl group, C₇₋₁₁ aralkyl group (e.g., benzyl), phenyl group, trityl group, substituted silyl group (e.g., trimethylsilyl, triethylsilyl, dimethylphenylsilyl, tert-butyldimethylsilyl, tert-butyldiethylsilyl), C₂₋₆ alkenyl group (e.g., 1-allyl) and the like. These groups are optionally substituted by 1 to 3 substituents selected from a halogen atom, a C₁₋₆ alkoxy group and a nitro group.
Examples of the hydroxy-protecting group include C₁₋₆ alkyl group, phenyl group, trityl group, C₇₋₁₀ aralkyl group (e.g., benzyl), formyl group, C₁₋₆ alkyl-carbonyl group, benzoyl group, C₇₋₁₀ aralkyl-carbonyl group (e.g., benzylcarbonyl), 2-tetrahydropyranyl group, 2-tetrahydrofuranyl group, substituted silyl (e.g., trimethylsilyl, triethylsilyl, dimethylphenylsilyl, tert-butyldimethylsilyl, tert-butyldiethylsilyl), C₂₋₆ alkenyl group (e.g., 1-allyl) and the like. These groups are optionally substituted by 1 to 3 substituents selected from a halogen atom, a C₁₋₆ alkyl group, C₁₋₆ alkoxy group and a nitro group.
Examples of the carbonyl-protecting group include cyclic acetal (e.g., 1,3-dioxane), acyclic acetal (e.g., di-C₁₋₆ alkylacetal) and the like.
The above-mentioned protecting groups can be removed according to a method known per se, for example, the method described in Protective Groups in Organic Synthesis, John Wiley and Sons (1980) and the like. Specifically, methods using acid, base, ultraviolet rays, hydrazine, phenylhydrazine, sodium N-methyl dithiocarbamate, tetrabutylammonium fluoride, palladium acetate, trialkylsilyl halide (e.g., trimethylsilyl iodide, trimethylsilyl bromide and the like) and the like, reduction method and the like are used.
In the above-mentioned production methods, the starting material compound may be in the form of a salt. Examples of the salt include those similar to the salts of the aforementioned compound represented by the formula (I).
Compound (I) obtained by the above-mentioned production methods can be isolated and purified by a known method, for example, solvent extraction, liquid conversion, phase transfer, crystallization, recrystallization, chromatography and the like.

When the optically active compound or a salt thereof contains an enantiomer, general separation means may be applied such as diastereomeric salt methods wherein a salt with an optically active acid (e.g., camphorsulfonic acid etc.) or optically active base (e.g., 1-methylbenzylamine etc.) is formed, inclusion compound methods using an optically active host molecule (e.g., 1,6-bis(2-chlorophenyl)-1,6-diphenylhexa-2,4-diyn-1,6-diol), various chromatographies (e.g., liquid chromatography using a chiral column etc.), fractional recrystallization and the like, whereby an optically pure compound can be obtained.

The compound (I) may be a hydrate or an anhydrate.
In addition, compound (I) may be labeled with an isotope (e.g., ³H, ¹⁴C, ³⁵S, ¹²⁵I etc.) and the like.

Compound (I) of the present invention is low toxic and shows a nitric oxide (NO) production inhibitory action, and an inhibitory action on the production of inflammatory cytokines such as TNF-α, IL-1, IL-6 and the like, and can be useful as, for example, a nitric oxide (NO) production inhibitor, or an inhibitor of the production of inflammatory cytokines such as TNF-α, IL-1, IL-6 and the like for mammals (e.g., human, mouse, rat, rabbit, dog, cat, bovine, horse, swine, monkey etc.).
In addition, compound (I) of the present invention is highly safe for living organisms and can be used for mammals (e.g., human, mouse, rat, rabbit, dog, cat, bovine, horse, swine, monkey etc.) as a pharmaceutical agent (e.g., agent for the prophylaxis or treatment of various diseases involving nitric oxide (NO) production and/or inflammatory cytokine production), an animal drug and the like.
Specifically, compound (I) can be useful as an agent for the prophylaxis or treatment of sepsis, particularly severe sepsis, in mammals. Here, severe sepsis refers to a systemic inflammatory response syndrome caused by infection, which shows high levels of severity, satisfies at least two of the following items of, for example, body temperature: not less than 38°C or less than 36°C, cardiac rate: not less than 90 bpm/min, respiration rate: not less than 20 times/min, and leukocyte count: not less than 12000 leukocytes/mm³ or less than 4000 leukocytes/mm³, shows a disease state of hypotension (systolic blood pressure of not more than 90 mmHg), half consciousness, cryptic speech and action, urine per 1 hr of less than 0.5 mL/kg, platelets of less than 80000 platelets/mm³ and the like, and typically accompanies symptoms such as organ failure, hypoperfusion, hypotension and the like.

Compound (I) can be used as an agent for the prophylaxis or treatment of diseases in mammals such as cardiac disease, autoimmune disease, inflammatory disease, central nervous system diseases, infectious disease, septic shock, immune dysfunction and the like, including, for example, septicemia, endotoxin shock, exotoxin shock, systemic inflammatory response syndrome (SIRS), compensatory anti-inflammatory response syndrome (CARS), burn, trauma, post-operative complications, cardiac deficiency, shock, hypotension, rheumatoid arthritis, osteoarthritis, gastritis, ulcerative colitis, peptic ulcer, stress-induced gastric ulcer, Crohn's disease, autoimmune disease, post-transplant tissue failure and rejection, postischemic re-perfusion failure, acute coronary microvascular embolism, shock-induced vascular embolism (disseminated intravascular coagulation (DIC) etc.), ischemic cerebral disorder, arteriosclerosis, pernicious anemia, Fanconi's anemia, drepanocythemia, pancreatitis, nephrose syndrome, nephritis, renal failure, insulin-dependent diabetes, insulin-independent diabetes, hepatic porphyria, alcoholism, Parkinson's disease, chronic leukemia, acute leukemia, tumor, myeloma, infantile and adult respiratory distress syndrome, pulmonary emphysema, dementia, Alzheimer's disease, multiple sclerosis, vitamin E deficiency, aging, sunburn, muscular dystrophy, myocarditis, cardiomyopathy, myocardial infarction, myocardial post infarction syndrome, osteoporosis, pneumonia, hepatitis, psoriasis, pain, cataract, influenza infection, malaria, human immunodeficiency virus (HIV) infection, radiation hazard, burn, *in vitro* fertilization efficiency, hypercalcemia, tonic spondylitis, osteopenia, bone Paget's disease, osteomalacia, fracture, acute bacterial meningitis, Helicobacter pylori infection, invasive staphylococcal infection, tuberculosis, systemic mycosis, herpes simplex virus infection, varicella-zoster virus infection, human papilloma virus infection, acute viral encephalitis, encephalitis, meningitis, immune dysfunction due to infections, asthma, atopic dermatitis, allergic rhinitis, reflux esophargitis, fever, hyper cholesteremia, hyperglycemia, hyperlipidemia, diabetic complication, diabetic renal disease, diabetic neuropathy, diabetic retinopathy, gout, gastric atony, hemorrhoid, systemic lupus erythematosus, spinal damage, insomnia, schizophrenia, epilepsy, cirrhosis, hepatic failure, instable angina, valvular disease, dialysis-induced thrombocytopenia or hypotonia, acute ischemic cerebral apoplexy, acute cerebral thrombosis, cancer metastasis, urinary bladder cancer, mammary cancer, uterine cervical cancer, colon cancer, gastric cancer, ovarian cancer, prostatic cancer, parvicellular pulmonary cancer, non-parvicellular pulmonary cancer, malignant melanoma, Hodgkin's disease, non-Hodgkin lymphoma, side effects caused by administration of anticancer agents or immunosuppressants and the like. Moreover, compound (I) can also be useful as a therapeutic agent for severe sepsis patients accompanying the above-mentioned diseases.

Compound (I) can be used in combination with other drugs. Examples of such concomitant drug include antibacterial agents, antifungal agents, non-steroidal antiinflammatory drugs, steroids, anticoagulants, antithrombotic drugs, thrombolytic drugs, immunomodulators, antiprotozoals, antitussive and expectorant drugs, sedatives, anesthetics, antinarcotics, antiulcer drugs, hyperlipidemia treating agents, therapeutic agents for arteriosclerosis, HDL increasing agents, unstable plaque stabilizing agents, myocardial protecting agent, hypothyroidism treating agent, nephrotic syndrome treating agent, chronic renal failure treating agent, diuretics, hypertension treating agents, cardiac failure treating agents, muscle relaxants, anticonvulsants, cardiacs, vasodilators, vasoconstrictors, antiarrhythmics, antidiabetic drugs, hypertensors, tranquilizers, antipsychotics, therapeutic agents for Alzheimer's diseases, anti-Parkinson drugs, therapeutic agents for amyotrophic spinal lateral sclerosis, neurotrophic factors, antidepressants, therapeutic agents for schizophrenia, antitumor drugs, vitamins, vitamin derivatives, therapeutic agents for arthritis, antirheumatics, antiallergic drugs, antiasthmatics, therapeutic agents for atopic dermatitis, therapeutic agents for allergic rhinitis, therapeutic agents for pollakisuria/anischuria, protease drugs, protease inhibitors, anti-SIDS drugs, anti-sepsis drugs, antiseptic shock drugs, endotoxin-antagonists or -antibodies, signal transduction inhibitors, inhibitors of inflammatory mediator activity, antibodies to inhibit inflammatory mediator activity, inhibitors of inflammatory mediator production, inhibitors of anti-inflammatory mediator activity, antibodies to inhibit anti-inflammatory mediator activity, inhibitors of anti-inflammatory mediator production, α1-adrenergic agonists and the like. Among these, antibacterial agents, antifungal agents, non-steroidal antiinflammatory drugs, steroids, anticoagulants and the like are preferable.

Specifically, the following concomitant drugs can be recited.
(1) Antibacterial agents
   (i) Sulfa agents
      sulfamethizole, sulfisoxazole, sulfamonomethoxine, sulfamethizole, salazosulfapyridine, silver sulfadiazine and the like.
   (ii) Quinoline antibacterial agents
      nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin and the like.
   (iii) Antiphthisics
      isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like.
   (iv) Antiacidfast bacterium drugs
      diaphenylsulfone, rifampicin and the like.
   (v) Antiviral drugs
      idoxuridine, acyclovir, vidarabine, ganciclovir and the like.
   (vi) Anti-HIV agents
      zidovudine, didanosine, zalcitabine, indinavir sulfate ethanolate, ritonavir and the like.
   (vii) Antispirocheteles
   (viii) Antibiotics
      tetracycline hydrochloride, ampicillin, piperacillin, gentamicin, dibekacin, kanendomycin, lividomycin, tobramycin, amikacin, fradiomycin, sisomycin, tetracycline, oxytetracycline, rolitetracycline, doxycycline, piperacillin, ticarcillin, cephalothin, cephapirin, cephaloridine, cefaclor, cephalexin, cefroxadine, cefadroxil, cefamandole, cefotoam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt thereof, griseofulvin, lankacidin-group [Journal of Antibiotics (J. Antibiotics), 38, 877-885(1985)] and the like.
(2) Antifungal agents
   (i) polyethylene antibiotics (e.g., amphotericin B, nystatin, trichomycin)
   (ii) griseofulvin, pyrrolnitrin and the like.
   (iii) cytosine metabolism antagonists (e.g., flucytosine)
   (iv) imidazole derivatives (e.g., econazole, clotrimazole, miconazole nitrate, bifonazole, croconazole)
   (v) triazole derivatives (e.g. fluconazole, itraconazole, azole compound [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone]
   (vi) thiocarbamic acid derivatives (e.g. trinaphthol)
   (vii) echinocandin derivatives (e.g., caspofungin, micafungin, anidulafungin) and the like.
(3) Non-steroidal antiinflammatory drugs
   acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, epirizole, tiaramide hydrochloride, zaltoprofen, gabexate mesylate, camostat mesylate, urinastatin, colchicine, probenecid, sulfinpyrazone, benzbromarone, allopurinol, gold sodium thiomalate, sodium hyaluronate, sodium salicylate, morphine hydrochloride, salicylic acid, atropine, scopolamine, morphine, pethidine, levorphanol, ketoprofen, naproxen, oxymorphone or a salt thereof, and the like.
(4) Steroids
   dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclometasone propionate, estriol and the like.
(5) Anticoagulants
   heparin sodium, sodium citrate, activated protein C, tissue factor pathway inhibitor, antithrombin III, dalteparin sodium, warfarin potassium, argatroban, gabexate, sodium citrate and the like.

(6) Antithrombotic drugs
   ozagrel sodium, ethyl icosapentate, beraprost sodium, alprostadil, ticlopidine hydrochloride, pentoxifylline, dipyridamole and the like.
(7) Thrombolytic drugs tisokinase, urokinase, streptokinase and the like.
(8) Immunomodulators
   cyclosporin, tacrolimus, gusperimus, azathioprine, antilymphocyte serum, dried sulfonated immunoglobulin, erythropoietin, colony-stimulating factor, interleukin, interferon and the like.
(9) Antiprotozoals
   metronidazole, tinidazole, diethylcarbamazine citrate, quinine hydrochloride, quinine sulfate and the like.
(10) Antitussive and expectorant drugs
   ephedrine hydrochloride, noscapine hydrochloride, codeine phosphate, dihydrocodeine phosphate, isoproterenol hydrochloride, ephedrine hydrochloride, methylephedrine hydrochloride, noscapine hydrochloride, alloclamide, chlophedianol, picoperidamine, chloperastine, protokylol, isoproterenol, salbutamol, terbutaline, oximetebanol, morphine hydrochloride, dextromethorphan hydrobromide, oxycodone hydrochloride, dimemorphan phosphate, tipepidine hibenzate, pentoxyverine citrate, clofedanol hydrochloride, benzonatate, guaifenesin, bromhexine hydrochloride, ambroxol hydrochloride, acetylcysteine, ethyl cysteine hydrochloride, carbocysteine and the like.
(11) Sedatives
   chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium and the like.
(12) Anesthetics
   (12-1) Local anesthetics
      cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine and the like.
   (12-2) General anesthetics
      (i) inhalation anesthetics (e.g., ether, halothane, nitrous oxide, influrane, enflurane),
      (ii) intravenous anesthetics (e.g., ketamine hydrochloride, droperidol, thiopental sodium, thiamylal sodium, pentobarbital) and the like.
(13) Antinarcotics
   levallorphan, nalorphine, naloxone or a salt thereof and the like.
(14) Antiulcer drugs
   metoclopromide, histidine hydrochloride, lansoprazole, metoclopramide, pirenzepine, cimetidine, ranitidine, famotidine, urogastrone, oxethazaine, proglumide, omeprazole, sucralfate, sulpiride, cetraxate, gefarnate, aldioxa, teprenone, prostaglandin and the like.

(15) Hyperlipidemia treating agents
   HMG-CoA reductase inhibitors (e.g., fluvastatin, cerivastatin, atorvastatin etc.), fibrates (e.g., simfibrate, clofibrate aluminum, clinofibrate, fenofibrate etc.), adsorbents for bile acid (e.g., cholestyramine etc.), nicotinic acid formulations (e.g., nicomol, niceritrol, tocopherol nicotinate etc.), probucol and a derivative thereof, polyvalent unsaturated fatty acid derivatives (e.g., ethyl icosapentate, polyene phosphatidylcholine, melinamide etc.), vegetable sterols (e.g., gamma-oryzanol, soysterol etc.), elastases, sodium dextran sulfate, squalene synthetase inhibitor, squalene epoxidase inhibitor, CETP inhibitor, ethyl 2-chloro-3-[4-(2-methyl-2-phenylpropoxy)phenyl]propionate [chemical and pharmaceutical bulletin (Chem. Pharm. Bull.), 38, 2792, 2796 (1990)], LDL receptor enhancing drug, cholesterol absorption inhibitor (Ezetimibe etc.), MTP inhibitor, ileal bile acid transporter inhibitor, SCAP ligand, FXR ligand and the like.
(16) Therapeutic agents for arteriosclerosis
   MMP inhibitor, chymase inhibitor, ACAT inhibitor(Avasimibe, Eflucimibe etc.), apoAI Milano and an analogue thereof, scavenger receptor inhibitor, 15-lipoxygenase inhibitor, phospholipase A2 inhibitor, ABCA1 activator, LXR ligand, sphingomyelinase inhibitor, paraoxonase activator, estrogen receptor agonist and the like.
(17) HDL increasing agents
   squalene synthetase inhibitors, CETP inhibitors, LPL activators and the like.
(18) Unstable plaque stabilizing agents
   MMP inhibitors, chymase inhibitors, ACAT inhibitors, lipid-rich plaque regressing agents and the like.
(19) Myocardial protecting agents
   cardiac ATP-K oral formulation, endoserine antagonist, urotensin antagonist and the like.
(20) Hypothyroidism treating agents
   dried thyroid gland (thyreoid), levothyroxine sodium (thyradin S), liothyronidin sodium (thyronine, thyromin) and the like.
(21) Nephrotic syndrome treating agents
   prednisolone (Predonine), prednisolone succinate sodium (Predonine), methylprednisolone succinate sodium (Solu medrol), betamethasone (rinderon) and the like.
(22) Chronic renal failure treating agents
   diuretics [e.g., furosemide (Lasix), bumetamide (Lunetron), azosemide (Diart)], hypotensive agent [e.g., ACE inhibitor, enalapril maleate (Renivase), Ca antagonist (manidipine), α-receptor blocker, AII antagonist (candesartan)] and the like.
(23) Diuretics
   thiazide diuretics (benzylhydro-chlorothiazide, cyclopenthiazide, ethiazide, hydrochlorothiazide, hydroflumethiazide, methyclothiazide, penfluthiazide, polythiazide, trichloromethiazide etc.), loop diuretics (clortharidone, clofenamide, indapamide, mefruside, meticrane, sotolazone, tripamide, quinethazone, metolazone, furosemide etc.), potassium retaining diuretics (spironolacton, triamterene etc.).

(24) Hypertension treating agents
   (i) sympathetic nerve suppressants
      α₂ stimulants (e.g., clonidine, guanabenz, guanfacine, methyldopa etc.), ganglionic blocking agents (e.g., hexamethonium, trimethaphan etc.), presynaptic blockers (e.g., alseroxylon, dimethylaminoreserpinate, rescinamine, reserpine, syrosingopine etc.), neuron blockers (e.g., betanidine, guanethidine etc.), α₁ blockers (e.g., bunazosin, doxazocin, prazosin, terazosin, urapidil etc.), β blockers (e.g., propranolol, nadolol, timolol, nipradilol, bunitrolol, indenolol, penbutolol, carteolol, carvedilol, pindolol, acebutolol, atenolol, bisoprolol, metoprolol, labetalol, amosulalol, arotinolol etc.).
   (ii) vasodilators
      calcium channel antagonists (e.g., manidipine, nicardipine, nilvadipine, nisoldipine, nitrendipine, benidipine, amlodipine, aranidipine etc.), phthalazine derivatives (e.g., budralazine, cadralazine, ecarazine, hydralazine, todralazine etc.) and the like.
   (iii) ACE inhibitors
      alacepril, captopril, cilazapril, delapril, enalapril, lisinopril, temocapril, trandolapril, quinapril, imidapril, benazepril, perindopril and the like.
   (iv) AII antagonists
      losartan, candesartan, valsartan, termisartan, irbesartan, forasartan and the like.
   (v) Diuretics
      e.g., diuretics described above
(25) Cardiac failure treating agents
   cardiotonic agents (e.g.., digitoxin, digoxin, methyldigoxin, lanatoside C, proscillaridine etc.), α,β-stimulants (e.g., epinephrine, norepinephrine, isoproterenol, dopamine, docarpamine, dobutamine, denopamine etc.), phosphodiesterase inhibitors (e.g., amrinone, milrinone, olprinone hydrochloride etc.), calcium channel sensitivity promoters (e.g., pimobendan etc.), nitrate agents (e.g., nitroglycerin, isosorbide nitrate etc.), ACE inhibitors (e.g., ACE inhibitors described above), diuretics (e.g., diuretics described above), carperitide, ubidecarenone, vesnarinone, aminophylline and the like.
(26) Muscle relaxants
   pridinol, tubocurarine, pancuronium, tolperisone hydrochloride, chlorphenesin carbamate, baclofen, chlormezanone, mephenesin, chlorzoxazone, eperisone, tizanidine and the like.
(27) Anticonvulsants
   phenytoin, ethosuximide, acetazolamide, chlordiazepoxide, trimethadione, carbamazepine, phenobarbital, primidone, sulthiame, sodium valproate, clonazepam, diazepam, nitrazepam and the like.
(28) Cardiacs
   trans-π-oxocamphor, terephyllol, aminophyllin, etilefrine, dopamine, dobutamine, denopamine, aminophyllin, vesnarinone, amrinone, pimobendan, ubidecarenone, digitoxin, digoxin, methyldigoxin, lanatoside C, G-strophanthin and the like.
(29) Vasodilators
   oxyfedrine, diltiazem, tolazoline, hexobendine, bamethan, clonidine, methyldopa, guanabenz and the like.
(30) Vasoconstrictors
   dopamine, dobutamine, denopamine and the like.
(31) Antiarrhythmics
   (i) Na channel blockers (e.g., quinidine, procainamide, disopyramide, ajmaline, cibenzoline, lidocaine, diphenylhydantoin, mexiletine, propafenone, flecainide, pilsicainide, phenitoin etc.),
   (ii) β-blockers (e.g., propranolol, alprenolol, bufetolol, oxprenolol, atenolol, acebutolol, metoprolol, bisoprolol, pindolol, carteolol, arotinolol etc.),
   (iii) K channel blockers (e.g., amiodarone etc.),
   (iv) Ca channel blockers (e.g., verapamil, diltiazem etc.) and the like.
(32) Hypertensors
   dopamine, dobutamine, denopamine, digitoxin, digoxin, methyldigoxin, lanatoside C, G-strophanthin and the like.
(33) Antidiabetic drugs
   sulfonylureas (e.g., tolbutamide, chlorpropamide, glyclopyramide, acetohexamide, tolazamide, glibenclamide, glybuzole etc.), biguanides (e.g., metformin hydrochloride, buformin hydrochloride etc.), α-glucosidase inhibitors (e.g., voglibose, acarbose etc.), insulin sensitizers (e.g., pioglitazone, rosiglitazone, troglitazone etc.), insulin, glucagon, agents for treating diabetic complications (e.g., epalrestat etc.) and the like.

(34) Tranquilizers
   diazepam, lorazepam, oxazepam, chlordiazepoxide, medazepam, oxazolam, cloxazolam, clotiazepam, bromazepam, etizolam, fludiazepam, hydroxyzine and the like.
(35) Antipsychotics
   chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine and the like.
(36) Therapeutic agents for Alzheimer's diseases
   (i) choline esterase inhibitors such as donepezil, rivastigmine, galanthamine, TAK-147 and the like.
   (ii) cerebral function activators such as Idebenone, Memantine, vinpocetine and the like.
(37) Anti-Parkinson drugs
   L-dopa, Deprenyl, carbidopa+levodopa, Pergolide, Ropinirole, cabergoline, Pramipexol, Entacapone, Lazabemide and the like.
(38) Therapeutic agents for amyotrophic spinal lateral sclerosis
   riluzole, mecasermin, Gabapentin and the like.
(39) Antidepressants
   imipramine, clomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride and the like.
(40) Therapeutic agents for schizophrenia
   Olanzapine, risperidone, Quetiapine, Iloperidone and the like.
(41) Antitumor drugs
   6-O-(N-chloroacetylcarbamoyl)fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, busulphan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestol, chlormadinone acetate, leuprorelin acetate, buserelin acetate and the like.
(42) Vitamins
   (i) vitamin A: vitamin A₁, vitamin A₂ and retinol palmitate
   (ii) vitamin D: vitamin D₁, D₂, D₃, D₄ and D₅
   (iii) vitamin E: α-tocopherol, β-tocopherol, γ-tocopherol, δ-tocopherol, dl-α-tocopherol nicotinate
   (iv) vitamin K: vitamin K₁, K₂, K₃ and K₄
   (v) folic acid (vitamin M)
   (vi) vitamin B: vitamin B₁, vitamin B₂, vitamin B₃, vitamin B₅, vitamin B₆ and vitamin B₁₂
   (vii) biotin (vitamin H) and the like.
(43) Vitamin derivatives
   various derivatives of vitamins, for example, ascorbic acid, vitamin D₃ derivatives such as 5,6-trans-cholecalciferol, 2,5-hydroxycholecalciferol, 1-α-hydroxycholecalciferol and the like, vitamin D₂ derivatives such as 5,6-trans-ergocalciferol and the like.

(44) Antiallergic drugs
   diphenhydramine, chlorpheniramine, tripelennamine, metodilamine, clemizole, diphenylpyraline, methoxyphenamine, sodium cromoglycate, tranilast, repirinast, amlexanox, ibudilast, ketotifen, terfenadine, mequitazine, azelastine, epinastine, ozagrel hydrochloride, pranlkast hydrate, seratrodast and the like.
(45) Antiasthmatics
   isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, iprotropium bromide, oxitropium bromide, flutropium bromide, theophyline, aminophyllin, sodium cromoglycate, tranilast, repirinast, anrexanone, ibudilast, ketotifen, terfenadine, mequitazine, azelastine, epinastine, ozagrel hydrochloride, pranlkast hydrate, seratrodast, dexamethasone, prednisolone, hydrocortisone, beclometasone dipropionate and the like.
(46) Therapeutic agents for atopic dermatitis
   sodium cromoglycate and the like.
(47) Therapeutic agents for allergic rhinitis
   sodium cromoglycate, chlorpheniramine maleate, alimemazine tartrate, clemastine fumarate, homochlorcyclizine hydrochloride, terfenadine, mequitazine and the like.
(48) Therapeutic agents for pollakisuria/anischuria
   flavoxate hydrochloride and the like.
(49) Anti-sepsis drugs
   peptidic compounds such as rBPI-21 (bactericidal permeability increasing protein), BI-51017 (antithrombin III), SC-59735 (rTFPI), r-PAF acetylhydrase, LY-203638 (r-activated protein C), anti-TNF-α antibody, anti-CD14 antibody, CytoFab, alkaline phosphatase (LPS inactivator) and the like, and non-peptidic compounds such as JTE-607, E-5531, E-5564, S-5920, FR-167653, ONO-1714, ONO-5046 (sivelestat), GW-273629, RWJ-67657, GR-270773, NOX-100, GR-270773, NOX-100 and the like.
(50) Antiemetic
   phenothiazine derivative, 5-HT3 receptor antagonist and the like.
(51) methemoglobin increase preventive drugs
   Methylene blue, ascorbic acid and the like.
(52) Others
   hydroxycam, diaserine, megestrol acetate, nicerogolin, prostaglandins and the like.

When compound (I) and other drug are combined, the following effects are afforded.
(1) The doses thereof can be reduced as compared to a single administration of each of compound (I) and a concomitant drug.
(2) A synergistic treatment effect can be achieved for the above-mentioned diseases such as sepsis, particularly severe sepsis, septic shock, inflammatory disease, infectious disease and the like.
(3) A broad treatment effect can be provided on various diseases developed by diseases such as bacterial infection and the like.
With regard to the combined use, the timing of the administration of compound (I) and a concomitant drug is not limited. Compound (I) or a pharmaceutical composition thereof and a concomitant drug or a pharmaceutical composition thereof may be simultaneously administered to the administration subject, or may be administered in a staggered manner. The dose of the concomitant drug follows a clinical dose and can be appropriately determined depending on the administration subject, administration route, disease, combination and the like.

The mode of combined administration is not particularly limited, and compound (I) and a concomitant drug only need to be combined on administration. Examples of such administration mode include the following:
(1) administration of a single preparation obtained by simultaneously processing compound (I) or a pharmaceutical composition thereof and the concomitant drug (hereinafter to be also referred to as the combination drug of the present invention), (2) simultaneous administration of two kinds of preparations of compound (I) or a pharmaceutical composition thereof and the concomitant drug or a pharmaceutical composition thereof, which have been separately produced, by the same administration route, (3) administration of two kinds of preparations of compound (I) or a pharmaceutical composition thereof and the concomitant drug or a pharmaceutical composition thereof, which have been separately produced, by the same administration route in a staggered manner, (4) simultaneous administration of two kinds of preparations of compound (I) or a pharmaceutical composition thereof and the concomitant drug or a pharmaceutical composition thereof, which have been separately produced, by different administration routes, (5) administration of two kinds of preparations of compound (I) or a pharmaceutical composition thereof and the concomitant drug or a pharmaceutical composition thereof, which have been separately produced, by different administration routes in a staggered manner (e.g., administration in the order of compound (I) or a pharmaceutical composition thereof and the concomitant drug or a pharmaceutical composition thereof, or in the reverse order) and the like.

The mixing ratio of compound (I) and a concomitant drug in the combination drug of the present invention can be appropriately determined according to the subject of administration, administration route, disease and the like.
For example, the content of compound (I) of the present invention in the combination agent of the present invention differs depending on the form of a preparation, and is usually from about 0.01 to 100% by weight, preferably from about 0.1 to 50% by weight, further preferably from about 0.5 to 20% by weight, based on total of the preparation.
The content of the concomitant drug in the combination agent of the present invention differs depending on the form of a preparation, and is usually from about 0.01 to 100% by weight, preferably from about 0.1 to 50% by weight, further preferably from about 0.5 to 20% by weight, based on total of the preparation.
The content of additives such as carrier in the combination agent of the present invention differs depending on the form of a preparation, and is usually from about 1 to 99.99% by weight, preferably from about 10 to 90% by weight, based on total of the preparation.
When compound (I) and the concomitant drug are independently prepared, the contents thereof may be the same as those mentioned above.

When compound (I) is administered to a human, it can be safely administered orally or parenterally as it is or in a mixture with an appropriate pharmacologically acceptable carrier, excipient and diluent, in a pharmaceutical composition such as an oral agent (e.g., powder, granule, tablet, capsule etc.), a parenteral agent (e.g., injection, external formulation (e.g., nasal formulation, percutaneous formulation etc.) and suppository (e.g., rectal suppository and vaginal suppository etc.).
Any of these formulations may be produced by any method known per se which is employed ordinarily for producing a pharmaceutical formulation. The amount of compound (I) to be incorporated into a formulation may vary depending on the dosage forms, and is preferably about 10 to 95% by weight in an oral formulation described above and about 0.001 to about 95% by weight in a parenteral agent described above.

For example, compound (I) can be prepared into an aqueous injection together with a solubilizer (e.g., β-cyclodextrins etc.), a dispersant (e.g., Tween 80 (manufactured by ATLASPOWDER USA), HCO 60 (manufactured by NIKKO CHEMICALS), carboxymethylcellulose, sodium arginate etc.), a preservative (e.g., methyl paraben, propyl paraben, benzyl alcohol chlorobutanol etc.), an isotonic agent (e.g., sodium chloride, glycerine, sorbitol, glucose etc.) and the like according to a conventional method, or into an oil-based injection by appropriately dissolving, suspending or emulsifying using a vegetable oil (e.g., olive oil, sesame oil, peanut oil, cottonseed oil, corn oil etc.) and propylene glycol and the like.
Since compound (I) of the present invention is water soluble, an injection can be prepared from compound (I) alone. However, injection containing a higher concentration of compound (I) can be prepared by combining compound (I) and a solubilizer.
An oral formulation can be produced by, for example, compressing compound (I) together with an excipient (e.g., lactose, sucrose, starch etc.), a disintegrant (e.g., starch, calcium carbonate etc.), a binder (e.g., starch, gum arabic, carboxymethyl cellulose, polyvinyl pyrrolidone, hydroxypropyl cellulose etc.), a lubricant (e.g., talc, magnesium stearate, polyethylene glycol 6000 etc.), and the like, followed by, where necessary, a coating process known *per se* for the purpose of masking a taste, forming an enteric coat, or achieving a sustained release. For such coating agent, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (manufactured by ROHM, Germany, a copolymer of methacrylic acid and acrylic acid), a dye (e.g., titanium oxide, colcothar etc.) and the like may appropriately be used.
Compound (I) can also be employed as an external formulation in the form of a solid or semi-solid or a liquid.
For example, a solid external formulation may be compound (I) as it is or can be produced by mixing compound (I) with an excipient (e.g., glycol, mannitol, starch, crystalline cellulose etc.), a thickening agent (e.g., natural gums, cellulose derivatives, acrylic acid polymers etc.) which is then converted into a powder composition. A semi-solid external formulation may be produced by a standard method and preferably used in the form of an aqueous or oil-based gel or ointment. A liquid external formulation may be produced by a method employed for producing an injection formulation or an analogous method in the form of an oil-based or aqueous suspension.
The solid, semi-solid or liquid external formulation may be supplemented also with a pH modifier (e.g., carbonic acid, phosphoric acid, citric acid, hydrochloric acid, sodium hydroxide etc.), an antiseptic (e.g., p-oxybenzoates, chlorobutanol, benzalkonium chloride etc.) and the like, as appropriate. Typically, an ointment usually containing about 0.1 to 100 mg of compound (I) per 1 g of vaseline or lanolin etc. as a formulation base, can be used.
Compound (I) may be also formulated as an oil or aqueous, solid or semi-solid or liquid suppository. As an oil base in preparing suppository, for example, a high fatty acid glyceride (e.g., cocoa butter, WITEPSOL (manufactured by DYNAMIT NOBEL) etc.), a middle fatty acid (e.g., MYGLYOL (manufactured by DYNAMIT NOBEL) etc.), a vegetable oil (e.g., sesame oil, soybean oil, cottonseed oil etc.) and the like are used as appropriate. An aqueous base may be, for example, polyethylene glycol or propylene glycol, and an aqueous gel base may be, for example, a natural gum, a cellulose derivative, a vinyl polymer, an acrylic polymer and the like.

While the dose of compound (I) varies depending on the age, body weight, symptom, dosage form, administration method, dosing period and the like, it is, for example, generally about 0.01 to about 1000 mg/kg, preferably about 0.01 to about 100 mg/kg, more preferably about 0.1 to about 100 mg/kg, particularly about 0.1 to about 50 mg/kg, especially about 1.5 to about 30 mg/kg, a day in the amount of compound (I) for a patient (adult, body weight about 60 kg) with severe sepsis, which is orally or parenterally administered in one to several portions a day. As mentioned above, since the dose varies depending on various conditions, an amount less than the aforementioned dose may be sufficient or administration of an excess amount may be necessary.
While the dose of a combination drug in the present invention varies depending on the kind of compound, the age, body weight, symptom, dosage form, administration method, dosing period and the like, it is, for example, generally about 0.01 to about 1000 mg/kg, preferably about 0.01 to about 100 mg/kg, more preferably about 0.1 to about 100 mg/kg, particularly about 0.1 to about 50 mg/kg, especially about 1.5 to about 30 mg/kg, a day in the amount of each of compound (I) and a concomitant drug for a patient (adult, body weight about 60 kg) with severe sepsis, which is intravenously administered in one to several portions a day. Of course, as mentioned above, since the dose varies depending on various conditions, an amount less than the aforementioned dose may be sufficient or administration of an excess amount may be necessary.
The concomitant drug may be contained in any amount as long as a side effect does not pose a problem. While the daily dose of the combination drug may vary depending on the disease state, the age, sex, body weight and difference in sensitivity of the administration object, timing and interval of administration, characteristics, dispensing and kind of the pharmaceutical preparation, the kind of active ingredient and the like and is not particularly limited, the amount of the drug is generally about 0.001 to 2000 mg, preferably about 0.01 to 500 mg, more preferably about 0.1 to 100 mg, per 1 kg body weight of mammal by oral administration, which is generally administered in 1 to 4 portions during a day.

For administration of a combination drug of the present invention, compound (I) may be administered after administration of the concomitant drug or the concomitant drug may be administered after administration of compound (I), though they may be administered simultaneously. When administered at a time interval, the interval differs depending on the effective ingredient to be administered, drug form and administration method, and for example, when the concomitant drug is administered first, a method in which compound (I) is administered within time range of from 1 min to 3 days, preferably from 10 min to 1 day, more preferably from 15 min to 1 hr after administration of the concomitant drug is exemplified. When compound (I) is administered first, a method in which the concomitant drug is administered within time range of from 1 min to 1 day, preferably from 10 min to 6 hrs, more preferably from 15 min to 1 hr after administration of compound (I) is exemplified.

Compound (I) can also be useful as a TLR signal inhibitor, since it has an NO and/or cytokine production inhibitory action based on the inhibition of TLR (Toll-like receptor) signal. Here, by the "TLR signal" is meant a signal transduction, with which any Toll-like receptor recognizes bacterial component and the like of microorganism and induces biological defense response, and, for example, signal transduction via known TLR1-TLR10 can be mentioned. Compound (I) can be particularly useful as a TLR4 signal inhibitor.
Compound (I) can also be useful for the prophylaxis or treatment of a disease caused by a change in the TLR signal, for example, organ disorder and the like. As used herein, by the organ is meant various organs of the central nervous system, the circulatory system, respiratory system, the bone and joint system, the digestive system and the renal and urinary system.
Specifically, compound (I) is useful for the prophylaxis and/or treatment of diseases caused by a change in the TLR signal, such as
(1) central nervous system diseases [(i) neurodegenerative disease (e.g., senile dementia, Alzheimer's disease, Down's syndrome, Parkinson's syndrome, Creutzfeldt-Jakob disease, amyotrophic spinal lateral sclerosis, diabetic neuropathy etc.), (ii) neuropathy in cerebrovascular diseases (e.g. impairment of cerebral blood flow based on cerebral infarction, cerebral hemorrhage, cerebral sclerosis, etc.), brain trauma, spiral cord injury, cerebritis sequela and cerebral palsy, (iii) dysmnesia (e.g. senile dementia, amnesia, etc.) and the like], particularly Alzheimer's disease,
(2) circulatory system diseases [(i) acute coronary artery syndrome such as acute cardiac infarction, unstable angina pectoris and the like, (ii) peripheral obstruction, (iii) restenosis after coronary intervention (percutaneous transluminal coronary angioplasty (PTCA), atherectomy (DCA), stenting etc.), (iv) restenosis after coronary bypass surgery, (v) restenosis after other peripheral arterial interventions (angioplasty, atherectomy, stenting etc.) and bypass surgery, (vi) ischemic heart disease such as cardiac infarction, angina pectoris and the like, (vii) intermittent claudication, (viii) stroke (cerebral infarction, cerebral embolus, cerebral hemorrhage etc.), (ix) lacunar infarct, (x) cerebrovascular dementia, (xi) arteriosclerosis (e.g., atherosclerosis etc.) and diseases caused thereby (e.g., ischemic heart disease such as cardiac infarction and the like, cerebrovascular disorder such as cerebral infarction, stroke, etc.), (xii) cardiac failure, (xiii) arrhythmia, (xiv) progression of focus of arteriosclerosis, (xv) thrombogenesis, (xvi) hypotension, (xvii) shock, (xviii) shock-induced vascular embolism (disseminated intravascular coagulation (DIC) etc.)], particularly arteriosclerosis,
(3) respiratory system diseases [respiratory distress syndrome, respiratory failure, emphysema, pneumonia, bronchitis, bronchiolitis and the like],
(4) diseases of bone and joint system [arthritis rheumatoides, osteoporosis, osteomalacia, osteopenia, Paget's disease of bone, osteomalacia and the like], particularly arthritis rheumatoides,
(5) diseases of digestive-liver, biliary tract and pancreas system [ulcerative colitis, gastritis, digestive ulcer, cirrhosis, hepatic failure, hepatitis, cholecystitis, pancreatitis and the like], particularly ulcerative colitis,
(6) diseases of renal and urinary system [nephritis, kidney failure, cystitis and the like] or
a combination of these diseases (multiple organ failure etc.) and the like. Moreover, a TLR signal inhibitor containing compound (I) of the present invention is also useful for the prophylaxis or treatment of infections caused by a change in the TLR signal, particularly sepsis (severe sepsis) accompanying an organ disorder.
Compound (I) can be formulated into a TLR signal inhibitor or an agent containing the inhibitor for the prophylaxis or treatment of a disease caused by a change in the TLR signal, by a method similar to that of the aforementioned therapeutic agent for severe sepsis and the like, and can be administered to a mammal by an administration route and at a dose similar to those of the aforementioned therapeutic agent for severe sepsis and the like.

### Examples

The present invention is explained in detail by way of the following Reference Examples, Examples, Formulation Examples and Experimental Examples but these do not limit the present invention.
The elution in column chromatography in Reference Examples and Examples was performed under observation by TLC (thin-layer chromatography). In the TLC observation, Kieselgel 60F₂₅₄ plate (Merck) was used as a TLC plate, the solvent used as an elution solvent in the column chromatography was used as an eluent, and the means of detection used was an UV detector. As silica gel for column, Kieselgel 60F₂₅₄ (70-230 mesh) manufactured by Merck was used. NMR spectra are shown by proton NMR with tetramethylsilane as the internal standard, using VARIAN Gemini-200 (200 MHz type spectrometer) or VARIAN Mercury-300 (300 MHz); δ values are expressed in ppm.
The abbreviations used in Reference Examples and Examples mean the following:
- s:: singlet
- br:: broad
- d:: doublet
- t:: triplet
- q:: quartet
- dd:: double doublet
- m:: multiplet
- J:: coupling constant
- Hz:: hertz
- Me:: methyl group
- Et:: ethyl group
- Bn:: benzyl group
- Ac:: acetyl group
- Ts:: p-toluenesulfonyl group
- Boc:: tert-butoxycarbonyl group
- Py:: pyridine
- DMF:: N,N-dimethylformamide
- THF:: tetrahydrofuran

### Reference Example 1

### (1R,2S,5R)-2-isopropyl-5-methylcyclohexyl chloromethyl carbonate

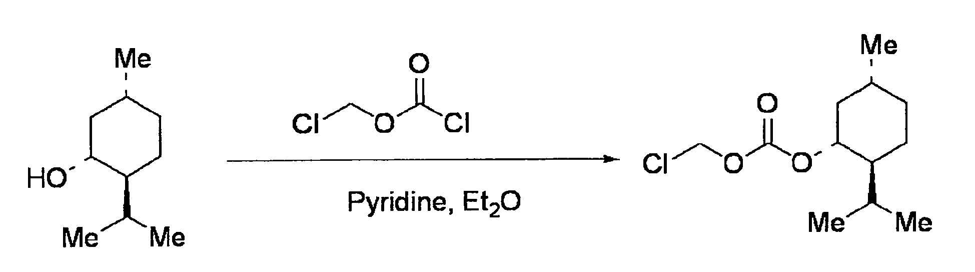

To a solution of L-menthol (2 g) and pyridine (1.14 mL) in diethyl ether (30 mL) was added dropwise a solution of chloromethyl chloroformate in diethyl ether (10 mL) at -10°C, and the mixture was stirred at room temperature for 24 hr. The insoluble material was filtered off. The filtrate was washed with saturated brine (50 mL), dried over anhydrous magnesium sulfate and concentrated under reduced pressure. The residue was purified by column chromatography (silica gel 25 g, ethyl acetate/hexane 1/20) to give the title compound (2.64 g) as a colorless oil.
¹H-NMR (CDCl₃, 200 MHz):δ 0.78-2.16 (18H, m), 4.54-4.67 (1H, m), 5.73 (2H, dd, J = 8.8 Hz, 6.2 Hz).

### Reference Example 2

### (1R,2S,5R)-2-isopropyl-5-methylcyclohexyl iodomethyl carbonate

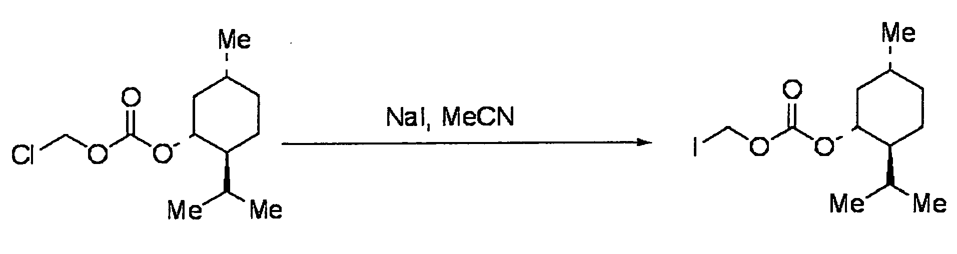

A solution of (1R,2S,5R)-2-isopropyl-5-methylcyclohexyl chloromethyl carbonate (1 g) obtained in Reference Example 1 and sodium iodide (2.41 g) in acetonitrile (40 mL) was stirred at 80°C for 2 hr. The solvent was evaporated, diethyl ether (100 mL) was added, and the mixture was washed successively with water (100 mL), 5% aqueous sodium thiosulfate solution (100 mLx2) and saturated brine (50 mL), dried over anhydrous sodium sulfate, and concentrated under reduced pressure to give the title compound (1.33 g) as a pale-yellow oil. ¹H-NMR (CDCl₃, 200 MHz):δ 0.78-1.17 (12H, m), 1.35-2.12 (6H, m), 4.53-4.66 (1H, m), 5.95 (2H, s).

### Reference Example 3

### 2-benzyl 1-(chloromethyl) (2S)-pyrrolidine-1,2-dicarboxylate

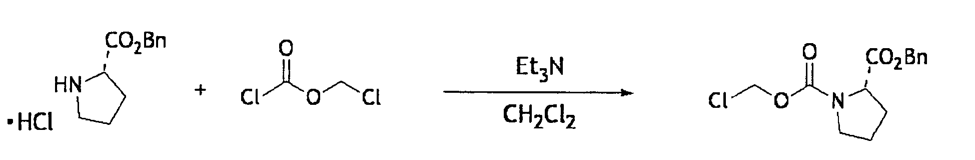

To a mixed solution of L-proline benzyl ester hydrochloride (757 mg) and methylene chloride (7.5 mL) was added dropwise a solution (2.5 mL) of triethylamine (873 µL) and chloromethyl chloroformate (279 µL) in methylene chloride under ice-cooling, and the mixture was stirred at room temperature for 1.5 hr under a nitrogen atmosphere. The reaction mixture was filtered with a glass filter, and the filtrate was washed with water and brine. The organic layer was dried over anhydrous magnesium sulfate and concentrated under reduced pressure to give the title compound (934 mg). ¹H-NMR(CDCl₃, 200MHz):δ 1.87-2.33 (4H, m), 3.46-3.72 (2H, m), 4.37-4.51 (1H, m), 5.10-5.25 (2H, m), 5.52 (0.5H, d, J = 6.1 Hz), 5.70 (1H, dd, J = 6.1, 0.8 Hz), 5.89 (0.5H, d, J = 6.1 Hz), 7.36 (5H, m).

### Reference Example 4

### 2-benzyl 1-(chloromethyl) (2R)-pyrrolidine-1,2-dicarboxylate

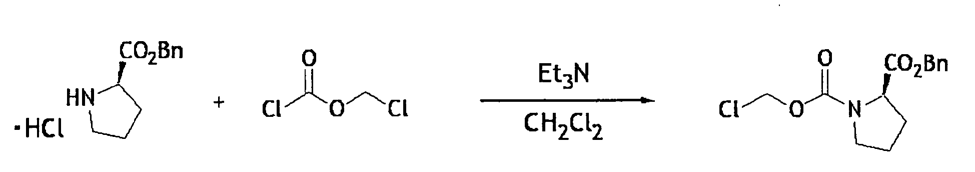

To a mixed solution of D-proline benzyl ester hydrochloride (1.96 g) and methylene chloride (10 mL) was added dropwise a solution (10 mL) of triethylamine (2.26 mL) and chloromethyl chloroformate (721 µL) in methylene chloride under ice-cooling, and the mixture was stirred at room temperature for 1.5 hr under a nitrogen atmosphere. The reaction mixture was filtered with a glass filter, and the filtrate was washed with water and brine. The organic layer was dried over anhydrous magnesium sulfate and concentrated under reduced pressure to give the title compound (2.46 g). ¹H-NMR(CDCl₃, 200MHz):δ 1.88-2.38 (4H, m), 3.47-3.72 (2H, m), 4.37-4.51 (1H, m), 5.10-5.30 (2H, m), 5.52 (0.5H, d, J = 6.0 Hz), 5.71 (1H, dd, J = 6.0, 1.2 Hz), 5.89 (0.5H, d, J = 6.0 Hz), 7.46 (5H, br s).

### Reference Example 5

### chloromethyl 4-[2-(benzyloxy)-2-oxoethyl]piperidine-1-carboxylate

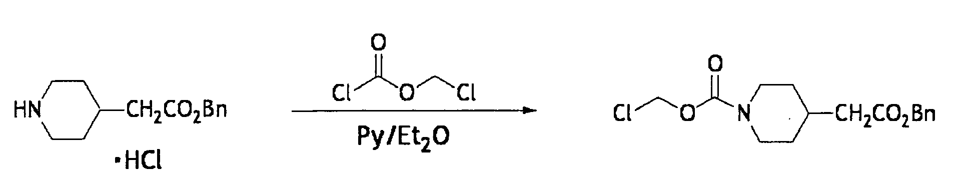

To a mixed solution of benzyl piperidin-4-yl acetate hydrochloride (1.03 g) and diethyl ether (10.8 mL) was added dropwise a solution (10.8 mL) of pyridine (649 µL) and chloromethyl chloroformate (374 µL) in diethyl ether over 40 min under ice-cooling, and the mixture was stirred for 1.5 hr under a nitrogen atmosphere. The reaction mixture was diluted with diethyl ether, washed with water and brine, dried over anhydrous magnesium sulfate, and concentrated under reduced pressure. The residue was purified by silica gel column chromatography (ethyl acetate-hexane) to give the title compound (412 mg) as an oil.
¹H-NMR(CDCl₃, 200MHz): δ 1.06-1.33 (2H, m), 1.74 (2H, br d, J = 12.8 Hz), 1.94-2.06 (1H, m), 2.31 (2H, d, J = 7.0 Hz), 2.75-2.91 (2H, m), 4.05-4.23 (2H, m), 5.12 (2H, s), 5.78 (2H, s), 7.35 (5H, s).

### Reference Example 6

### benzyl N-[(chloromethoxy)carbonyl]glycinate

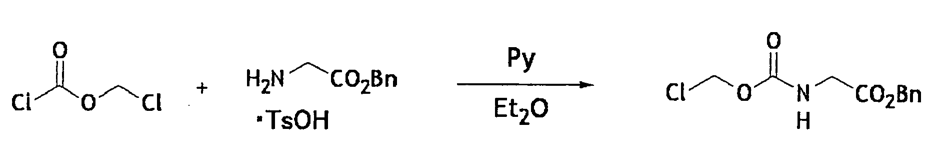

To a mixture of glycine benzyl ester p-toluenesulfonate (1.66 g) and diethyl ether (13 mL) was added dropwise a solution (13 mL) of pyridine (836 µL) and chloromethyl chloroformate (481 µL) in diethyl ether over 45 min under ice-cooling, and the mixture was stirred for 2.5 hr under a nitrogen atmosphere. The reaction mixture was diluted with diethyl ether, washed with water and brine, dried over anhydrous magnesium sulfate, and concentrated under reduced pressure. The residue was purified by silica gel column chromatography (ethyl acetate-hexane) to give the title compound (1.08 g) as a white powder.
¹H-NMR(CDCl₃, 200MHz): δ 4.06 (2H, d, J = 5.6 Hz), 5.20 (2H, s), 5.46 (1H, br s), 5.75 (2H, s), 7.36 (5H, s).

### Reference Example 7

### N-[(chloromethoxy)carbonyl]-D-leucinate

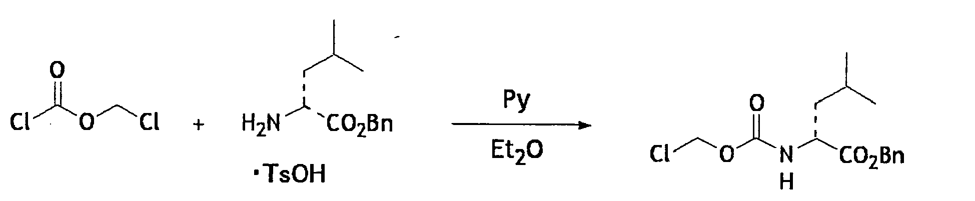

To a mixed solution of D-leucine benzyl ester p-toluenesulfonate (2.20 g) and diethyl ether (18 mL) was added dropwise a solution (18 mL) of pyridine (950 µL) and chloromethyl chloroformate (547 µL) in diethyl ether over 40 min under ice-cooling, and the mixture was stirred for 5 hr under a nitrogen atmosphere. The reaction mixture was diluted with diethyl ether, washed with water and brine, dried over anhydrous magnesium sulfate, and concentrated under reduced pressure. The residue was purified by silica gel column chromatography (ethyl acetate-hexane) to give the title compound (1.57 g) as a white powder.
¹H-NMR (CDCl₃, 200MHz):δ 0.92 (3H, d, J = 6.0 Hz), 0.94 (3H, d, J= 6.0 Hz), 1.50-1.71 (3H, m), 4.40-4.51 (1H, m), 5.18 (2H, s), 5.35 (1H, d, J = 9.2 Hz), 5.74 (2H, q, J= 6.2 Hz), 7.32-7.42 (5H, m).

### Reference Example 8

### ethyl 6-[((2-chloro-4-fluorophenyl){[({[(1R,2S,5R)-2-isopropyl-5-methylcyclohexyl]oxy}carbonyl)oxy]methyl}-amino)sulfonyl]cyclohex-1-ene-1-carboxylate

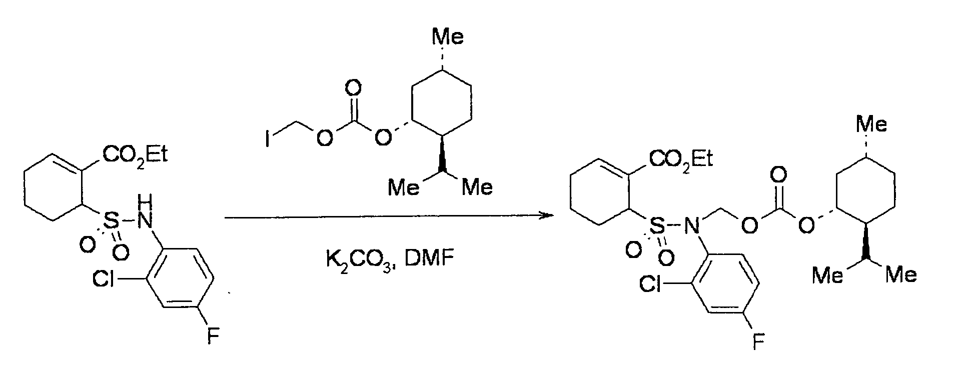

(1R,2S,5R)-2-Isopropyl-5-methylcyclohexyl iodomethyl carbonate (564 mg) obtained in Reference Example 2 and potassium carbonate (382 mg) were added to a solution of ethyl 6-[N-(2-chloro-4-fluorophenyl)sulfamoyl]cyclohex-1-ene-1-carboxylate (500 mg) in N,N-dimethylformamide (5 mL), and the mixture was stirred at room temperature for 30 min. The reaction mixture was diluted with ethyl acetate (50 mL), washed with water (50 mL) and saturated brine (50 mL), dried over anhydrous sodium sulfate, and concentrated under reduced pressure. The residue was purified by column chromatography (silica gel 20 g, ethyl acetate/hexane 1/20) and crystallized from hexane-diisopropyl ether to give the title compound (432 mg) as a white powder crystal.
¹H-NMR (DMSO-d₆, 200 MHz):δ 0.68 (3H, d, J = 7.0 Hz), 0.85-2.43 (24H, m), 3.98-4.11 (2H, m), 4.20-4.40 (1H, m), 4.40-4.80 (1H, m), 5.40-5.80 (2H, m), 7.18 (1H, brs), 7.28-7.36 (1H, m), 7.56-7.66 (2H, m).

### Reference Example 9

### ethyl 6-{[[(acetyloxy)methyl](2-chloro-4-fluorophenyl)amino]sulfonyl}cyclohex-1-ene-1-carboxylate

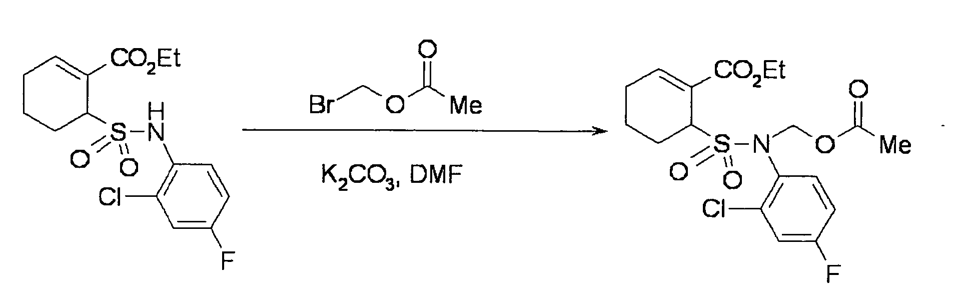

To a solution of ethyl 6-[N-(2-chloro-4-fluorophenyl)sulfamoyl]cyclohex-1-ene-1-carboxylate (200 mg) in N,N-dimethylformamide (2 mL) were added bromomethyl acetate (101 mg) and potassium carbonate (152 mg), and the mixture was stirred at room temperature for 2 hr. The reaction mixture was diluted with ethyl acetate (20 mL), washed with water (20 mL) and saturated brine (20 mL), dried over anhydrous sodium sulfate, and concentrated under reduced pressure. The residue was purified by column chromatography (silica gel 7 g, ethyl acetate/hexane 1/5) to give the title compound (225 mg) as a white amorphous powder.
¹H-NMR (CDCl₃, 200 MHz)):δ 1.19-1.30 (3H, m), 1.58-1.84 (2H, m), 2.05 (3H, s), 2.15-2.49 (4H, m), 4.07-4.17 (2H, m), 4.58 (1H, br s), 5.40-5.80 (2H, m), 7.00-7.09 (1H, m), 7.22-7.27 (2H, m), 7.63 (1H, br s).
elemental analysis value: as C₁₈H₂₁NO₆SClF
Calculated (%): C,49.83; H, 4.88; N, 3.23.
Found (%): C, 49.75; H, 4.88; N, 3.11.

### Reference Example 10

### ethyl 6-{[[(acetyloxy)acetyl](2-chloro-4-fluorophenyl)amino]sulfonyl}cyclohex-1-ene-1-carboxylate

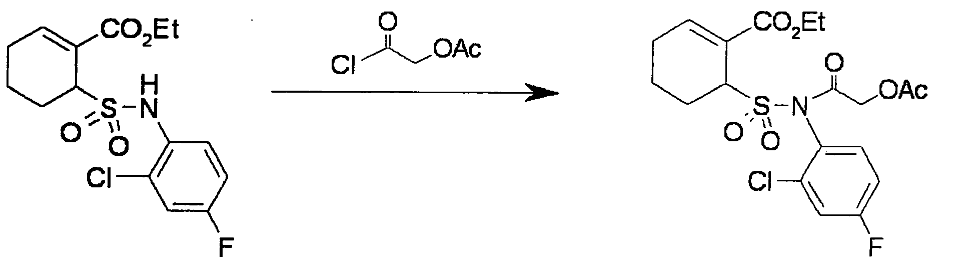

To a solution of ethyl 6-[N-(2-chloro-4-fluorophenyl)sulfamoyl]cyclohex-1-ene-1-carboxylate (2.0 g) and triethylamine (1.07 mL) in tetrahydrofuran (10 mL) was added dropwise a solution of acetoxyacetyl chloride (0.85 mL) in tetrahydrofuran (1 mL) under ice-cooling, and the mixture was stirred for 30 min under ice-cooling and at room temperature for 1 hr. The reaction mixture was diluted with ethyl acetate (50 mL), washed with water (50 mLx2) and saturated brine (50 mL), dried over anhydrous magnesium sulfate, and concentrated under reduced pressure. The residue was purified by column chromatography (silica gel 50 g, ethyl acetate/hexane 1/2) to give the title compound (2.28 g) as a white powder.
¹H-NMR (CDCl₃, 200 MHz):δ 1.28, 1.35 (3H, t×2, J = 7 Hz), 1.72-2.96 (9H, m), 4.17-4.58 (4H, m), 4.96, 5.27 (1H, br), 7.02-7.18 (1H, m), 7.28-7.87 (3H, m).

### Reference Example 11

### ethyl 6-{[(2-chloro-4-fluorophenyl)(glycoloyl)amino]sulfonyl}cyclohex-1-ene-1-carboxylate

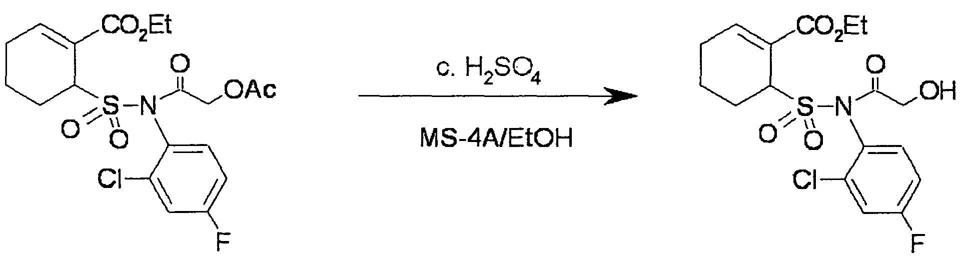

To a solution of ethyl 6-{[[(acetyloxy)acetyl](2-chloro-4-fluorophenyl)amino]sulfonyl}cyclohex-1-ene-1-carboxylate (6.70 g) obtained in Reference Example 10 in ethanol (40 mL) were added molecular sieves 4A (MS-4A: 0.2 g) and concentrated sulfuric acid (2.0 mL), and the mixture was stirred at 60°C for 1.2 hr. The solution was concentrated under reduced pressure to a residual amount of about 20 mL, and diluted with ethyl acetate (100 mL). The mixture was washed successively with ice water (60 mL), 5% aqueous sodium hydrogen carbonate (50 mL) and saturated brine (50 mL), dried over anhydrous magnesium sulfate, and concentrated under reduced pressure. The residue was purified by column chromatography (silica gel 60 g, ethyl acetate/hexane 1/2) and crystallized from diisopropyl ether to give the title compound (4.14 g) as a colorless powder crystal. ¹H-NMR (CDCl₃, 200 MHz) δ 1.31, 1.33 (3H, t×2, J = 7 Hz), 1.75-2.99 (7H, m), 3.71-3.94 (2H, m), 4.18-4.34 (2H, m), 4.88, 5.34 (1H, br), 7.01-7.19 (1H, m), 7.27-7.93 (3H, m).
elemental analysis value: as C₁₇H₁₉ClFNO₆S
Calculated (%): C, 48.63; H, 4.56; N, 3.34.
Found (%): C, 48.67; H, 4.39; N, 3.09.

### Reference Example 12

### ethyl 6-{[(chloroacetyl)(2-chloro-4-fluorophenyl)amino]sulfonyl}cyclohex-1-ene-1-carboxylate

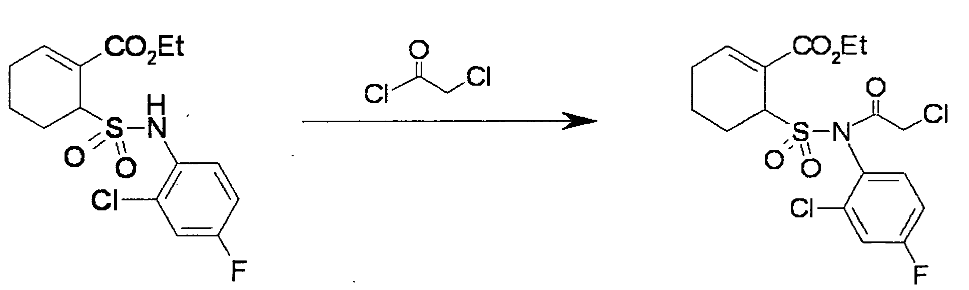

To a solution of ethyl 6-[N-(2-chloro-4-fluorophenyl)sulfamoyl]cyclohex-1-ene-1-carboxylate (3.0 g) and triethylamine (1.39 mL) in tetrahydrofuran (12 mL) was added dropwise a solution of chloroacetyl chloride (0.80 mL) in tetrahydrofuran (1 mL) under ice-cooling, and the mixture was stirred for 30 min under ice-cooling and at room temperature for 2 hr. Triethylamine (0.14 mL) and chloroacetyl chloride (0.08 mL) were added to the reaction mixture, and the mixture was further stirred at room temperature for 1 hr. The reaction mixture was diluted with ethyl acetate (60 mL), washed with water (50 mL) and saturated brine (50 mL×2), dried over anhydrous magnesium sulfate, and concentrated under reduced pressure. The residue was purified by column chromatography (silica gel 50 g, ethyl acetate/hexane 1/3) and crystallized from-diisopropyl ether to give the title compound (2.70 g) as a colorless powder crystal.
¹H-NMR (CDCl₃, 200 MHz):δ 1.33, 1.35 (3H, t×2, J = 7 Hz), 1.75-3.01 (6H, m), 3.92-3.98 (2H, m), 4.18-4.36 (2H, m), 4.94, 5.32(1H, br), 7.05-7.21 (1H, m), 7.28-7.94 (3H, m).
elemental analysis value: as C₁₇H₁₈Cl₂FNO₅S
Calculated (%): C, 46.59; H, 4.14; N, 3.20.
Found (%): C, 46.61; H, 4.13; N, 3.10.

### Reference Example 13

### ethyl 6-({(2-chloro-4-fluorophenyl)[4-oxo-4-(2-oxo-2-phenylethoxy)butanoyl]amino}sulfonyl)cyclohex-1-ene-1-carboxylate

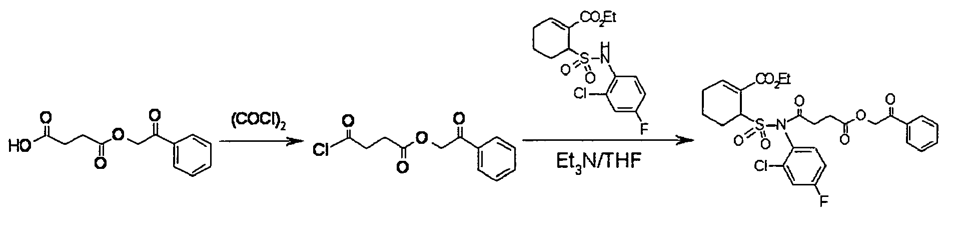

To a mixture of 4-oxo-4-(2-oxo-2-phenylethoxy)butanoic acid (354 mg), tetrahydrofuran (3 mL) and N,N-dimethylformamide (10 µL) was added oxalyl chloride (175 µL), and the mixture was stirred at room temperature for 1 hr. The solvent was evaporated, and the residue was dissolved in tetrahydrofuran (10 mL) and concentrated under reduced pressure. This operation was repeated three times to give 4-oxo-4-(2-oxo-2-phenylethoxy)butanoyl chloride. A solution of this product in tetrahydrofuran (1 mL) was added dropwise to a solution of ethyl 6-[N-(2-chloro-4-fluorophenyl)sulfamoyl]cyclohex-1-ene-1-carboxylate (362 mg) and triethylamine (0.14 mL) in tetrahydrofuran (4 mL) under ice-cooling, and the mixture was stirred for 30 min under ice-cooling and at room temperature overnight. The reaction mixture was diluted with ethyl acetate (30 mL), washed with water (30 mLx2) and saturated brine (20 mL), dried over anhydrous magnesium sulfate, and concentrated under reduced pressure. The residue was purified by column chromatography (silica gel 25 g, ethyl acetate/hexane 1/3) to give the title compound (390 mg) as a white amorphous powder. ¹H-NMR (CDCl₃, 200 MHz):δ 1.32 (3H, J = 7 Hz), 1.72-2.99 (10H, m), 4.16-4.32 (2H, m), 5.14-5.46 (3H, m), 7.03-7.77 (7H, m), 7.89-7.93 (2H, m).

### Reference Example 14

### ethyl 6-{[[4-(benzyloxy)-4-oxobutanoyl](2-chloro-4-fluorophenyl)amino]sulfonyl}cyclohex-1-ene-1-carboxylate

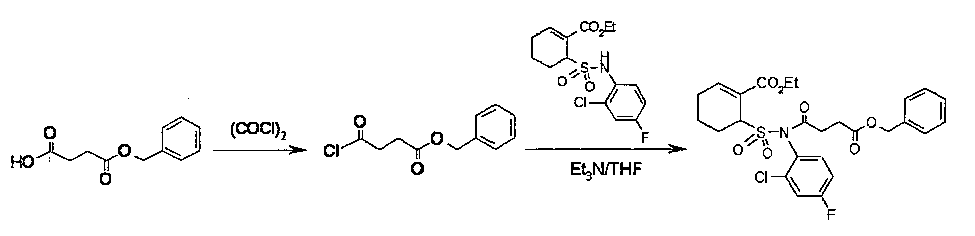

To a mixture of 4-(benzyloxy)-4-oxobutanoic acid (1.66 g), tetrahydrofuran (6 mL) and N,N-dimethylformamide (20 µL) was added oxalyl chloride (1.0 mL), and the mixture was stirred at room temperature for 1 hr. The solvent was evaporated, and the residue was dissolved in tetrahydrofuran (10 mL) and under reduced pressure. This operation was repeated three times to give 4-(benzyloxy)-4-oxobutanoyl chloride. A solution of this product in tetrahydrofuran (2 mL) was added dropwise to a solution of ethyl 6-[N-(2-chloro-4-fluorophenyl)sulfamoyl]cyclohex-1-ene-1-carboxylate (1.45 g) and triethylamine (0.56 mL) in tetrahydrofuran (8 mL) under ice-cooling, and the mixture was stirred for 15 min under ice-cooling and at room temperature for 2 hr. The reaction mixture was diluted with ethyl acetate (40 mL), washed successively with water (40 mL), 1N aqueous sodium hydroxide solution (20 mL), water (40 mL) and saturated brine (20 mL), dried over anhydrous magnesium sulfate, and concentrated under reduced pressure. The residue was purified by column chromatography (silica gel 40 g, ethyl acetate/hexane 1/3) and crystallized from diisopropyl ether to give the title compound (1.78 g) as a colorless powder crystal.
¹H-NMR (CDCl₃, 200 MHz):δ 1.34 (3H, J = 7 Hz), 1.70-2.98 (10H, m), 4.17-4.35 (2H, m), 5.12-5.36 (3H, m), 7.01-7.16 (1H, m), 7.26-7.73 (8H, m).
elemental analysis value: as C₂₆H₂₇ClFNO₇S
Calculated (%): C, 56.57; H, 4.93; N, 2.54.
Found (%): C, 56.55; H, 4.87; N, 2.48.

### Example 1

### ethyl 6-{[({[4-(benzyloxy)-4-oxobutoxy]carbonyl}oxy)methyl](2-chloro₋4-fluorophenyl)sulfamoyl}cyclohex-1-ene-1-carboxylate

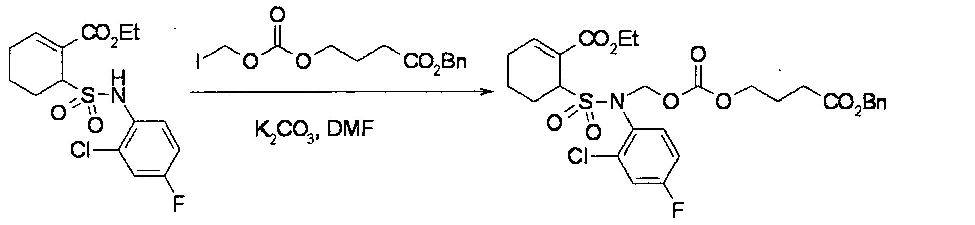

To a solution of ethyl 6-[N-(2-chloro-4-fluorophenyl)sulfamoyl]cyclohex-1-ene-1-carboxylate (550 mg) in N,N-dimethylformamide (5 mL) were added benzyl 4-{[(iodomethoxy)carbonyl]oxy}butanoate (690 mg) and potassium carbonate (420 mg), and the mixture was stirred at room temperature for 1 hr. The reaction mixture was diluted with ethyl acetate (20 mL), washed with water (20 mL×3) and saturated brine (20 mL), dried over anhydrous sodium sulfate, and concentrated under reduced pressure. The residue was purified by column chromatography (silica gel 20 g, ethyl acetate/hexane 1/5→1/4) to give the title compound (570 mg) as a white amorphous powder.
¹H-NMR (DMSO-d₆, 200 MHz):δ 1.06 (3H, t, J = 7.0 Hz), 1.60-2.46 (10H, m), 3.98-4.10 (4H, m), 4.50-4.80 (1H, m), 5.08 (2H, s), 5.40-5.80 (2H, m), 7.17 (1H, t, J = 2.8 Hz), 7.26-7.35 (6H, m), 7.61-7.67 (2H, m).

### Example 2

### 4-({[((2-chloro-4-fluorophenyl){[2-(ethoxycarbonyl)cyclohex-2-en-1-yl]sulfonyl}amino)methoxy]carbonyl}oxy)butanoic acid

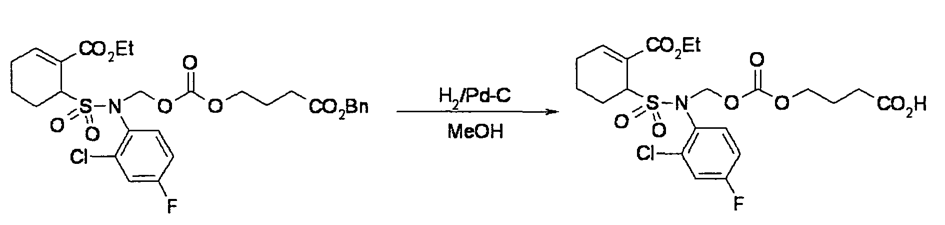

Ethyl 6-{[({[4-(benzyloxy)-4-oxobutoxy]carbonyl}oxy)methyl](2-chloro-4-fluorophenyl)sulfamoyl}cyclohex-1-ene-1-carboxylate (252 mg) obtained in Example 1 was dissolved in methanol (4 mL), 10% palladium-carbon (150 mg) was added, and catalytic hydrogenation was performed at room temperature for 1 hr. The catalyst was filtered off and the filtrate was concentrated under reduced pressure. The obtained residue was purified by column chromatography (silica gel 16 g, ethyl acetate/hexane 1/7→methylene chloride/ethanol 20/1) to give the title compound (116 mg) as a white amorphous powder.
¹H-NMR (CDCl₃, 200 MHz):δ 1.00-1.30 (3H, m), 1.60-2.50 (10H, m), 4.07-4.19 (4H, m), 4.20-4.80 (1H, m), 5.40-6.00 (2H, m), 7.00-7.10 (1H, m), 7.23-7.28 (2H, m), 7.50-7.80 (1H, m).

### Example 3

### sodium 4-({[((2-chloro-4-fluorophenyl){[2-(ethoxycarbonyl)cyclohex-2-en-1-yl]sulfonyl}amino)methoxy]carbonyl}oxy)butanoate

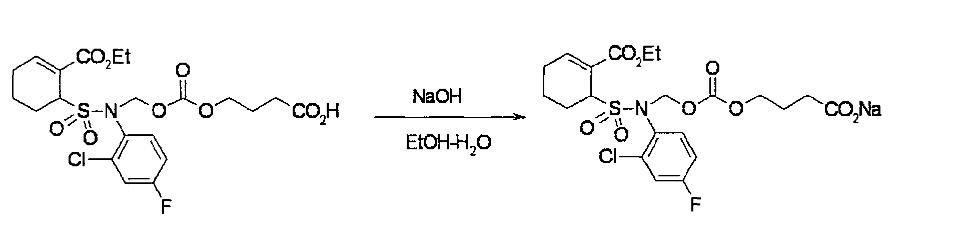

To a solution of 4-({[((2-chloro-4-fluorophenyl){[2-(ethoxycarbonyl)cyclohex-2-en-l-yl]sulfonyl}amino)methoxy]carbonyl}oxy)butanoic acid (104 mg) obtained in Example 2 in ethanol (2 mL)-water (10 mL) was added 1N aqueous sodium hydroxide solution (0.199 mL) under ice-cooling. The solution was concentrated under reduced pressure to a residual amount of about 3 mL, filtered with a membrane filter, and freeze-dried to give the title compound (95.4 mg) as a white amorphous powder.
¹H-NMR (DMSO-d₆, 200 MHz):δ 1.06 (3H, t, J = 7.4 Hz), 1.42-2.43 (8H, m), 3.38-3.44 (2H, m), 3.89-4.30 (4H, m), 4.50-4.80 (1H, m), 5.40-5.80 (2H, m), 7.17 (1H, t, J = 4.0 Hz), 7.32-7.46 (1H, m), 7.58-7.68 (2H, m).
elemental analysis value: as C₂₁H₂₄NO₉SClFNa·H₂O
Calculated (%):C, 44.88; H, 4.66; N, 2.49.
Found (%):C, 44.69; H, 4.55; N, 2.61.

### Example 4

### ethyl 6-{[[({[(1S)-2-(benzyloxy)-1-methyl-2-oxoethoxy]carbonyl}oxy)methyl](2-chloro-4-fluorophenyl)amino]sulfonyl}cyclohex-1-ene-1-carboxylate

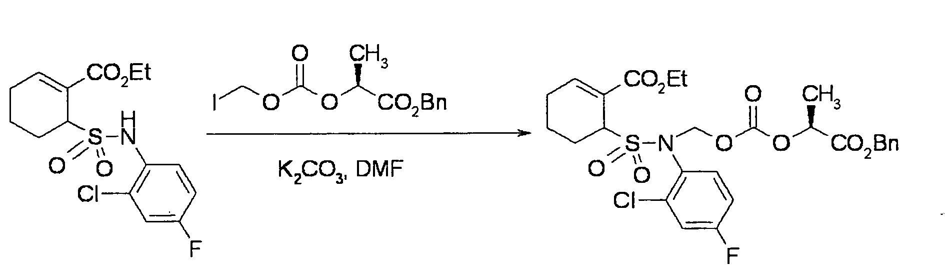

To a solution of ethyl 6-[N-(2-chloro-4-fluorophenyl)sulfamoyl]cyclohex-1-ene-1-carboxylate (500 mg) in N,N-dimethylformamide (5 mL) were added benzyl (2S)-2-{[(iodomethoxy)carbonyl]oxy}propanoate (604 mg) and potassium carbonate (382 mg), and the mixture was stirred at room temperature for 1 hr. The reaction mixture was diluted with ethyl acetate (20 mL), washed with water (20 mLx3) and saturated brine (20 mL), dried over anhydrous sodium sulfate, and concentrated under reduced pressure. The residue was purified by column chromatography (silica gel 20 g, ethyl acetate/hexane 1/10→1/5) to give the title compound (587 mg) as a white amorphous powder.
¹H-NMR (DMSO-d₆, 200 MHz):δ 1.05 (3H, t, J = 7.0 Hz), 1.43 (3H, d, J = 7.0 Hz), 1.60-2.00 (3H, m), 2.14-2.42 (3H, m), 4.03 (2H, q, J = 7.0 Hz), 4.50-4.80 (1H, m), 5.01 (1H, q, J = 7.0 Hz), 5.16 (2H, s), 5.40-5.80 (2H, m), 7.18 (1H, t, J = 2.8 Hz), 7.28-7.35 (6H, m), 7.66 (2H, dd, J = 8.6, 3.0 Hz).

### Example 5

### (2S)-2-({[((2-chloro-4-fluorophenyl){[2-(ethoxycarbonyl)cyclohex-2-en-1-yl]sulfonyl}amino)methoxy]carbonyl}oxy)propanoic acid

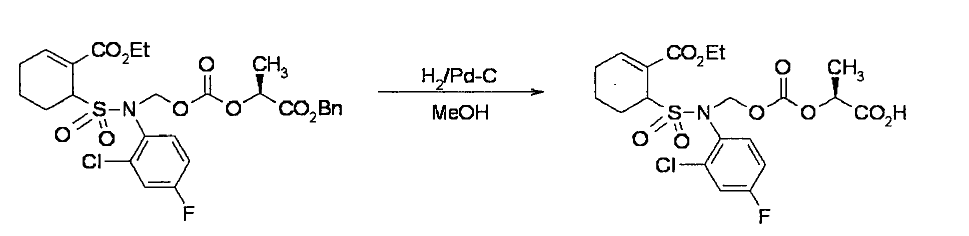

Ethyl 6-{[[({[(1S)-2-(benzyloxy)-1-methyl-2-oxoethoxy]carbonyl}oxy)methyl](2-chloro-4-fluorophenyl)amino]sulfonyl}cyclohex-1-ene-1-carboxylate (312 mg) obtained in Example 4 was dissolved in methanol (5 mL), 10% palladium-carbon (200 mg) was added, and catalytic hydrogenation was performed at room temperature for 30 min. The catalyst was filtered off and the filtrate was concentrated under reduced pressure. The obtained residue was purified by column chromatography (silica gel 10 g, ethyl acetate/hexane 1/3→methylene chloride/ethanol 20/1) to give the title compound (135 mg) as a white amorphous powder. ¹H-NMR (CDCl₃, 200 MHz):δ 1.14-1.23 (3H, m), 1.55 (3H, d, J = 7.0 Hz), 1.60-1.86 (2H, m), 2.05-2.47 (4H, m), 4.07-4.23 (2H, m), 4.50-4.80 (1H, m), 4.97 (1H, q, J = 7.0 Hz), 5.40-6.00 (2H, m), 6.99-7.08 (1H, m), 7.21-7.27 (2H, m), 7.40-7.80 (1H, m), 7.80-8.00 (1H, m).

### Example 6

### sodium (2S)-2-({[((2chloro-4-fluorophenyl){[2-(ethoxycarbonyl)cyclohex-2-en-1-yl]sulfonyl}amirio)methoxy]carbonyl}oxy)propanoate

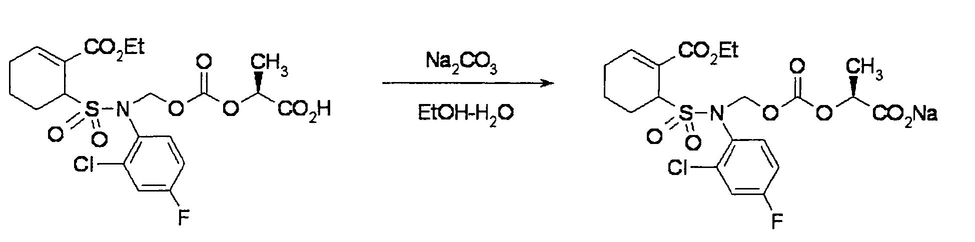

To a solution of (2S)-2-({[((2-chloro-4-fluorophenyl){[2-(ethoxycarbonyl)cyclohex-2-en-1-yl]sulfonyl}amino)methoxy]carbonyl}oxy)propanoic acid (135 mg) obtained in Example 5 in ethanol (5 mL)-water (20 mL) was added an aqueous solution (2 mL) of sodium carbonate (14.03 mg) under ice-cooling, and the mixture was stirred at the same temperature for 10 min. The solution was concentrated under reduced pressure to a residual amount of about 10 mL, filtered with a membrane filter, further concentrated to a residual amount of about 2 mL, and freeze-dried to give the title compound (95.4 mg) as a white amorphous powder.
¹H-NMR (DMSO-d₆, 200 MHz):δ 1.05 (3H, t, J = 7.4 Hz), 1.25 (3H, d, J = 7.0 Hz), 1.60-2.50 (6H, m), 4.03 (2H, q, J = 7.4 Hz), 4.44 (1H, q, J = 7.0 Hz), 4.49-4.80 (1H, m), 5.20-5.80 (2H, m), 7.10-7.20 (1H, m), 7.27-7.37 (1H, m), 7.61-7.79 (2H, m).
elemental analysis value: as C₂₀H₂₂NO₉SClFNa·2.0H₂O
Calculated (%):C, 42.45; H, 4.63; N, 2.37.
Found (%):C, 42.66; H, 4.31; N, 2.37.

### Example 7

### 2-benzyl 1-[((2-chloro-4-fluorophenyl){[2-(ethoxycarbonyl)cyclohex-2-en-1-yl]sulfonyl}amino)methyl] (2S)-pyrrolidine-1,2-dicarboxylate

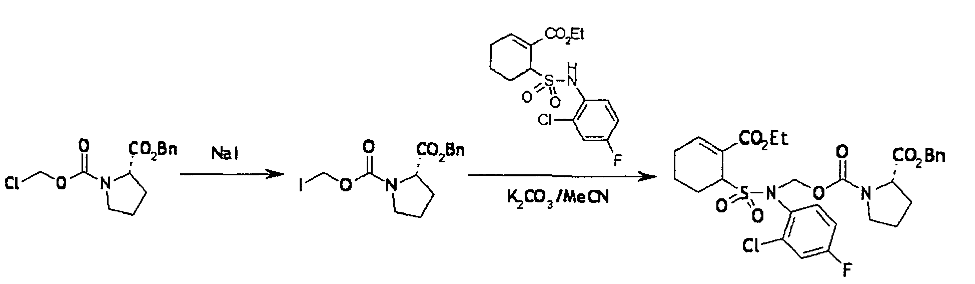

To a solution (15 mL) of 2-benzyl 1-(chloromethyl) (2S)-pyrrolidine-1,2-dicarboxylate (575 mg) obtained in Reference Example 3 in acetonitrile was added sodium iodide (1.11 g), and the mixture was stirred at 60°C for 3.5 hr under a nitrogen atmosphere. The reaction mixture was concentrated under reduced pressure, diluted with diethyl ether, and washed successively with 5% aqueous sodium thiosulfate solution, water and brine. The organic layer was dried over anhydrous magnesium sulfate and concentrated under reduced pressure to give 2-benzyl 1-(iodomethyl) (2S)-pyrrolidine-1,2-dicarboxylate (934 mg). A solution of this product (585 mg) in acetonitrile (2 mL) and potassium carbonate (346 mg) were added to a solution of ethyl 6-[N-(2-chloro-4-fluorophenyl)sulfamoyl]cyclohex-1-ene-1-carboxylate (453 mg) in acetonitrile (10 mL), and the mixture was stirred at room temperature for 1 hr. The reaction mixture was diluted with ethyl acetate, washed with water and brine, dried over anhydrous magnesium sulfate, and concentrated under reduced pressure. The residue was purified by silica gel column chromatography (ethyl acetate-hexane) to give the title compound (409 mg) as an amorphous powder.
¹H-NMR(DMSO-d₆, 200MHz):δ 1.06 (3H, t, J = 6.6 Hz), 1.54-1.98 (6H, m), 2.04-2.49 (4H, m), 3.24-3.54 (2H, m), 4.30 (2H, q, J= 6.6 Hz), 4.20-4.80 (2H, m), 5.11 (2H, br s), 5.33-5.71 (2H, m), 7.14 (1H, br s), 7.22-7.40 (6H, m), 7.58-7.69 (2H, m).

### Example 8

### 1-{[((2-chloro-4-fluorophenyl){[2-(ethoxycarbonyl)cyclohex-2-en-1-yl]sulfonyl}amino)methoxy]carbonyl}-L-proline

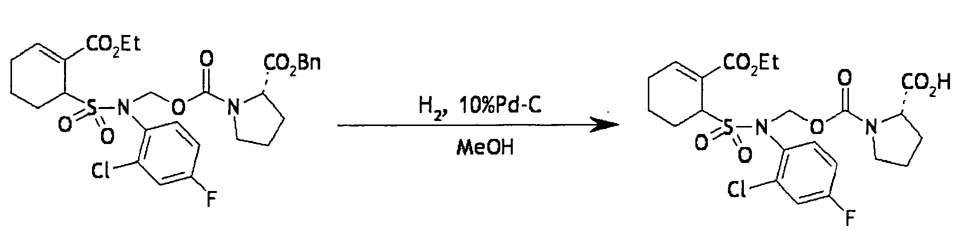

2-Benzyl 1-[((2-chloro-4-fluorophenyl){[2-(ethoxycarbonyl)cyclohex-2-en-1-yl]sulfonyl}amino)methyl] (2S)-pyrrolidine-1,2-dicarboxylate (230 mg) obtained in Example 7 was dissolved in methanol (6 mL), 10% palladium-carbon (203 mg) was added, and catalytic hydrogenation was performed at room temperature for 3 hr. The catalyst was filtered off and the filtrate was concentrated under reduced pressure. The obtained residue was purified by silica gel column chromatography (methanol-ethyl acetate) to give the title compound (148 mg) as an amorphous powder. ¹H-NMR(DMSO-d₆, 200MHz):δ 0.94-1.11 (3H, m), 1.51-1.98 (6H, m), 1.99-2.46 (4H, m), 3.16-3.47 (2H, m), 3.98-4.12 (2H, m), 4.19 (1H, dd, J= 5.2, 3.0 Hz), 4.53-4.79 (1H, m), 5.27-5.66 (2H, m), 7.13 (1H, br s), 7.27-7.37 (1H, m), 7.53-7.88 (3H, m).

### Example 9

### sodium (2S)-1-{[((2-chloro-4-fluorophenyl){[2-(ethoxycarbonyl)cyclohex-2-en-1-yl]sulfonyl}amino)methoxy]carbonyl}pyrrolidine-2-carboxylate

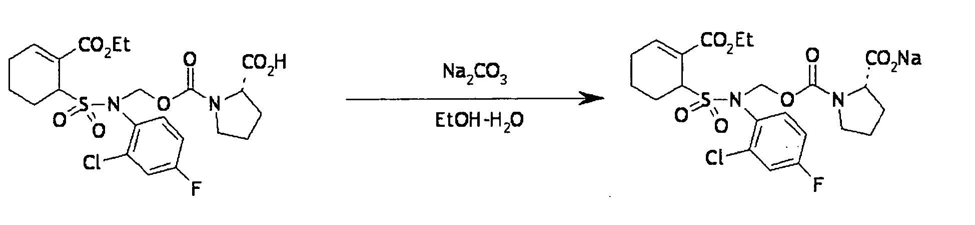

To a mixture of 1-{[((2-chloro-4-fluorophenyl){[2-(ethoxycarbonyl)cyclohex-2-en-1-yl]sulfonyl}amino)methoxy]carbonyl}-L-proline (99 mg) obtained in Example 8, ethanol (4 mL) and water (16 mL) was added an aqueous solution (2 mL) of sodium carbonate (9.84 mg) under ice-cooling, and the mixture was stirred for 20 min. The reaction mixture was concentrated under reduced pressure to a residual amount of about 10 mL, filtered with a membrane filter, and freeze-dried to give the title compound (96 mg) as a white powder.
¹H-NMR(DMSO-d₆, 200MHz):δ 0.89 (3H, t, J = 7.0 Hz), 1.05-2.41 (10H, m), 3.15-3.35 (2H, m), 3.79-4.13 (3H, m), 4.67-4.90 (1H, m), 5.26-5.58 (2H, m), 7.11 (1H, m), 7.22-7.38 (1H, m), 7.50-7.75 (2H, m).

### Example 10

### 2-benzyl 1-[((2-chloro-4-fluorophenyl){[2-(ethoxycarbonyl)cyclohex-2-en-1-yl]sulfonyl}amino)methyl] (2R)-pyrrolidine-1,2-dicarboxylate

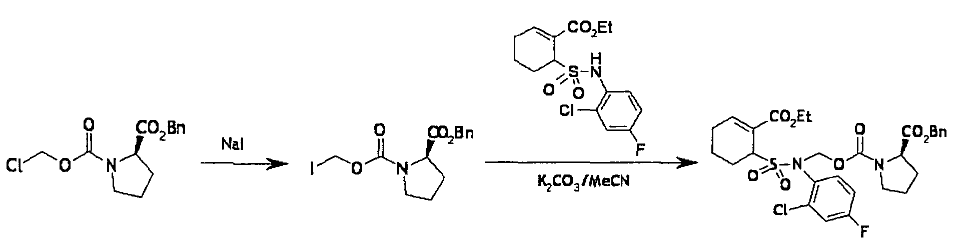

To a solution (60 mL) of 2-benzyl 1-(chloromethyl) (2R)-pyrrolidine-1,2-dicarboxylate (2.31 g) obtained in Reference Example 4 in acetonitrile was added sodium iodide (4.65 g), and the mixture was stirred at 60°C for 3 hr under a nitrogen atmosphere. The reaction mixture was concentrated under reduced pressure, diluted with diethyl ether, and washed successively with 5% aqueous sodium thiosulfate solution, water and brine. The organic layer was dried over anhydrous magnesium sulfate and concentrated under reduced pressure to give 2-benzyl 1-(iodomethyl) (2R)-pyrrolidine-1,2-dicarboxylate (2.06 g). A solution of this product (2.05 g) in acetonitrile (5 mL) and potassium carbonate (1.21 g) were added to a solution of ethyl 6-[N-(2-chloro-4-fluorophenyl)sulfamoyl]cyclohex-1-ene-1-carboxylate (1.59 g) in acetonitrile (35 mL), and the mixture was stirred at room temperature for 12 hr. The reaction mixture was diluted with ethyl acetate, washed with water and brine, dried over anhydrous magnesium sulfate, and concentrated under reduced pressure. The residue was purified by silica gel column chromatography (ethyl acetate-hexane) to give the title compound (2.15 g) as an amorphous powder.
¹H-NMR(DMSO-d₆, 20OMHz):δ 0.99-1.09 (3H, m), 1.52-1.96 (6H, m), 2.06-2.46 (4H, m), 3.39-3.51 (2H, m), 3.95-4.08 (2H, m), 4.21-4.27 (1H, m), 4.40-4.77 (1H, m), 5.11 (2H, br s), 5.26-5.48 (2H, m), 7.14 (1H, br s), 7.20-7.34 (6H, m), 7.57-7.65 (2H, m).

### Example 11

### 1-{[((2-chloro-4-fluorophenyl){[2-(ethoxycarbonyl)cyclohex-2-en-1-yl]sulfonyl}amino)methoxy]carbonyl}-D-proline

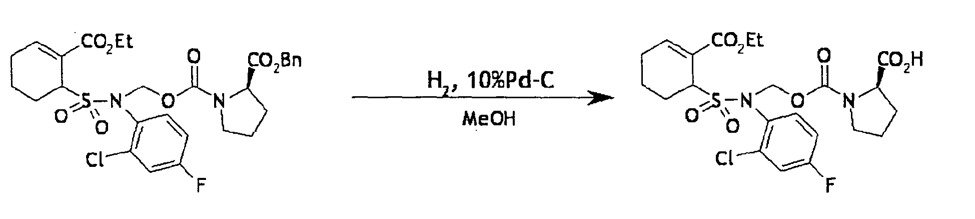

2-Benzyl 1-[((2-chloro-4-fluorophenyl){[2-(ethoxycarbonyl)cyclohex-2-en-1-yl]sulfonyl}amino)methyl] (2R)-pyrrolidine-1,2-dicarboxylate (1.24 g) obtained in Example 10 was dissolved in methanol (30 mL), 10% palladium-carbon (1.24 g) was added, and catalytic hydrogenation was performed at room temperature for 4.5 hr. The catalyst was filtered off and the filtrate was concentrated under reduced pressure. The obtained residue was purified by silica gel column chromatography (methanol-ethyl acetate) to give the title compound (751 mg) as an amorphous powder.
¹H-NMR(DMSO-d₆, 200MHz):δ 1.05 (3H, t, J = 7.0 Hz), 1.55-1.70 (2H, m), 1.74-1.98 (4H, m), 2.07-2.42 (4H, m), 3.36-3.46 (1H, m), 4.03 (2H, q, J = 7.0 Hz), 4.20-4.27 (1H, m), 4.65 (1H, br s), 5.29-5.62 (2H, m), 7.13 (1H, m), 7.24-7.35 (1H, m), 7.60-7.70 (2H, m).

### Example 12

### sodium (2R)-1-{[((2-chloro-4-fluorophenyl){[2-(ethoxycarbonyl)cyclohex-2-en-1-yl]sulfonyl}amino)methoxy]carbonyl}pyrrolidine-2-carboxylate

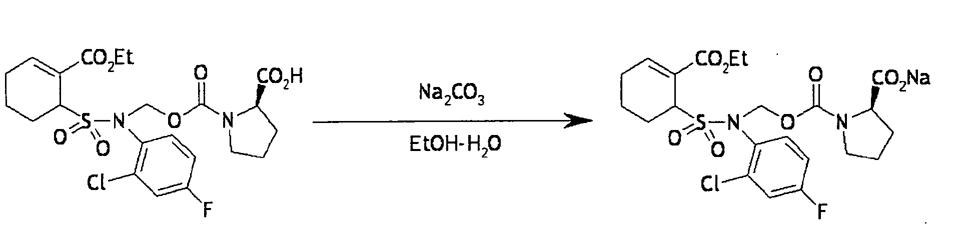

To a mixture of 1-{[((2-chloro-4-fluorophenyl){[2-(ethoxycarbonyl)cyclohex-2-en-1-yl]sulfonyl}amino)methoxy]carbonyl}-D-proline (130 mg) obtained in Example 11, ethanol (5 mL) and water (20 mL) was added aqueous sodium carbonate (12.9 mg) solution (2 mL) under ice-cooling, and the mixture was stirred for 20 min. The reaction mixture was concentrated under reduced pressure to a residual amount of about 10 mL, filtered with a membrane filter, and freeze-dried to give the title compound (122 mg) as a white powder.
¹H-NMR(DMSO-d₆, 200MHz):δ 0.85-1.19 (3H, m), 1.55-2.11 (7H, m), 2.11-2.45 (3H, m), 3.14-3.34 (2H, m), 3.76-4.16 (3H, m), 4.66-4.90 (1H, m), 5.23-5.60 (2H, m), 7.10 (1H, br), 7.22-7.36 (1H, m), 7.52-7.77 (2H, m).

### Example 13

### ((2-chloro-4-fluorophenyl){[2-(ethoxycarbonyl)cyclohex-2-en-1-yl]sulfonyl}amino)methyl 4-[2-(benzyloxy)-2-oxoethyl]piperidine-1-carboxylate

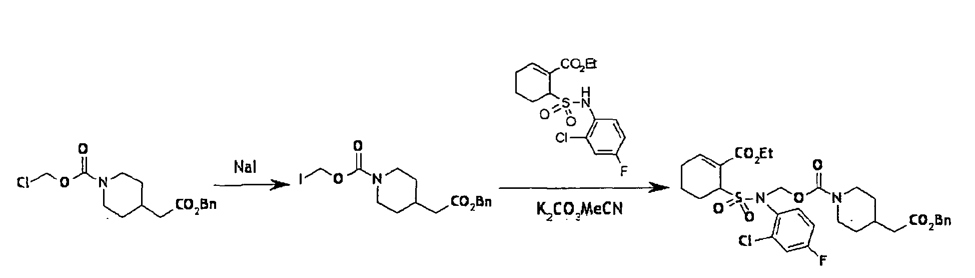

To a solution (10 mL) of chloromethyl 4-[2-(benzyloxy)-2-oxoethyl]piperidine-1-carboxylate (400 mg) obtained in Reference Example 5 in acetonitrile was added sodium iodide (736 mg), and the mixture was stirred at 60°C for 1.5 hr under a nitrogen atmosphere. The reaction mixture was concentrated under reduced pressure, diluted with diethyl ether, and washed successively with 5% aqueous sodium thiosulfate solution, water and brine. The organic layer was dried over anhydrous magnesium sulfate and concentrated under reduced pressure to give iodomethyl 4-[2-(benzyloxy)-2-oxoethyl]piperidine-1-carboxylate (451 mg). A solution of this product (451 mg) in acetonitrile (3 mL) and potassium carbonate (199 mg) were added to a solution of ethyl 6-[N-(2-chloro-4-fluorophenyl)sulfamoyl]cyclohex-1-ene-1-carboxylate (283 mg) in acetonitrile (5 mL), and the mixture was stirred at room temperature for 2 hr. The reaction mixture was diluted with ethyl acetate, washed with water and brine, dried over anhydrous magnesium sulfate, and concentrated under reduced pressure. The residue was purified by silica gel column chromatography (ethyl acetate-hexane) to give the title compound (455 mg) as an oil.
¹H-NMR(DMSO-d₆, 200MHz):δ 1.02-1.22 (2H, m), 1.06 (3H, t, J= 7.0 Hz), 1.64 (2H, br d, J = 11.6 Hz), 1.67-2.50 (7H, m), 2.32 (2H, d, J = 6.6 Hz), 2.64-2.86 (2H, m), 3.80-3.97 (2H, m), 4.03 (2H, q, J = 7.0 Hz), 4.55-4.72 (1H, m), 5.09 (2H, s), 5.49 (2H, br), 7.16 (1H, m), 7.28-7.36 (6H, m), 7.61-7.66 (2H, m).

### Example 14

### (1-{[((2-chloro-4-fluorophenyl){[2-(ethoxycarbonyl)cyclohex-2-en-1-yl]sulfonyl}amino)methoxy]carbonyl}piperidin-4-yl)acetic acid

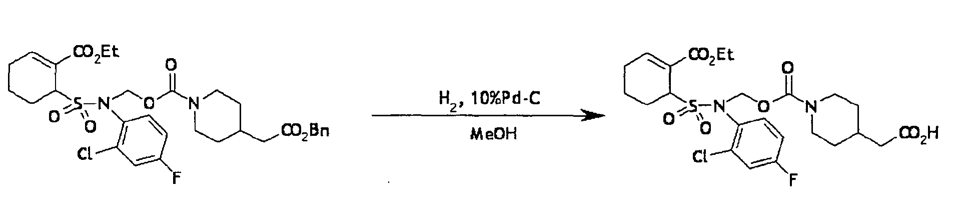

Ethyl ((2-chloro-4-fluorophenyl){[2-(ethoxycarbonyl)cyclohex-2-en-1-yl]sulfonyl}amino)methyl 4-[2-(benzyloxy)-2-oxoethyl]piperidine-1-carboxylate (318 mg) obtained in Example 13 was dissolved in methanol (8 mL), 10% palladium-carbon (322 mg) was added, and catalytic hydrogenation was performed at room temperature for 2 hr. The catalyst was filtered off and the filtrate was concentrated under reduced pressure. The obtained residue was purified by silica gel column chromatography (methanol-ethyl acetate) to give the title compound (190 mg) as an amorphous powder.
¹H-NMR(DMSO-d₆, 200MHz):δ 1.06 (3H, t, J = 7.2 Hz), 1.63-1.70 (2H, m), 1.80-2.49 (8H, m), 2.16 (2H, d, J = 6.6 Hz), 2.63-2.92 (2H, m), 3.83-4.09 (5H, m), 4.55-4.77 (1H, m), 5.56 (2H, br d), 7.16 (1H, br), 7.32-7.39 (1H, m), 7.62-7.68 (2H, m).

### Example 15

### sodium (1-{[((2-chloro-4-fluorophenyl){[2-(ethoxycarbonyl)cyclohex-2-en-1-yl]sulfonyl}amino)methoxy]carbonyl}piperidin-4-yl)acetate

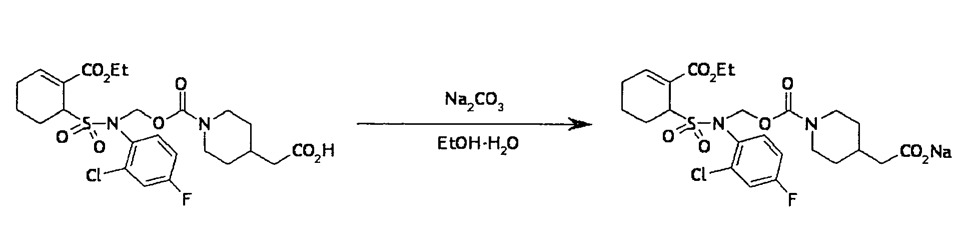

To a mixture of (1-{[((2-chloro-4-fluorophenyl){[2-(ethoxycarbonyl)cyclohex-2-en-1-yl]sulfonyl}amino)methoxy]carbonyl}piperidin-4-yl)acetic acid (70 mg) obtained in Example 14, ethanol (3 mL) and water (9.5 mL) was added an aqueous solution (1.5 mL) of sodium carbonate (6.6 mg) under ice-cooling, and the mixture was stirred for 15 min. The reaction mixture was concentrated under reduced pressure to a residual amount of about 6 mL, filtered with a membrane filter, and freeze-dried to give the title compound (67 mg) as a white powder.
¹H-NMR(DMSO-d₆, 200MHz):δ 0.86-1.10 (3H, m), 1.06 (3H, t, J= 7.2 Hz), 1.58-2.39 (10H, m), 2.61-2.90 (2H, m), 3.81-4.08 (2H, m), 4.03 (2H, q, J = 7.2 Hz), 4.56 (1H, br), 5.51 (2H, br), 7.15 (1H, m), 7.30-7.39 (1H, m), 7.58-7.67 (2H, m).

### Example 16

### ethyl 6-{[{[({[2-(benzyloxy)-2-oxoethyl]amino}carbonyl)oxy]methyl}(2-chloro-4-fluorophenyl)amino]sulfonyl}cyclohex-1-ene-1-carboxylate

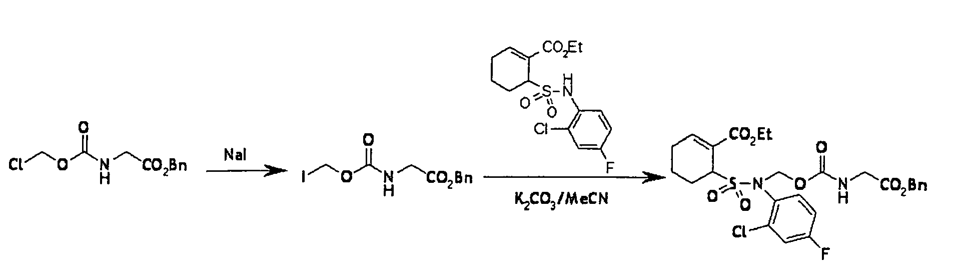

To a solution (25 mL) of benzyl N-[(chloromethoxy)carbonyl]glycinate (1.0 g) obtained in Reference Example 6 in acetonitrile was added sodium iodide (2.33 g), and the mixture was stirred at 60°C for 1.5 hr under a nitrogen atmosphere. The reaction mixture was concentrated under reduced pressure, diluted with diethyl ether, and washed successively with 5% aqueous sodium thiosulfate solution, water and brine. The organic layer was dried over anhydrous magnesium sulfate and concentrated under reduced pressure to give benzyl N-[(iodomethoxy)carbonyl]glycinate (1.29 g). A solution of this product (813 mg) in acetonitrile (3 mL) and potassium carbonate (429 mg) were added to a solution of ethyl 6-[N-(2-chloro-4-fluorophenyl)sulfamoyl]cyclohex-1-ene-1-carboxylate (702 mg) in acetonitrile (12 mL), and the mixture was stirred at room temperature for 1.5 hr. The reaction mixture was diluted with ethyl acetate, washed with water and brine, dried over anhydrous magnesium sulfate, and concentrated under reduced pressure. The residue was purified by silica gel column chromatography (ethyl acetate-hexane) to give the title compound (121 mg) as an oil.
¹H-NMR(DMSO-d₆, 200MHz):δ 1.04 (3H, t, J = 7.0 Hz), 1.56-1.98 (3H, m), 1.99-2.38 (3H, m), 3.77 (2H, d, J = 6.4 Hz), 4.01 (2H, q, J= 7.0 Hz), 4.56 (1H, br), 5.12 (2H, s), 5.49 (2H, m), 7.15 (1H, m), 7.22-7.35 (6H, m), 7.55-7.65 (2H, m), 7.91-7.97 (1H, m).

### Example 17

### N-{[((2-chloro-4-fluorophenyl){[2-(ethoxycarbonyl)cyclohex-2-en-1-yl]sulfonyl}amino)methoxy]carbonyl}glycine

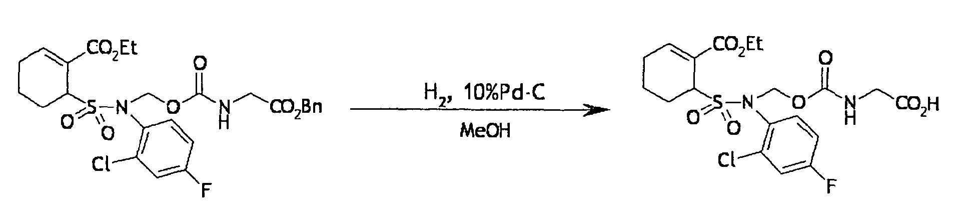

Ethyl 6-{[{[({[2-(benzyloxy)-2-oxoethyl]amino}carbonyl)oxy]methyl}(2-chloro-4-fluorophenyl)amino]sulfonyl}cyclohex-1-ene-1-carboxylate (79 mg) obtained in Example 16 was dissolved in methanol (2 mL), 10% palladium-carbon (78 mg) was added, and catalytic hydrogenation was performed at room temperature for 1.5 hr. The catalyst was filtered off and the filtrate was concentrated under reduced pressure. The obtained residue was purified by silica gel column chromatography (methanol-ethyl acetate) to give the title compound (48 mg) as an amorphous powder.
¹H-NMR(DMSO-d₆, 200MHz):δ 1.04 (3H, t, J = 7.0 Hz), 1.58-2.50 (6H, m), 3.33 (2H, d, J= 5.2 Hz), 4.01 (2H, q, J = 7.0 Hz), 4.57 (1H, m), 5.47 (2H, br d), 6.89 (1H, br s), 7.14 (1H, br s), 7.26-7.35 (1H, m), 7.60-7.70 (2H, m).

### Example 18

### benzyl N-{[((2-chloro-4-fluorophenyl){[2-(ethoxycarbonyl)cyclohex-2-en-1-yl]sulfonyl}amino)methoxy]carbonyl}-D-leucinate

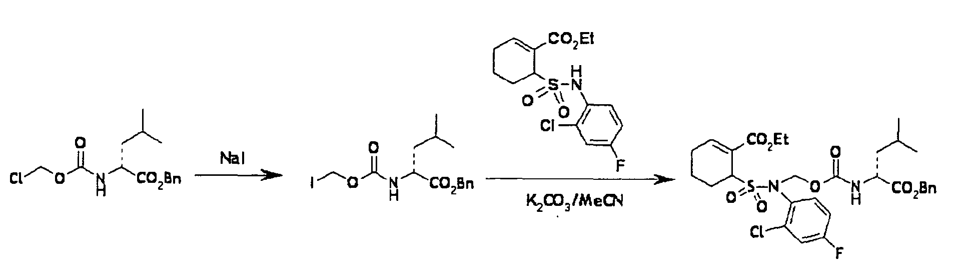

To a solution (18 mL) of benzyl N-[(chloromethoxy)carbonyl]-D-leucinate (800 mg) obtained in Reference Example 7 in acetonitrile was added sodium iodide (1.53 g), and the mixture was stirred at 60°C for 1.5 hr under a nitrogen atmosphere. The reaction mixture was concentrated under reduced pressure, diluted with diethyl ether, and washed successively with 5% aqueous sodium thiosulfate solution, water and brine. The organic layer was dried over anhydrous magnesium sulfate and concentrated under reduced pressure to give benzyl N-[(iodomethoxy)carbonyl]-D-leucinate (1.03 g). A solution of this product (1.0 g) in acetonitrile (3 mL) and potassium carbonate (397 mg) were added to a solution of ethyl 6-[N-(2-chloro-4-fluorophenyl)sulfamoyl]cyclohex-1-ene-1-carboxylate (631 mg) in acetonitrile (15 mL), and the mixture was stirred at room temperature for 2.5 hr. The reaction mixture was diluted with ethyl acetate, washed with water and brine, dried over anhydrous magnesium sulfate, and concentrated under reduced pressure. The residue was purified by silica gel column chromatography (ethyl acetate-hexane) to give the title compound (604 mg) as an oil.
¹H-NMR(DMSO-d₆, 200MHz):δ 0.79 (3H, d, J = 6.0 Hz), 0.87 (3H, d, J = 6.0 Hz), 1.00-1.08 (3H, m), 1.43-1.98 (6H, m), 1.99-2.42 (3H, m), 3.98.-4.08 (3H, m), 4.51 (1H, m), 5.10 (2H, s), 5.54 (2H, br s), 7.15 (1H, br s), 7.22-7.36 (6H, m), 7.54-7.65 (2H, m), 7.99 (1H, d, J = 7.6 Hz).

### Example 19

### N-{[((2-chloro-4-fluorophenyl){[2-(ethoxycarbonyl)cyclohex-2-en-1-yl]sulfonyl}amino)methoxy]carbonyl}-D-leucine

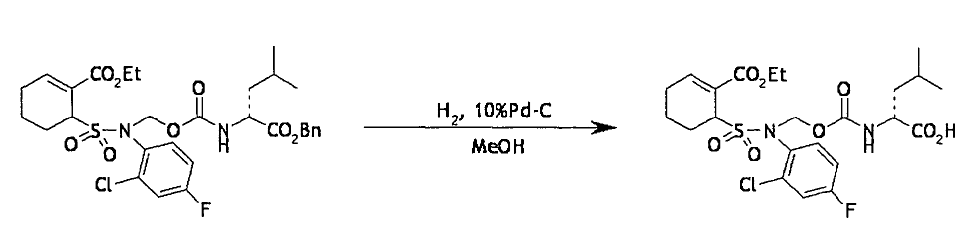

Benzyl N-{[((2-chloro-4-fluorophenyl){[2-(ethoxycarbonyl)cyclohex-2-en-1-yl]sulfonyl}amino)methoxy]carbonyl}-D-leucinate (325 mg) obtained in Example 18 was dissolved in methanol (7 mL), 10% palladium-carbon (304 mg) was added, and catalytic hydrogenation was performed at room temperature for 1.5 hr. The catalyst was filtered off and the filtrate was concentrated under reduced pressure. The obtained residue was purified by silica gel column chromatography (methanol-ethyl acetate) to give the title compound (207 mg) as an amorphous powder.
¹H-NMR (DMSO-d₆, 200MHz):δ 0.80 (3H, d, J = 6.0 Hz), 0.87 (3H, d, J = 6.0 Hz), 1.00-1.09 (3H, m), 1.46-1.59 (2H, m), 1.60-2.39 (7H, m), 2.93-3.90 (1H, br), 3.75-3.86 (1H, m), 3.94-4.09 (2H, m), 4.55-4.80 (1H, m), 5.36-5.53 (2H, m), 7.14 (1H, br s), 7.23-7.63 (4H, m).

### Example 20

### ethyl 6-{[(4-{[N-(tert-butoxycarbonyl)-N-methylglycyl]oxy}-3,5-dimethylbenzyl)(2-chloro-4-fluorophenyl)amino]sulfonyl}cyclohex-1-ene-1-carboxylate

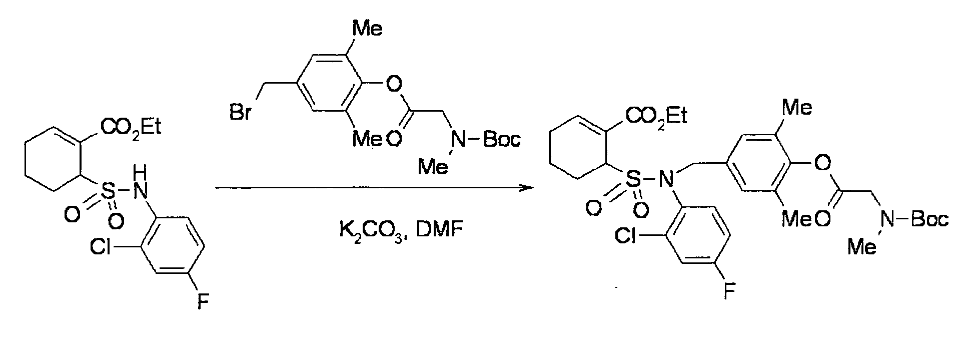

To a solution of ethyl 6-[N-(2-chloro-4-fluorophenyl)sulfamoyl]cyclohex-1-ene-1-carboxylate (500 mg) in N,N-dimethylformamide (5 mL) were added 4-(bromomethyl)-2,6-dimethylphenyl N-(tert-butoxycarbonyl)-N-methylglycinate (641 mg) and potassium carbonate (229 mg), and the mixture was stirred at room temperature for 30 min. The reaction mixture was diluted with ethyl acetate (100 mL), washed with water (100 mL×3) and saturated brine (100 mL), dried over anhydrous sodium sulfate, and concentrated under reduced pressure. The residue was purified by column chromatography (silica gel 25 g, ethyl acetate/hexane 1/5) to give the title compound (636 mg) as a white amorphous powder.
¹H-NMR (DMSO-d₆, 200 MHz):δ 1.00-1.22 (3H, m), 1.38 (4.5H, s), 1.41 (4.5H, s), 1.61-2.41 (12H, m), 2.89 (1.5H, s), 2.94 (1.5H, s), 3.98-4.09 (2H, m), 4.26-4.32 (2H, m), 4.65-4.81 (3H, m), 6.97 (2H, s), 7.15-7.24 (2H, m), 7.50 (2H, dd, J = 8.4, 3.0 Hz).
elemental analysis value: as C₃₂H₄₀N₂O₈SClF
Calculated (%): C, 57.61; H, 6.04; N, 4.20.
Found (%): C, 57.58; H, 5.99; N, 4.28.

### Example 21

### ethyl 6-[((2-chloro-4-fluorophenyl){3,5-dimethyl-4-[(N-methylglycyl)oxy]benzyl}amino)sulfonyl]cyclohex-1-ene-1-carboxylate hydrochloride

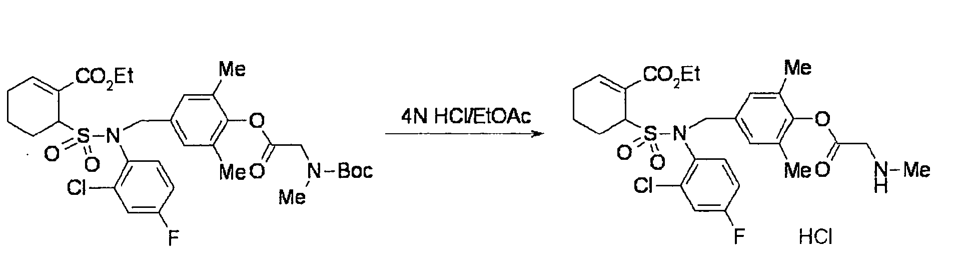

To a solution of ethyl 6-{[(4-{[N-(tert-butoxycarbonyl)-N-methylglycyl]oxy}-3,5-dimethylbenzyl)(2-chloro-4-fluorophenyl)amino]sulfonyl}cyclohex-1-ene-1-carboxylate (176 mg) obtained in Example 20 in ethyl acetate (1 mL) was added 4N hydrogen chloride-ethyl acetate solution (2 mL) under ice-cooling, and the mixture was stirred at room temperature for 1 hr. The solvent was evaporated under reduced pressure, and the residue was purified by Amberlyst A-27 (Cl⁻) (resin 1 g, MeOH) and crystallized from diisopropyl ether to give the title compound (139 mg) as a white amorphous powder.
¹H-NMR (DMSO-d₆, 200 MHz): δ 1.02-1.10 (3H, m), 1.66-2.40 (6H, m), 2.06 (6H, s), 2.63 (3H, s), 4.00-4.20 (2H, m), 4.35 (2H, s), 4.60-5.00 (3H, m), 6.99 (2H, s), 7.15-7.25 (2H, m), 7.52 (2H, dd, J = 8.0, 2.6 Hz), 9.81 (2H, brs).
elemental analysis value: as C₂₇H₃₃N₂O₆SCl₂F·1.5H₂O
Calculated (%): C, 51.43; H, 5.75; N, 4.44.
Found (%): C, 51.77; H, 5.77; N, 4.29.

### Example 22

### 2-{4-[((2-chloro-4-fluorophenyl){[2-(ethoxycarbonyl)cyclohex-2-en-1-yl]sulfonyl}amino)methyl]-2,6-dimethylphenyl} 1-tert-butyl (2S)-pyrrolidine-1,2-dicarboxylate

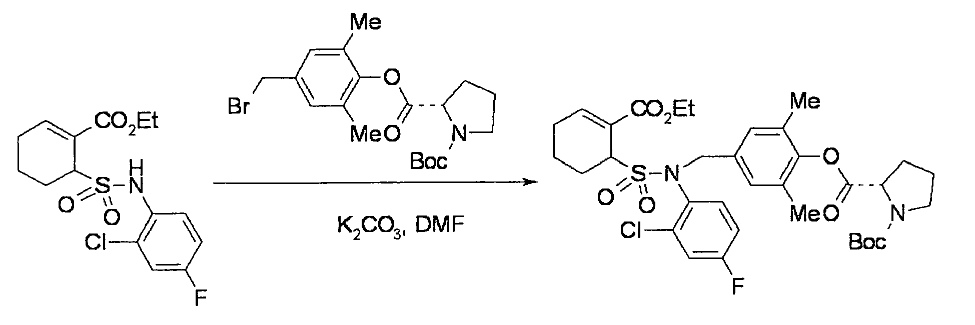

To a solution of ethyl 6-[N-(2-chloro-4-fluorophenyl)sulfamoyl]cyclohex-1-ene-1-carboxylate (500 mg) in N,N-dimethylformamide (5 mL) were added 1-tert-butyl 2-[4-(bromomethyl)-2,6-dimethylphenyl] (2S)-pyrrolidine-1,2-dicarboxylate (684 mg) and potassium carbonate (229 mg), and the mixture was stirred at room temperature for 2 hr. The reaction mixture was diluted with ethyl acetate (100 mL), washed with water (100 mL×3) and saturated brine (100 mL), dried over anhydrous sodium sulfate, and concentrated under reduced pressure. The residue was purified by column chromatography (silica gel 25 g, ethyl acetate/hexane 1/5) to give the title compound (856 mg) as a white amorphous powder.
¹H-NMR (DMSO-d₆, 200 MHz) :δ 1.00 -1.17 (3H, m), 1.37 (4.5H, s), 1.40 (4.5H, m), 1.50-2.41 (16H, m), 3.31 -3.41 (2H, m), 4.01-4.08 (2H, m), 4.43-4.80 (4H, m), 6.94-6.97 (2H, m), 7.14-7.24 (2H, m), 7.10 (2H, dd, J = 8.4, 3.0 Hz).
elemental analysis value: as C₃₄H₄₂N₂O₈SClF
Calculated (%): C, 58.91; H, 6.11; N, 4.04.
Found (%): C, 58,77; H, 5.84; N, 4.02.

### Example 23

### 4-[((2-chloro-4-fluorophenyl){[2-(ethoxycarbonyl)cyclohex-2-en-1-yl]sulfonyl}amino)methyl]-2,6-dimethylphenyl L-prolinate

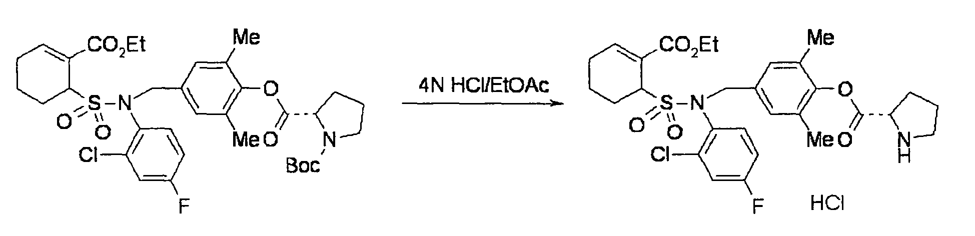

To a solution of 2-{4-[((2-chloro-4-fluorophenyl){[2-(ethoxycarbonyl)cyclohex-2-en-1-yl]sulfonyl}amino)methyl]-2,6-dimethylphenyl} 1-tert-butyl (2S)-pyrrolidine-1,2-dicarboxylate (200 mg) obtained in Example 22 in ethyl acetate (1 mL) was added 4N hydrogen chloride-ethyl acetate solution (2 mL) under ice-cooling, and the mixture was stirred at room temperature for 2 hr. The solvent was evaporated under reduced pressure, and the residue was purified by Amberlyst A-27(Cl⁻) (resin 2 g, MeOH) and crystallized from diisopropyl ether to give the title compound (128 mg) as a white amorphous powder.
¹H-NMR (DMSO-d₆, 200 MHz) :δ 1.02-1.30 (3H, m), 1.65-2.56 (10H, m), 2.06 (6H, s), 3.18-3.39 (2H, m), 4.00-4.09 (2H, m), 4.60-4.81 (4H, m),7.00 (2H, s), 7.15-7.27 (2H, m), 7.52 (2H, dd, J = 8.4, 3.0 Hz), 10.05 (2H, br s).
elemental analysis value: as C₂₉H₃₅N₂O₆SCl₂F·H₂O
Calculated (%): C, 53.79; H, 5.76; N, 4.33.
Found (%): C, 54.01; H, 5.64; N, 4.16.

### Example 24

### ethyl 6-({(2-chloro-4-fluorophenyl)[5-oxo-5-(2,2,2-trichloroethoxy)pentanoyl]amino}sulfonyl)cyclohex-1-ene-1-carboxylate

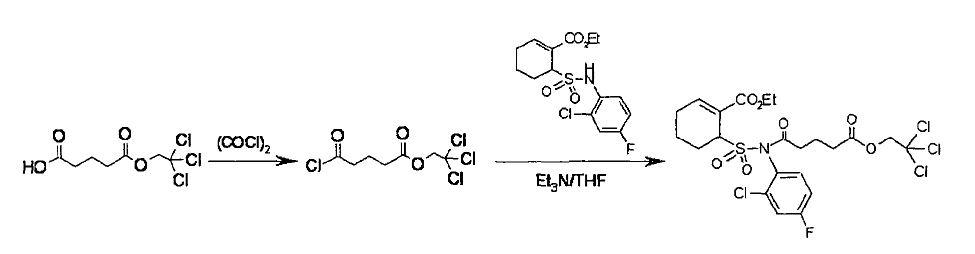

To a mixture of 5-oxo-5-(2,2,2-trichloroethoxy)pentanoic acid (4.0 g), tetrahydrofuran (12 mL) and N,N-dimethylformamide (30 µL) was added dropwise oxalyl chloride (1.85 mL), and the mixture was stirred at room temperature for 40 min. The solvent was evaporated, and the residue was dissolved in tetrahydrofuran (10 mL) and concentrated under reduced pressure. This operation was repeated three times to give 5-oxo-5-(2,2,2-trichloroethoxy)pentanoyl chloride. A solution of this product in tetrahydrofuran (5 mL) was added dropwise to a solution of ethyl 6-[N-(2-chloro-4-fluorophenyl)sulfamoyl]cyclohex-1-ene-1-carboxylate (2.90 g) and triethylamine (1.12 mL) in tetrahydrofuran (15 mL) under ice-cooling, and the mixture was stirred for 1 hr under ice-cooling and at room temperature for 3 hr. The reaction mixture was diluted with ethyl acetate (100 mL), washed successively with water (100 mL), 5% aqueous sodium bicarbonate (50 mL) and saturated brine (50 mL), dried over anhydrous magnesium sulfate, and concentrated under reduced pressure. The residue was purified by column chromatography (silica gel 50 g, ethyl acetate/hexane 1/3) to give the title compound (3.67 g) as a colorless oil.
¹H-NMR (CDCl₃, 200 MHz):δ 1.34 (3H, t, J = 7.2 Hz), 1.72-2.95 (12H, m), 4.17-4.32 (2H, m), 4.73 (2H, d, J = 8.2 Hz), 4.98, 5.33 (1H, br), 7.00-7.20 (1H, m), 7.25-7.80 (3H, m).

### Example 25

### 5-((2-chloro-4-fluorophenyl){[2-(ethoxycarbonyl)cyclohex-2-en-1-yl]sulfonyl}amino)-5-oxopentanoic acid

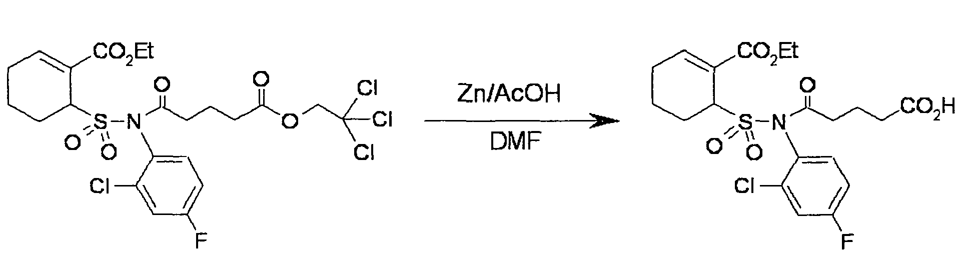

Ethyl 6-({(2-chloro-4-fluorophenyl)[5-oxo-5-(2,2,2-trichloroethoxy)pentanoyl]amino}sulfonyl)cyclohex-1-ene-1-carboxylate (2.4 g) obtained in Example 24 was dissolved in N,N-dimethylformamide (10 mL), zinc powder (0.50 g) and acetic acid (0.72 mL) were added under ice-cooling, and the mixture was stirred for 15 min under ice-cooling and at room temperature for 1 hr. The reaction mixture was poured into ice water (80 mL), and the mixture was extracted with ethyl acetate (80 mL). The ethyl acetate layer was washed with water (80 mL) and saturated brine (50 mL), dried over anhydrous magnesium sulfate, and concentrated under reduced pressure. The residue was purified by column chromatography (silica gel 30 g, ethyl acetate/hexane 1/3→ ethyl acetate/ethanol 19/1) to give the title compound (1.35 g) as a colorless oil.
¹H-NMR (CDCl₃, 300 MHz):δ 1.33 (3H, t, J = 7.2 Hz), 1.75-2.94 (12H, m), 4.18-4.42 (2H, m), 5.00, 5.33 (1H, br), 7.01-7.15 (1H, m), 7.25-7.76 (3H, m).
SIMS m/z:476 [M+H].

### Example 26

### ethyl 6-({(2-chloro-4-fluorophenyl)[4-oxo-4-(2,2,2-trichloroethoxy)butanoyl]amino}sulfonyl)cyclohex-1-ene-1-carboxylate

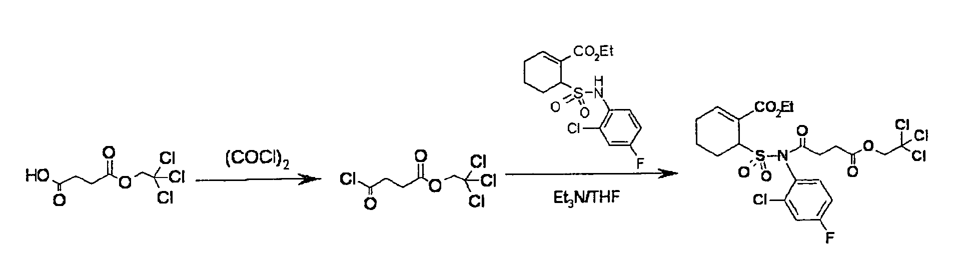

To a mixture of 4-oxo-4-(2,2,2-trichloroethoxy)butanoic acid (2.0 g), tetrahydrofuran (6 mL) and N,N-dimethylformamide (20 µL) was added dropwise oxalyl chloride (1.0 mL), and the mixture was stirred at room temperature for 1 hr. The solvent was evaporated, and the residue was dissolved in tetrahydrofuran (10 mL) and concentrated under reduced pressure. This operation was repeated three times to give 4-oxo-4-(2,2,2-trichloroethoxy)butanoyl chloride. A solution of this product in tetrahydrofuran (3 mL) was added dropwise to a solution of ethyl 6-[N-(2-chloro-4-fluorophenyl)sulfamoyl]cyclohex-1-ene-1-carboxylate (1.45 g) and triethylamine (0.56 mL) in tetrahydrofuran (8 mL) under ice-cooling, and the mixture was stirred for 30 min under ice-cooling and at room temperature overnight. The reaction mixture was diluted with ethyl acetate (30 mL), washed successively with water (30 mL), 5% aqueous sodium bicarbonate (20 mLx2) and saturated brine (20 mL), dried over anhydrous magnesium sulfate, and concentrated under reduced pressure. The residue was purified by column chromatography (silica gel 60 g, ethyl acetate/hexane 1/4) to give the title compound (1.80 g) as a white powder.
¹H-NMR (CDCl₃, 200 MHz):δ 1.31, 1.34 (3H, tx2, J = 7 Hz), 1.71-2.98 (10H, m), 4.17-4.26 (2H, m), 4.68-4.83 (2H, m), 5.11, 5.32 (1H, br), 7.03-7.20 (1H, m), 7.31-7.79 (3H, m).

### Example 27

### 4-((2-chloro-4-fluorophenyl){[2-(ethoxycarbonyl)cyclohex-2-en-1-yl]sulfonyl}amino)-4-oxobutanoic acid

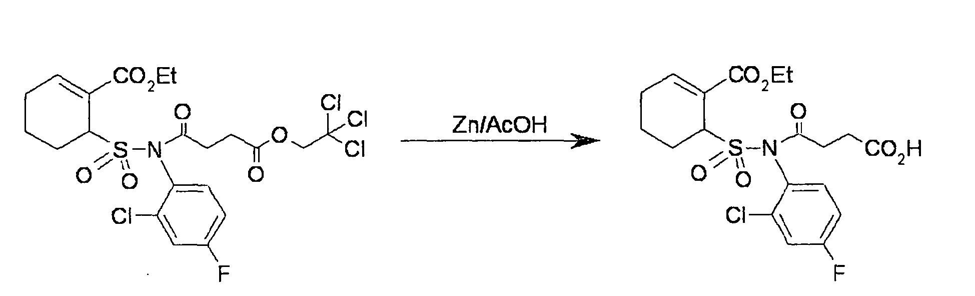

Ethyl 6-({(2-chloro-4-fluorophenyl)[4-oxo-4-(2,2,2-trichloroethoxy)butanoyl]amino}sulfonyl)cyclohex-1-ene-1-carboxylate (360 mg) obtained in Example 26 was dissolved in N,N-dimethylformamide (2 mL), zinc powder (79 mg) and acetic acid (0.18 mL) were added under ice-cooling, and the mixture was stirred at room temperature for 10 min. Ice water was added to the reaction mixture, and the mixture was extracted with ethyl acetate (20 mL). The ethyl acetate layer was washed with cold water (20 mL), dried over anhydrous magnesium sulfate, and concentrated under reduced pressure to give the title compound (0.30 g) as a colorless oil.
¹H-NMR (CDCl₃, 300 MHz)):δ 1.23-1.34 (3H, m), 1.71-3.01 (10H, m), 4.08-4.25 (2H, m), 5.10, 5.29 (1H, br), 7.04-8.02 (4H, m).

### Formulation Example 1 (Production of injection)

Using the compound obtained in Example 3, an injection having the following composition was produced.

| | |
|---|---|
| Compound of Example 3 | 100 mg |
| Saline for injection | 100 mL |

### Experimental Example 1: confirmation of solubility

It was confirmed that the solubility of the compounds of Examples 3, 6, 21 and 23 in saline was not less than 1 mg/mL by visual observation.

### Experimental Example 2: Solubility test

The compounds of Examples 9, 12 and 15 were measured and placed in a glass container by 1 mg each, saline was added in small portions, and the mixture was stirred. The solubility was visually observed. In addition, as a conventional compound having a nitric oxide (NO) production inhibitory effect and an inflammatory cytokine production inhibitory effect, ethyl 6-[N-(2-chloro-4-fluorophenyl)sulfamoyl]cyclohex-1-ene-1-carboxylate (indicated as conventional compound in Table 1) was treated according to the Japanese Pharmacopoeia, general notices, and analyzed by HPLC to determined the solubility. The results are shown in Table 1.

**Table 1**

| Example No. | solubility (mg/mL) |
|---|---|
| 9 | >9.9 |
| 12 | >10.9 |
| 15 | 2.0 - 2.4 |
| Conventional compound | 0.01 |

### Experimental Example 3: Evaluation of efficacy in mouse endotoxin shock model

Female BALB/c mice (6-week-old) were purchased and, after preliminary rearing for 1 week, divided into groups (7 per group). The compound of Example 3 was dissolved in saline immediately before administration, and intravenously administered at 10 mg/kg. For the control group, a solvent was administered in the same manner. One hour later, LPS (5 mg/kg) was intraperitoneally inoculated, and the survival of the mice was observed. The efficacy was evaluated based on the survival rate at 5 days from LPS inoculation. The results are shown in Table 2.

**Table 2**

| Example No. | number of survived mice (N=7) |
|---|---|
| control | 0 |
| 3 | 6 |

### Industrial Applicability

Compound (I) of the present invention has high solubility in water and is suitable for use as a liquid preparation (e.g., injection).

This application is based on patent application No. 188238/2006 filed in Japan, the contents of which are hereby incorporated by reference.
Although the present invention have been presented or described by referring to preferred embodiments of this invention, it will, however, be understood by those of ordinary skill in the art that various modifications may be made to the forms and details without departing from the scope of the invention as set forth in the appended claims. All patents, patent publications and other publications indicated or cited in the Specification are hereby incorporated in their entireties by reference.

## Claims

1. A compound represented by the formula (I): 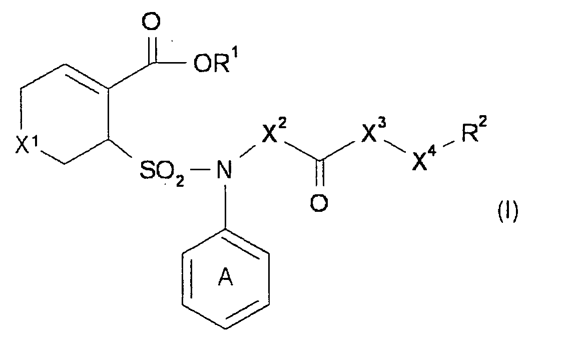 wherein
X¹ is a methylene group or an oxygen atom;
R¹ is a C₁₋₆ alkyl group;
ring A is a phenyl group optionally having one or two the same or different halogen atoms;
X² is a bond, -CHR^{x}-O- wherein R^{x} is a hydrogen atom or a C₁₋₆ alkyl group, or a group represented by the formula: 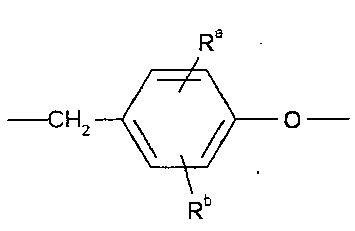
wherein R^{a} and R^{b} are the same or different and each is a hydrogen atom, a halogen atom, a C₁₋₆ alkyl group, a C₁₋₆ alkoxy group, a carboxyl group or a C₁₋₆ alkoxy-carbonyl group;
X³ is a bond, -O- or -NR³- wherein R³ is a hydrogen atom or an aliphatic hydrocarbon group optionally having substituent(s);
X⁴ is a divalent aliphatic hydrocarbon group optionally having substituent(s); and
R² is -COOH, -OPO(OH)₂ or a -NHR⁴ group wherein R⁴ is an aliphatic hydrocarbon group optionally having substituent(s), or R³ or R⁴ is optionally bonded to X⁴ to form a 5- to 8-membered ring optionally having substituent(s), or a salt thereof.

2. The compound of claim 1, wherein R¹ is an ethyl group, or a salt thereof.

3. The compound of claim 1, wherein ring A is a group represented by the formula: 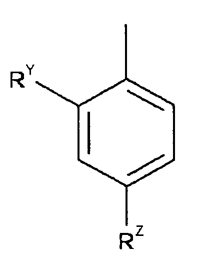 wherein R^{Y} and R^{Z} are the same or different and each is a halogen atom, or a salt thereof.

4. The compound of claim 1, wherein X² is a -CH₂-O- group; and X³ is -O- or -NR^{3a}- wherein R^{3a} is a hydrogen atom or a c₁₋₆ alkyl group optionally having substituent(s), or R^{3a} is optionally bonded to X⁴ to form a 5- to 8-membered ring optionally having substituent(s), or a salt thereof.

5. The compound of claim 1, wherein X² is a group represented by the formula: 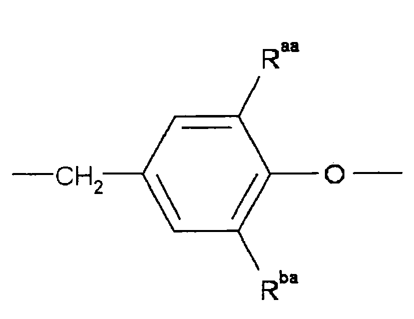 wherein R^{aa} and R^{ba} are the same or different and each is a C₁₋₆ alkyl group; and
X³ is a bond, or a salt thereof.

6. The compound of claim 1, wherein X² and X³ are each a bond, or a salt thereof.

7. The compound of claim 1, wherein R² is -COOH or a -NHR^{4a} group wherein R^{4a} is a C₁₋₆ alkyl group optionally having substituent(s), or R^{4a} is optionally bonded to X⁴ to form a 5-to 8-membered ring optionally having substituent(s), or a salt thereof.

8. The compound of claim 1, wherein X¹ is a methylene group, or a salt thereof.

9. The compound of claim 1, wherein X¹ is a methylene group;
R¹ is an ethyl group;
ring A is a group represented by the formula: 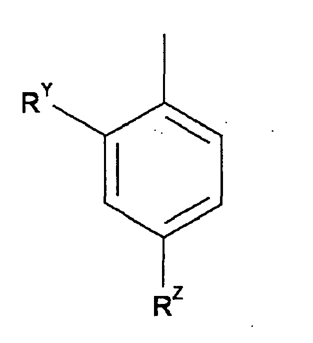 wherein R^{Y} and R^{Z} are the same or different and each is a halogen atom;
X² is a -CH₂-O- group;
X³ is -O- or -NR^{3a}- wherein R^{3a} is a hydrogen atom or an optionally substituted C₁₋₆ alkyl group, or R^{3a} is optionally bonded to X⁴ to form a 5- to 8-membered ring optionally having substituent(s);
X⁴ is an optionally substituted C₁₋₆ alkylene group; and
R² is -COOH, or a salt thereof.

10. The compound of claim 1, wherein X¹ is a methylene group;
R¹ is an ethyl group;
ring A is a group represented by the formula: 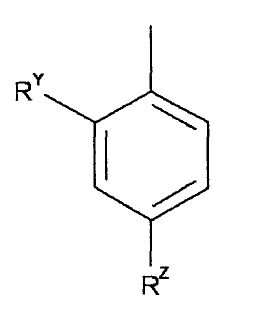 wherein R^{Y} and R^{Z} are the same or different and each is a halogen atom;
X² is a group represented by the formula: 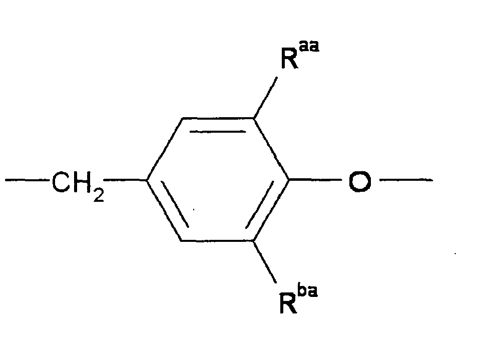 wherein R^{aa} and R^{ba} are the same or different and each is a C₁₋₆ alkyl group;
X³ is a bond;
X⁴ is an optionally substituted C₁₋₆ alkylene group; and
R² is a -NHR^{4a} group wherein R^{4a} is a C₁₋₆ alkyl group optionally having substituent(s), or R^{4a} is optionally bonded to X⁴ to form a 5- to 8-membered ring optionally having substituent(s), or a salt thereof.

11. The compound of claim 1, wherein X¹ is a methylene group;
R¹ is an ethyl group;
ring A is a group represented by the formula: 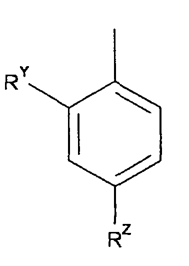 wherein R^{Y} and R^{Z} are the same or different and each is a halogen atom;
X² and X³ are each a bond;
X⁴ is an optionally substituted C₁₋₆ alkylene group; and
R² is -COOH, or a salt thereof.

12. The compound of claim 7, wherein the salt is selected from the group consisting of a sodium salt, a potassium salt and a hydrochloride.

13. A method of producing the compound of claim 1 or a salt thereof, which comprises condensing a compound represented by the formula (II): 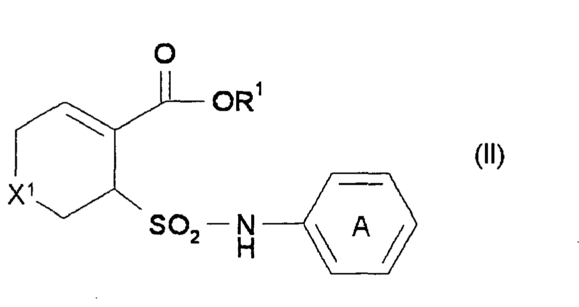 wherein each symbol is as defined in claim 1, or a salt thereof, with a compound represented by the formula (III): 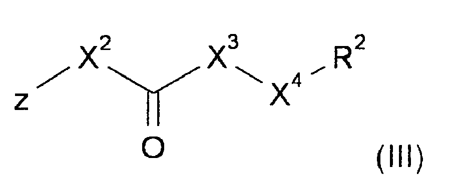 wherein Z is a halogen atom, and other symbols are as defined in claim 1, or a salt thereof.

14. A pharmaceutical composition comprising the compound of claim 1 or a salt thereof.

15. The pharmaceutical composition of claim 14, which is an agent for the prophylaxis or treatment of cardiac disease, autoimmune disease or septic shock.

16. The pharmaceutical composition of claim 14, which is an agent for the prophylaxis or treatment of an organ disorder or severe sepsis.

17. The pharmaceutical composition of claim 16, wherein the organ is an organ of the central nerve system, the circulatory system, the bone and joint system or the digestive system.

18. A method for the prophylaxis or treatment of a cardiac disease, an autoimmune disease or septic shock, which comprises administering an effective amount of the compound of claim 1 or a salt thereof to a mammal.

19. A method for the prophylaxis or treatment of an organ disorder or severe sepsis to a mammal, which comprises administering an effective amount of the compound of claim 1 or a salt thereof to a mammal.

20. Use of the compound of claim 1 or a salt thereof for the production of an agent for the prophylaxis or treatment of a cardiac disease, an autoimmune disease or septic shock.

21. Use of the compound of claim 1 or a salt thereof for the production of an agent for the prophylaxis or treatment of an organ disorder or severe sepsis.
